# EUROPEAN PATENT APPLICATION

(11) **EP 3 981 785 A1**
(43) Date of publication of application: **13.04.2022**
(21) Application number: 21206280.6
(22) Date of filing: 23.10.2017
(51) Int. Cl.: C07K 16/00, C07K 16/28, C07K 16/30, G01N 33/569, C12Q 1/68, C12Q 1/6809, G01N 33/50, A61K 39/00, A61P 35/00, A61P 37/04, C07K 14/725, G01N 33/574, A61P 43/00

(54) **METHODS FOR SCREENING B CELL LYMPHOCYTES**

(30) Priority: 23.10.2016 US 201662411690 P; 24.10.2016 US 201662412092 P
(62) Divisional of application: 17862641.2
(71) Applicant: Berkeley Lights, Inc., Emeryville, CA 94608 (US)
(72) Inventor: PARK, Minha, Emeryville, 94608 (US); BRIGGS, Jason C, Emeryville, 94608 (US); MCEWEN, Jason M, Emeryville, 94608 (US); RAMENANI, Ravi K, Emeryville, 94608 (US); CHIRRA DINAKAR, Hariharasudhan, Emeryville, 94608 (US); SZETO, Kai W., Emeryville, 94608 (US); HIGA, Adrienne T., Emeryville, 94608 (US); WHITE, Mark P., Emeryville, 94608 (US); LOWE, Randall D. Jr., Emeryville, 94608 (US); WANG, Xiaohua, Emeryville, 94608 (US); CHAPMAN, Kevin T., Emeryville, 94608 (US)
(74) Representative: D Young & Co LLP

(57) **Abstract**

Methods are described herein for screening an antibody producing cell within a microfluidic environment. The antibody producing cell may be a B cell lymphocyte, which may be a memory B cell or a plasma cell. An antigen of interest may be brought into proximity with the antibody producing cell and binding of the antigen by an antibody produced by the antibody producing cell may be monitored. Methods of obtaining a sequencing library from an antibody producing cell are also described.

## Description

This application is a non-provisional application claiming the benefit under 35 U.S.C. 119(e) of U.S. Provisional Application No. 62/411,690, filed on October 23, 2016, and U.S. Provisional Application No. 62/412,092, filed on October 24, 2016, each of which disclosures is herein incorporated by reference in its entirety.

### BACKGROUND OF THE INVENTION

It has been of interest to screen and identify cells that produce an antibody that is capable of binding specifically to an antigen of interest, including within the area of hybridoma development. Further it is of interest to identify a highly expressing antibody producing cell. It has been a difficult challenge to provide a suitable environment that permits a suitable growth environment for an antibody producing cell as well as providing an environment in which assay of binding/expression may be readily monitored. Further, it is desirable to provide correlation of the assay results with the specific cell which demonstrates desirable expression/binding properties of its secreted antibody. Improvements to these aspects of the field of antibody development are provided herein.

### SUMMARY OF THE INVENTION

The invention is based, in part, on the discovery that B cell lymphocytes, including primary B cells, can be screened within a microfluidic device to determine whether the B cell lymphocytes express antibodies that specifically bind to an antigen of interest. Accordingly, in one aspect, a method of detecting expression by an antibody-producing cell of an antibody that specifically binds to an antigen of interest is provided. The method includes the step of introducing the antibody-producing cell into a microfluidic device. The antibody-producing cell can be, for example, a B cell lymphocyte, such as a memory B cell or a plasma cell.

The microfluidic device, for example, can include a flow region, which may include a microfluidic channel, and at least one microfluidic sequestration pen (e.g., a plurality of sequestration pens). Each sequestration pen can include an isolation region and a connection region that fluidically connects the isolation region to the flow region (e.g., microfluidic channel).

Some of the disclosed methods include the additional steps of: loading the antibody-producing cell into the isolation region of the sequestration pen; introducing the antigen of interest into the microfluidic device, such that the antigen of interest is proximal to the antibody-producing cell; and monitoring binding of the antigen of interest to antibody expressed by the antibody-producing cell. The loaded cell can be one of a population of cells (e.g., B cells) loaded into a microfluidic device that has a plurality of sequestration pens. In such embodiments, one or more antibody-producing cells can be loaded into the isolation region of each of the plurality of sequestration pens. In some embodiments, a single antibody-producing cell is loaded into each sequestration pen. The antigen of interest, when provided in close proximity to the antibody-producing cell, can be soluble or attached to a micro-object, such as a cell, a liposome, a lipid nanoraft, or a synthetic bead (e.g., a microbead or a nanobead). Such micro-objects can be microscopically visible. Monitoring binding between the antigen of interest and antibodies produced by the antibody-producing cell(s) can include: providing a labeled antigen of interest, and detecting direct binding of the antigen of interest (e.g., labeled antigen of interest); providing a labeled antibody-binding agent, and detecting indirect binding of the labeled antibody-binding agent to the antigen of interest (e.g. to a micro-object that presents the antigen of interest); and providing an antibody-binding agent, and detecting indirect binding of labeled antigen of interest to antibody-binding agent (e.g., to a micro-object linked to a plurality of antibody-binding agents). The antibody-binding agent can be isotype specific (e.g., an anti-IgG antibody or IgG-binding fragment thereof). The label on the antigen or interest or the antibody-binding agent can be a fluorescent label.

For antibody-producing cells identified as expressing an antigen-binding antibody, the disclosed methods can further include the steps of: lysing the identified cell (e.g. B cell); reverse transcribing V_{H} mRNA and/or V_{L} mRNA originating from the lysed cell to form V_{H} cDNA and/or V_{L} cDNA, respectively; and sequencing at least a portion of said V_{H} cDNA and/or V_{L} cDNA. The lysing and reverse transcribing steps can be performed within the microfluidic device or external to the microfluidic device. For example, an identified cell can be exported (e.g., as a single cell) for cell lysis and further processing. Alternatively, an identified cell be lysed within the sequestration pen in which it was loaded, and the V_{H} mRNA and/or V_{L} mRNA released upon lysis can be sequestered by capture beads (i.e., beads having oliogonucleotides linked to their surface, with the oliogonucleotides being capable of specifically binding V_{H} mRNA and/or V_{L} mRNA). The capture beads can be exported from the microfluidic device either before or after the captured V_{H} mRNA and/or the captured V_{L} mRNA is reverse transcribed.

These and other features and advantages of the methods of the invention will be set forth or will become more fully apparent in the description that follows and in the appended claims. The features and advantages may be realized and obtained by means of the instruments and combinations particularly pointed out in the appended Examples and claims. Furthermore, the features and advantages of the described systems and methods may be learned by the practice or will be obvious from the description, as set forth hereinafter.

### BRIEF DESCRIPTION OF THE DRAWINGS

Figure 1A illustrates an example of a microfluidic device and a system for use with the microfluidic device, including associated control equipment according to some embodiments disclosed herein.
Figures 1B and 1C illustrate vertical and horizontal cross-sectional views, respectively, of a microfluidic device according to some embodiments disclosed herein.
Figures 2A and 2B illustrate vertical and horizontal cross-sectional views, respectively, of a microfluidic device having isolation pens according to some embodiments of the invention.
Figure 2C illustrates a detailed horizontal cross-sectional view of a sequestration pen according to some embodiments disclosed herein.
Figure 2D illustrates a partial horizontal cross-sectional view of a microfluidic device having isolation pens according to some embodiments disclosed herein.
Figures 2E and 2F illustrate detailed horizontal cross-sectional views of sequestration pens according to some embodiments disclosed herein.
Figure 2G illustrates a microfluidic device having a flow region which contains a plurality of flow channels, each flow channel fluidically connected to a plurality of sequestration pens, according to an embodiment disclosed herein.
Figure 2H illustrates a partial vertical cross-sectional view of a microfluidic device in which the inward facing surface of the base and the inward facing surface of the cover are conditioned surfaces according to an embodiment disclosed herein.
Figure 3A illustrates a specific example of a system nest, configured to operatively couple with a microfluidic device, and associated control equipment according to some embodiments disclosed herein.
Figure 3B illustrates an optical train of a system for controlling a microfluidic device according to some embodiments disclosed herein.

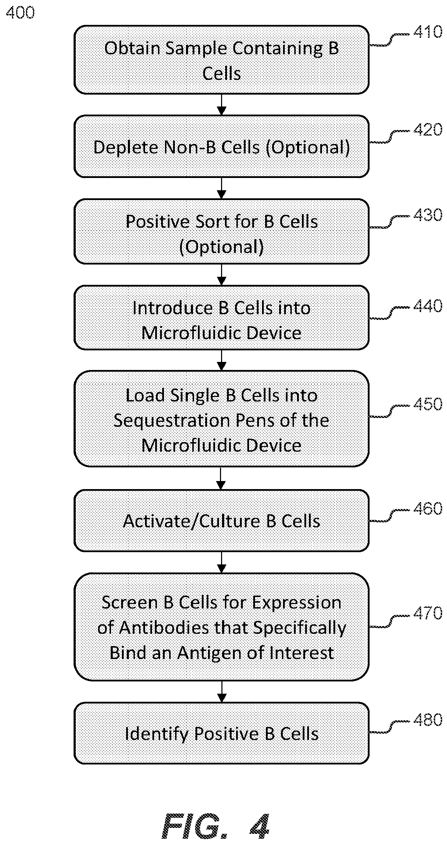

Figure 4 illustrates steps in an exemplary workflow for detecting a B cell lymphocyte expressing an antibody that specifically binds to an antigen of interest according to some embodiments disclosed herein.
Figures 5A-5C is a photographic representation of a microfluidic device comprising a plurality of microfluidic channels, each fluidically connected with a plurality of sequestration pens, and illustrates a method of screening a B cell lymphocyte according to some embodiments disclosed herein.
Figure 6A is a schematic representation of a method for activating and screening memory B cells according to an embodiment disclosed herein.
Figure 6B is an image of image of individual memory B cells being moved into sequestration pens according to an embodiment disclosed herein.
Figure 6C is a diagram of a multiplex assay according to some embodiments disclosed herein.
Figure 6D is a fluorescent image of memory B cells being assayed according to an embodiment disclosed herein.
Figure 6E is a schematic representation of steps in a method for screening memory B cells that begins with assaying a polyclonal group of memory B cells and then separating the group of memory B cells into individual sequestration pens for a subsequent assay, according to an embodiment disclosed herein.
Figure 7A is a schematic representation of a method for screening plasma cells according to an embodiment disclosed herein.
Figure 7B is a set of brightfield and corresponding fluorescent images of plasma cells being assayed according to an embodiment disclosed herein.
Figure 8 is a schematic representation of a method of producing a BCR sequencing library.
Figure 9A is a graphical representation of an electropherogram analysis of the size distribution of cDNA produced from single cell export and mRNA capture.
Figure 9B is a photographic representation of the electropherogram resulting from a single cell amplicon produced by an embodiment of the method described herein.
Figures 10A-10C are photographic representations of the electropherograms of amplicons produced from mRNA captured from 19 individual cells according to an embodiment of the method described herein.

### DETAILED DESCRIPTION OF THE INVENTION

This specification describes exemplary embodiments and applications of the invention. The invention, however, is not limited to these exemplary embodiments and applications or to the manner in which the exemplary embodiments and applications operate or are described herein. Moreover, the figures may show simplified or partial views, and the dimensions of elements in the figures may be exaggerated or otherwise not in proportion. In addition, as the terms "on," "attached to," "connected to," "coupled to," or similar words are used herein, one element (e.g., a material, a layer, a substrate, etc.) can be "on," "attached to," "connected to," or "coupled to" another element regardless of whether the one element is directly on, attached to, connected to, or coupled to the other element or there are one or more intervening elements between the one element and the other element. Also, unless the context dictates otherwise, directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. In addition, where reference is made to a list of elements (e.g., elements a, b, c), such reference is intended to include any one of the listed elements by itself, any combination of less than all of the listed elements, and/or a combination of all of the listed elements. Section divisions in the specification are for ease of review only and do not limit any combination of elements discussed.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

The term "ones" means more than one.

As used herein, the term "plurality" can be 2, 3, 4, 5, 6, 7, 8, 9, 10, or more.

As used herein, the term "disposed" encompasses within its meaning "located."

As used herein, a "microfluidic device" or "microfluidic apparatus" is a device that includes one or more discrete microfluidic circuits configured to hold a fluid, each microfluidic circuit comprised of fluidically interconnected circuit elements, including but not limited to region(s), flow path(s), channel(s), chamber(s), and/or pen(s), and at least one port configured to allow the fluid (and, optionally, micro-objects suspended in the fluid) to flow into and/or out of the microfluidic device. Typically, a microfluidic circuit of a microfluidic device will include a flow region, which may include a microfluidic channel, and at least one chamber, and will hold a volume of fluid of less than about 1 mL, e.g., less than about 750, 500, 250, 200, 150, 100, 75, 50, 25, 20, 15, 10, 9, 8, 7, 6, 5, 4, 3, or 2 µL. In certain embodiments, the microfluidic circuit holds about 1-2, 1-3, 1-4, 1-5, 2-5, 2-8, 2-10, 2-12, 2-15, 2-20, 5-20, 5-30, 5-40, 5-50, 10-50, 10-75, 10-100, 20-100, 20-150, 20-200, 50-200, 50-250, or 50-300 µL. The microfluidic circuit may be configured to have a first end fluidically connected with a first port (e.g., an inlet) in the microfluidic device and a second end fluidically connected with a second port (e.g., an outlet) in the microfluidic device.

As used herein, a "nanofluidic device" or "nanofluidic apparatus" is a type of microfluidic device having a microfluidic circuit that contains at least one circuit element configured to hold a volume of fluid of less than about 1 µL, e.g., less than about 750, 500, 250, 200, 150, 100, 75, 50, 25, 20, 15, 10, 9, 8, 7, 6, 5, 4, 3, 2, 1 nL or less. A nanofluidic device may comprise a plurality of circuit elements (e.g., at least 2, 3, 4, 5, 6, 7, 8, 9, 10, 15, 20, 25, 50, 75, 100, 150, 200, 250, 300, 400, 500, 600, 700, 800, 900, 1000, 1500, 2000, 2500, 3000, 3500, 4000, 4500, 5000, 6000, 7000, 8000, 9000, 10,000, or more). In certain embodiments, one or more (e.g., all) of the at least one circuit elements is configured to hold a volume of fluid of about 100 pL to 1 nL, 100 pL to 2 nL, 100 pL to 5 nL, 250 pL to 2 nL, 250 pL to 5 nL, 250 pL to 10 nL, 500 pL to 5 nL, 500 pL to 10 nL, 500 pL to 15 nL, 750 pL to 10 nL, 750 pL to 15 nL, 750 pL to 20 nL, 1 to 10 nL, 1 to 15 nL, 1 to 20 nL, 1 to 25 nL, or 1 to 50 nL. In other embodiments, one or more (e.g., all) of the at least one circuit elements is configured to hold a volume of fluid of about 20 nL to 200nL, 100 to 200 nL, 100 to 300 nL, 100 to 400 nL, 100 to 500 nL, 200 to 300 nL, 200 to 400 nL, 200 to 500 nL, 200 to 600 nL, 200 to 700 nL, 250 to 400 nL, 250 to 500 nL, 250 to 600 nL, or 250 to 750 nL.

A microfluidic device or a nanofluidic device may be referred to herein as a "microfluidic chip" or a "chip"; or "nanofluidic chip" or "chip".

A "microfluidic channel" or "flow channel" as used herein refers to a flow region of a microfluidic device having a length that is significantly longer than both the horizontal and vertical dimensions. For example, the flow channel can be at least 5 times the length of either the horizontal or vertical dimension, e.g., at least 10 times the length, at least 25 times the length, at least 100 times the length, at least 200 times the length, at least 500 times the length, at least 1,000 times the length, at least 5,000 times the length, or longer. In some embodiments, the length of a flow channel is in the range of from about 50,000 microns to about 500,000 microns, including any range therebetween. In some embodiments, the horizontal dimension is in the range of from about 100 microns to about 1000 microns (e.g., about 150 to about 500 microns) and the vertical dimension is in the range of from about 25 microns to about 200 microns, e.g., from about 40 to about 150 microns. It is noted that a flow channel may have a variety of different spatial configurations in a microfluidic device, and thus is not restricted to a perfectly linear element. For example, a flow channel may include one or more sections having any of the following configurations: curve, bend, spiral, incline, decline, fork (e.g., multiple different flow paths), and any combination thereof. In addition, a flow channel may have different cross-sectional areas along its path, widening and constricting to provide a desired fluid flow therein.

As used herein, the term "obstruction" refers generally to a bump or similar type of structure that is sufficiently large so as to partially (but not completely) impede movement of target micro-objects between two different regions or circuit elements in a microfluidic device. The two different regions/circuit elements can be, for example, a microfluidic sequestration pen and a microfluidic channel, or a connection region and an isolation region of a microfluidic sequestration pen.

As used herein, the term "constriction" refers generally to a narrowing of a width of a circuit element (or an interface between two circuit elements) in a microfluidic device. The constriction can be located, for example, at the interface between a microfluidic sequestration pen and a microfluidic channel, or at the interface between an isolation region and a connection region of a microfluidic sequestration pen.

As used herein, the term "transparent" refers to a material which allows visible light to pass through without substantially altering the light as is passes through.

As used herein, the term "micro-object" refers generally to any microscopic object that may be isolated and/or manipulated in accordance with the present invention. Non-limiting examples of micro-objects include: inanimate micro-objects such as microparticles; microbeads (e.g., polystyrene beads, Luminex^{™} beads, or the like); magnetic beads; microrods; microwires; quantum dots, and the like; biological micro-objects such as cells; biological organelles; vesicles, or complexes; synthetic vesicles; liposomes (e.g., synthetic or derived from membrane preparations); lipid nanorafts, and the like; or a combination of inanimate micro-objects and biological micro-objects (e.g., microbeads attached to cells, liposome-coated micro-beads, liposome-coated magnetic beads, or the like). Beads may include moieties/molecules covalently or non-covalently attached, such as fluorescent labels, proteins, carbohydrates, antigens, small molecule signaling moieties, or other chemical/biological species capable of use in an assay. Lipid nanorafts have been described, for example, in Ritchie et al. (2009) "Reconstitution of Membrane Proteins in Phospholipid Bilayer Nanodiscs," Methods Enzymol., 464:211-231.

As used herein, the term "cell" is used interchangeably with the term "biological cell." Non-limiting examples of biological cells include eukaryotic cells, plant cells, animal cells, such as mammalian cells, reptilian cells, avian cells, fish cells, or the like, prokaryotic cells, bacterial cells, fungal cells, protozoan cells, or the like, cells dissociated from a tissue, such as muscle, cartilage, fat, skin, liver, lung, neural tissue, and the like, immunological cells, such as T cells, B cells, natural killer cells, macrophages, and the like, embryos (e.g., zygotes), oocytes, ova, sperm cells, hybridomas, cultured cells, cells from a cell line, cancer cells, infected cells, transfected and/or transformed cells, reporter cells, and the like. A mammalian cell can be, for example, from a human, a mouse, a rat, a horse, a goat, a sheep, a cow, a primate, or the like.

A colony of biological cells is "clonal" if all of the living cells in the colony that are capable of reproducing are daughter cells derived from a single parent cell. In certain embodiments, all the daughter cells in a colonal colony are derived from the single parent cell by no more than 10 divisions. In other embodiments, all the daughter cells in a colonal colony are derived from the single parent cell by no more than 14 divisions. In other embodiments, all the daughter cells in a colonal colony are derived from the single parent cell by no more than 17 divisions. In other embodiments, all the daughter cells in a colonal colony are derived from the single parent cell by no more than 20 divisions. The term "clonal cells" refers to cells of the same clonal colony.

As used herein, a "colony" of biological cells refers to 2 or more cells (e.g. about 2 to about 20, about 4 to about 40, about 6 to about 60, about 8 to about 80, about 10 to about 100, about 20 about 200, about 40 about 400, about 60 about 600, about 80 about 800, about 100 about 1000, or greater than 1000 cells).

As used herein, the term "maintaining (a) cell(s)" refers to providing an environment comprising both fluidic and gaseous components and, optionally a surface, that provides the conditions necessary to keep the cells viable and/or expanding.

As used herein, the term "expanding" when referring to cells, refers to increasing in cell number.

A "component" of a fluidic medium is any chemical or biochemical molecule present in the medium, including solvent molecules, ions, small molecules, antibiotics, nucleotides and nucleosides, nucleic acids, amino acids, peptides, proteins, sugars, carbohydrates, lipids, fatty acids, cholesterol, metabolites, or the like.

As used herein in reference to a fluidic medium, "diffuse" and "diffusion" refer to thermodynamic movement of a component of the fluidic medium down a concentration gradient.

The phrase "flow of a medium" means bulk movement of a fluidic medium primarily due to any mechanism other than diffusion. For example, flow of a medium can involve movement of the fluidic medium from one point to another point due to a pressure differential between the points. Such flow can include a continuous, pulsed, periodic, random, intermittent, or reciprocating flow of the liquid, or any combination thereof. When one fluidic medium flows into another fluidic medium, turbulence and mixing of the media can result.

The phrase "substantially no flow" refers to a rate of flow of a fluidic medium that, averaged over time, is less than the rate of diffusion of components of a material (e.g., an analyte of interest) into or within the fluidic medium. The rate of diffusion of components of such a material can depend on, for example, temperature, the size of the components, and the strength of interactions between the components and the fluidic medium.

As used herein in reference to different regions within a microfluidic device, the phrase "fluidically connected" means that, when the different regions are substantially filled with fluid, such as fluidic media, the fluid in each of the regions is connected so as to form a single body of fluid. This does not mean that the fluids (or fluidic media) in the different regions are necessarily identical in composition. Rather, the fluids in different fluidically connected regions of a microfluidic device can have different compositions (e.g., different concentrations of solutes, such as proteins, carbohydrates, ions, or other molecules) which are in flux as solutes move down their respective concentration gradients and/or fluids flow through the device.

A microfluidic (or nanofluidic) device can comprise "swept" regions and "unswept" regions. As used herein, a "swept" region is comprised of one or more fluidically interconnected circuit elements of a microfluidic circuit, each of which experiences a flow of medium when fluid is flowing through the microfluidic circuit. The circuit elements of a swept region can include, for example, regions, channels, and all or parts of chambers. As used herein, an "unswept" region is comprised of one or more fluidically interconnected circuit element of a microfluidic circuit, each of which experiences substantially no flux of fluid when fluid is flowing through the microfluidic circuit. An unswept region can be fluidically connected to a swept region, provided the fluidic connections are structured to enable diffusion but substantially no flow of media between the swept region and the unswept region. The microfluidic device can thus be structured to substantially isolate an unswept region from a flow of medium in a swept region, while enabling substantially only diffusive fluidic communication between the swept region and the unswept region. For example, a flow channel of a microfluidic device is an example of a swept region while an isolation region (described in further detail below) of a microfluidic device is an example of an unswept region.

As used herein, a "flow path" refers to one or more fluidically connected circuit elements (e.g. channel(s), region(s), chamber(s) and the like) that define, and are subject to, the trajectory of a flow of medium. A flow path is thus an example of a swept region of a microfluidic device. Other circuit elements (e.g., unswept regions) may be fluidically connected with the circuit elements that comprise the flow path without being subject to the flow of medium in the flow path.

As used herein, "B" used to denote a single nucleotide, is a nucleotide selected from G (guanosine), C (cytidine) and T (thymidine) nucleotides but does not include A (adenine).

As used herein, "H" used to denote a single nucleotide, is a nucleotide selected from A, C and T, but does not include G.

As used herein, "D" used to denote a single nucleotide, is a nucleotide selected from A, G, and T, but does not include C.

As used herein, "K" used to denote a single nucleotide, is a nucleotide selected from G and T.

As used herein, "N" used to denote a single nucleotide, is a nucleotide selected from A, C, G, and T.

As used herein, "R" used to denote a single nucleotide, is a nucleotide selected from A and G.

As used herein, "S" used to denote a single nucleotide, is a nucleotide selected from G and C.

As used herein, "V" used to denote a single nucleotide, is a nucleotide selected from A, G, and C, and does not include T.

As used herein, "Y" used to denote a single nucleotide, is a nucleotide selected from C and T.

As used herein, "I" used to denote a single nucleotide is inosine.

As used herein, A, C, T, G followed by "*" indicates phosophorothioate substitution in the phosphate linkage of that nucleotide.

As used herein, IsoG is isoguanosine; IsoC is isocytidine; IsodG is a isoguanosine deoxyribonucleotide and IsodC is a isocytidine deoxyribonucleotide. Each of the isoguanosine and isocytidine ribo- or deoxyribo- nuleotides contain a nucleobase that is isomeric to guanine nucleobase or cytosine nucleobase, respectively, usually incorporated within RNA or DNA.

As used herein, rG denotes a ribonucleotide included within a nucleic acid otherwise containing deoxyribonucleotides. A nucleic acid containing all ribonucleotides may not include labeling to indicated that each nucleotide is a ribonucleotide, but is made clear by context.

As used herein, a "priming sequence" is an oligonucleotide sequence which is part of a larger oligonucleotide and, when separated from the larger oligonucleotide such that the priming sequence includes a free 3' end, can function as a primer in a DNA (or RNA) polymerization reaction.

As used herein: µm means micrometer, µm³ means cubic micrometer, pL means picoliter, nL means nanoliter, and µL (or uL) means microliter.

**Methods of loading.** Loading of biological micro-objects or micro-objects such as, but not limited to, beads, can involve the use of fluid flow, gravity, a dielectrophoresis (DEP) force, electrowetting, a magnetic force, or any combination thereof as described herein. The DEP force can be optically actuated, such as by an optoelectronic tweezers (OET) configuration and/or electrically actuated, such as by activation of electrodes/electrode regions in a temporal/spatial pattern. Similarly, electrowetting force may be optically actuated, such as by an opto-electro wetting (OEW) configuration and/or electrically actuated, such as by activation of electrodes/electrode regions in a temporal spatial pattern.

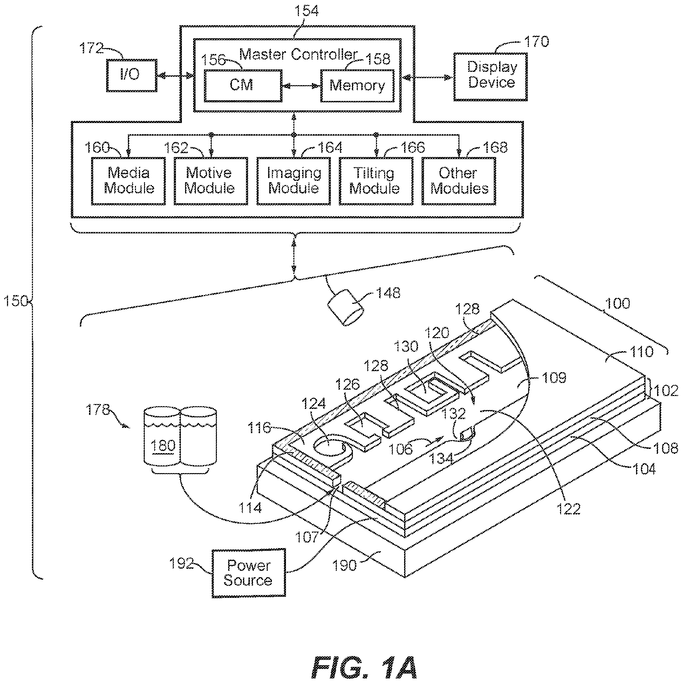

**Microfluidic devices and systems for operating and observing such devices.** Figure 1A illustrates an example of a microfluidic device 100 and a system 150 which can be used for the screening and detection of antibody-producing cells that secrete antibodies that bind (e.g., specifically bind) to an antigen of interest. A perspective view of the microfluidic device 100 is shown having a partial cut-away of its cover 110 to provide a partial view into the microfluidic device 100. The microfluidic device 100 generally comprises a microfluidic circuit 120 comprising a flow path 106 through which a fluidic medium 180 can flow, optionally carrying one or more micro-objects (not shown) into and/or through the microfluidic circuit 120. Although a single microfluidic circuit 120 is illustrated in Figure 1A, suitable microfluidic devices can include a plurality (e.g., 2 or 3) of such microfluidic circuits. Regardless, the microfluidic device 100 can be configured to be a nanofluidic device. In the embodiment illustrated in Figure 1A, the microfluidic circuit 120 comprises a plurality of microfluidic sequestration pens 124, 126, 128, and 130, each having an opening (e.g., a single opening) in fluidic communication with flow path 106. As discussed further below, the microfluidic sequestration pens comprise various features and structures that have been optimized for retaining micro-objects in the microfluidic device, such as microfluidic device 100, even when a medium 180 is flowing through the flow path 106. Before turning to the foregoing, however, a brief description of microfluidic device 100 and system 150 is provided.

As generally illustrated in Figure 1A, the microfluidic circuit 120 is defined by an enclosure 102. Although the enclosure 102 can be physically structured in different configurations, in the example shown in Figure 1A the enclosure 102 is depicted as comprising a support structure 104 (e.g., a base), a microfluidic circuit structure 108, and a cover 110. The support structure 104, microfluidic circuit structure 108, and cover 110 can be attached to each other. For example, the microfluidic circuit structure 108 can be disposed on an inner surface 109 of the support structure 104, and the cover 110 can be disposed over the microfluidic circuit structure 108. Together with the support structure 104 and cover 110, the microfluidic circuit structure 108 can define the elements of the microfluidic circuit 120.

The support structure 104 can be at the bottom and the cover 110 at the top of the microfluidic circuit 120 as illustrated in Figure 1A. Alternatively, the support structure 104 and the cover 110 can be configured in other orientations. For example, the support structure 104 can be at the top and the cover 110 at the bottom of the microfluidic circuit 120. Regardless, there can be one or more ports 107 each comprising a passage into or out of the enclosure 102. Examples of a passage include a valve, a gate, a pass-through hole, or the like. As illustrated, port 107 is a pass-through hole created by a gap in the microfluidic circuit structure 108. However, the port 107 can be situated in other components of the enclosure 102, such as the cover 110. Only one port 107 is illustrated in Figure 1A but the microfluidic circuit 120 can have two or more ports 107. For example, there can be a first port 107 that functions as an inlet for fluid entering the microfluidic circuit 120, and there can be a second port 107 that functions as an outlet for fluid exiting the microfluidic circuit 120. Whether a port 107 function as an inlet or an outlet can depend upon the direction that fluid flows through flow path 106.

The support structure 104 can comprise one or more electrodes (not shown) and a substrate or a plurality of interconnected substrates. For example, the support structure 104 can comprise one or more semiconductor substrates, each of which is electrically connected to an electrode (e.g., all or a subset of the semiconductor substrates can be electrically connected to a single electrode). The support structure 104 can further comprise a printed circuit board assembly ("PCBA"). For example, the semiconductor substrate(s) can be mounted on a PCBA.

The microfluidic circuit structure 108 can define circuit elements of the microfluidic circuit 120. Such circuit elements can comprise spaces or regions that can be fluidly interconnected when microfluidic circuit 120 is filled with fluid, such as flow regions (which may include or be one or more flow channels), chambers, pens, traps, and the like. In the microfluidic circuit 120 illustrated in Figure 1A, the microfluidic circuit structure 108 comprises a frame 114 and a microfluidic circuit material 116. The frame 114 can partially or completely enclose the microfluidic circuit material 116. The frame 114 can be, for example, a relatively rigid structure substantially surrounding the microfluidic circuit material 116. For example, the frame 114 can comprise a metal material.

The microfluidic circuit material 116 can be patterned with cavities or the like to define circuit elements and interconnections of the microfluidic circuit 120. The microfluidic circuit material 116 can comprise a flexible material, such as a flexible polymer (e.g. rubber, plastic, elastomer, silicone, polydimethylsiloxane ("PDMS"), or the like), which can be gas permeable. Other examples of materials that can compose microfluidic circuit material 116 include molded glass, an etchable material such as silicone (e.g. photo-patternable silicone or "PPS"), photo-resist (e.g., SU8), or the like. In some embodiments, such materials-and thus the microfluidic circuit material 116-can be rigid and/or substantially impermeable to gas. Regardless, microfluidic circuit material 116 can be disposed on the support structure 104 and inside the frame 114.

The cover 110 can be an integral part of the frame 114 and/or the microfluidic circuit material 116. Alternatively, the cover 110 can be a structurally distinct element, as illustrated in Figure 1A. The cover 110 can comprise the same or different materials than the frame 114 and/or the microfluidic circuit material 116. Similarly, the support structure 104 can be a separate structure from the frame 114 or microfluidic circuit material 116 as illustrated, or an integral part of the frame 114 or microfluidic circuit material 116. Likewise, the frame 114 and microfluidic circuit material 116 can be separate structures as shown in Figure 1A or integral portions of the same structure.

In some embodiments, the cover 110 can comprise a rigid material. The rigid material may be glass or a material with similar properties. In some embodiments, the cover 110 can comprise a deformable material. The deformable material can be a polymer, such as PDMS. In some embodiments, the cover 110 can comprise both rigid and deformable materials. For example, one or more portions of cover 110 (e.g., one or more portions positioned over sequestration pens 124, 126, 128, 130) can comprise a deformable material that interfaces with rigid materials of the cover 110. In some embodiments, the cover 110 can further include one or more electrodes. The one or more electrodes can comprise a conductive oxide, such as indiumtin-oxide (ITO), which may be coated on glass or a similarly insulating material. Alternatively, the one or more electrodes can be flexible electrodes, such as single-walled nanotubes, multiwalled nanotubes, nanowires, clusters of electrically conductive nanoparticles, or combinations thereof, embedded in a deformable material, such as a polymer (e.g., PDMS). Flexible electrodes that can be used in microfluidic devices have been described, for example, in U.S. 2012/0325665 (Chiou et al.), the contents of which are incorporated herein by reference. In some embodiments, the cover 110 can be modified (e.g., by conditioning all or part of a surface that faces inward toward the microfluidic circuit 120) to support cell adhesion, viability and/or growth. The modification may include a coating of a synthetic or natural polymer. In some embodiments, the cover 110 and/or the support structure 104 can be transparent to light. The cover 110 may also include at least one material that is gas permeable (e.g., PDMS or PPS).

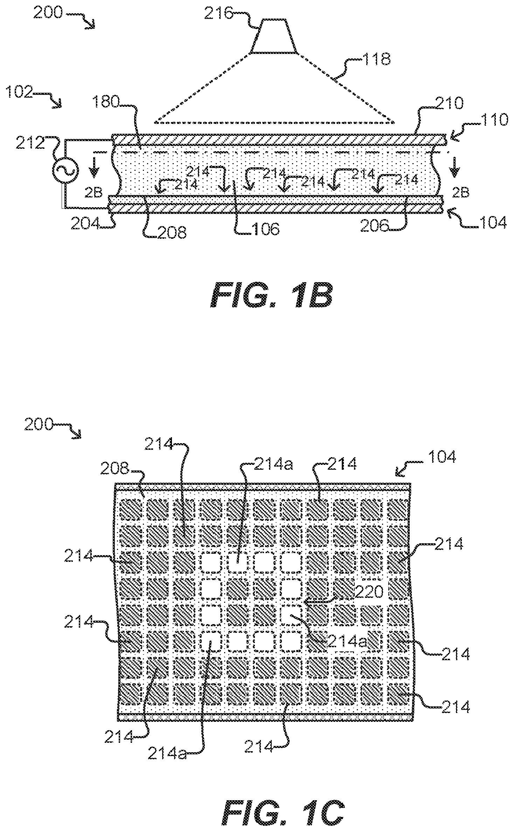

Figure 1A also shows a system 150 for operating and controlling microfluidic devices, such as microfluidic device 100. System 150 includes an electrical power source 192, an imaging device 194 (incorporated within imaging module 164, where device 194 is not illustrated in Figure 1A, per se), and a tilting device 190 (incorporated within tilting module 166, where device 190 is not illustrated in Figure 1).

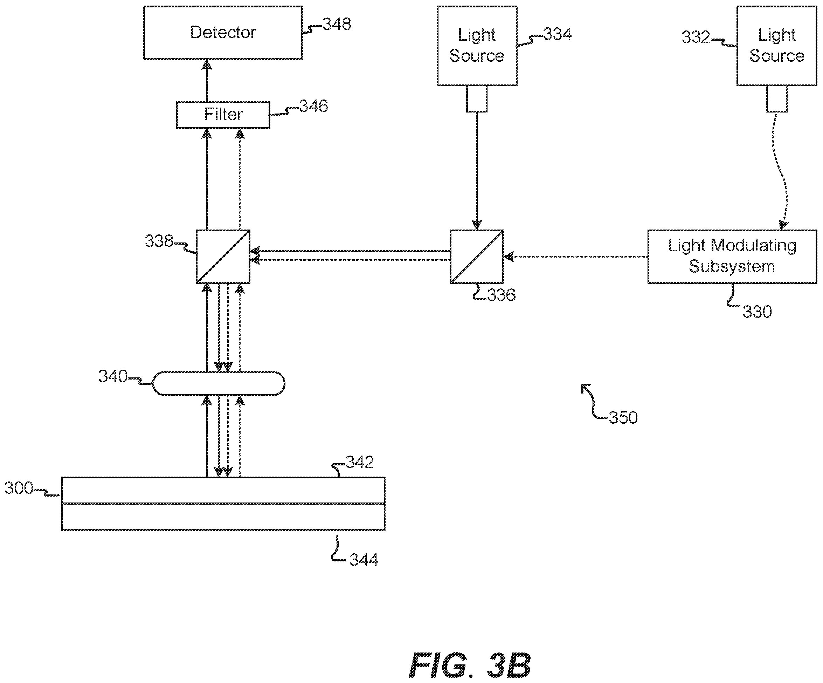

The electrical power source 192 can provide electric power to the microfluidic device 100 and/or tilting device 190, providing biasing voltages or currents as needed. The electrical power source 192 can, for example, comprise one or more alternating current (AC) and/or direct current (DC) voltage or current sources. The imaging device 194 (part of imaging module 164, discussed below) can comprise a device, such as a digital camera, for capturing images inside microfluidic circuit 120. In some instances, the imaging device 194 further comprises a detector having a fast frame rate and/or high sensitivity (e.g. for low light applications). The imaging device 194 can also include a mechanism for directing stimulating radiation and/or light beams into the microfluidic circuit 120 and collecting radiation and/or light beams reflected or emitted from the microfluidic circuit 120 (or micro-objects contained therein). The emitted light beams may be in the visible spectrum and may, e.g., include fluorescent emissions. The reflected light beams may include reflected emissions originating from an LED or a wide spectrum lamp, such as a mercury lamp (e.g. a high pressure mercury lamp) or a Xenon arc lamp. As discussed with respect to Figure 3B, the imaging device 194 may further include a microscope (or an optical train), which may or may not include an eyepiece.

System 150 further comprises a tilting device 190 (part of tilting module 166, discussed below) configured to rotate a microfluidic device 100 about one or more axes of rotation. In some embodiments, the tilting device 190 is configured to support and/or hold the enclosure 102 comprising the microfluidic circuit 120 about at least one axis such that the microfluidic device 100 (and thus the microfluidic circuit 120) can be held in a level orientation (i.e. at 0° relative to x- and y-axes), a vertical orientation (i.e. at 90° relative to the x-axis and/or the y-axis), or any orientation therebetween. The orientation of the microfluidic device 100 (and the microfluidic circuit 120) relative to an axis is referred to herein as the "tilt" of the microfluidic device 100 (and the microfluidic circuit 120). For example, the tilting device 190 can tilt the microfluidic device 100 at 0.1°, 0.2°, 0.3°, 0.4°, 0.5°, 0.6°, 0.7°, 0.8°, 0.9°, 1°, 2°, 3°, 4°, 5°, 10°, 15°, 20°, 25°, 30°, 35°, 40°, 45°, 50°, 55°, 60°, 65°, 70°, 75°, 80°, 90° relative to the x-axis or any degree therebetween. The level orientation (and thus the x- and y-axes) is defined as normal to a vertical axis defined by the force of gravity. The tilting device can also tilt the microfluidic device 100 (and the microfluidic circuit 120) to any degree greater than 90° relative to the x-axis and/or y-axis, or tilt the microfluidic device 100 (and the microfluidic circuit 120) 180° relative to the x-axis or the y-axis in order to fully invert the microfluidic device 100 (and the microfluidic circuit 120). Similarly, in some embodiments, the tilting device 190 tilts the microfluidic device 100 (and the microfluidic circuit 120) about an axis of rotation defined by flow path 106 or some other portion of microfluidic circuit 120.

In some instances, the microfluidic device 100 is tilted into a vertical orientation such that the flow path 106 is positioned above or below one or more sequestration pens. The term "above" as used herein denotes that the flow path 106 is positioned higher than the one or more sequestration pens on a vertical axis defined by the force of gravity (i.e. an object in a sequestration pen above a flow path 106 would have a higher gravitational potential energy than an object in the flow path). The term "below" as used herein denotes that the flow path 106 is positioned lower than the one or more sequestration pens on a vertical axis defined by the force of gravity (i.e. an object in a sequestration pen below a flow path 106 would have a lower gravitational potential energy than an object in the flow path).

In some instances, the tilting device 190 tilts the microfluidic device 100 about an axis that is parallel to the flow path 106. Moreover, the microfluidic device 100 can be tilted to an angle of less than 90° such that the flow path 106 is located above or below one or more sequestration pens without being located directly above or below the sequestration pens. In other instances, the tilting device 190 tilts the microfluidic device 100 about an axis perpendicular to the flow path 106. In still other instances, the tilting device 190 tilts the microfluidic device 100 about an axis that is neither parallel nor perpendicular to the flow path 106.

System 150 can further include a media source 178. The media source 178 (e.g., a container, reservoir, or the like) can comprise multiple sections or containers, each for holding a different fluidic medium 180. Thus, the media source 178 can be a device that is outside of and separate from the microfluidic device 100, as illustrated in Figure 1A. Alternatively, the media source 178 can be located in whole or in part inside the enclosure 102 of the microfluidic device 100. For example, the media source 178 can comprise reservoirs that are part of the microfluidic device 100.

Figure 1A also illustrates simplified block diagram depictions of examples of control and monitoring equipment 152 that constitute part of system 150 and can be utilized in conjunction with a microfluidic device 100. As shown, examples of such control and monitoring equipment 152 include a master controller 154, which can control other control and monitoring equipment, such as a media module 160 for controlling the media source 178, a motive module 162 for controlling movement and/or selection of micro-objects (not shown) and/or medium (e.g., droplets of medium) in the microfluidic circuit 120, an imaging module 164 for controlling an imaging device 194 (e.g., a camera, microscope, light source or any combination thereof) for capturing images (e.g., digital images), and a tilting module 166 for controlling a tilting device 190. The control equipment 152 can also include other modules 168 for controlling, monitoring, or performing other functions with respect to the microfluidic device 100. As shown, the equipment 152 can further include a display device 170 and an input/output device 172.

The master controller 154 can comprise a control module 156 and a digital memory 158. The control module 156 can comprise, for example, a digital processor configured to operate in accordance with machine executable instructions (e.g., software, firmware, source code, or the like) stored as non-transitory data or signals in the memory 158. Alternatively, or in addition, the control module 156 can comprise hardwired digital circuitry and/or analog circuitry. The media module 160, motive module 162, imaging module 164, tilting module 166, and/or other modules 168 can be similarly configured. Thus, functions, processes acts, actions, or steps of a process discussed herein as being performed with respect to the microfluidic device 100 or any other microfluidic apparatus can be performed by any one or more of the master controller 154, media module 160, motive module 162, imaging module 164, tilting module 166, and/or other modules 168 configured as discussed above. Similarly, the master controller 154, media module 160, motive module 162, imaging module 164, tilting module 166, and/or other modules 168 may be communicatively coupled to transmit and receive data used in any function, process, act, action or step discussed herein.

The media module 160 controls the media source 178. For example, the media module 160 can control the media source 178 to input a selected fluidic medium 180 into the enclosure 102 (e.g., through an inlet port 107). The media module 160 can also control removal of media from the enclosure 102 (e.g., through an outlet port (not shown)). One or more media can thus be selectively input into and removed from the microfluidic circuit 120. The media module 160 can also control the flow of fluidic medium 180 in the flow path 106 inside the microfluidic circuit 120. For example, in some embodiments media module 160 stops the flow of media 180 in the flow path 106 and through the enclosure 102 prior to the tilting module 166 causing the tilting device 190 to tilt the microfluidic device 100 to a desired angle of incline.

The motive module 162 can be configured to control selection, trapping, and movement of micro-objects (not shown) in the microfluidic circuit 120. As discussed below with respect to Figures 1B and 1C, the enclosure 102 can comprise a dielectrophoresis (DEP), optoelectronic tweezers (OET), and/or opto-electrowetting (OEW) configuration (not shown in Figure 1A), and the motive module 162 can control the activation of electrodes and/or transistors (e.g., phototransistors) to select and move micro-objects (not shown) and/or droplets of medium (not shown) in the flow path 106 and/or sequestration pens 124, 126, 128, 130.

The imaging module 164 can control the imaging device 194. For example, the imaging module 164 can receive and process image data from the imaging device 194. Image data from the imaging device 194 can comprise any type of information captured by the imaging device 194 (e.g., the presence or absence of micro-objects, droplets of medium, accumulation of label, such as fluorescent label, etc.). Using the information captured by the imaging device 194, the imaging module 164 can further calculate the position of objects (e.g., micro-objects, droplets of medium) and/or the rate of motion of such objects within the microfluidic device 100.

The tilting module 166 can control the tilting motions of tilting device 190. Alternatively, or in addition, the tilting module 166 can control the tilting rate and timing to optimize transfer of micro-objects to the one or more sequestration pens via gravitational forces. The tilting module 166 is communicatively coupled with the imaging module 164 to receive data describing the motion of micro-objects and/or droplets of medium in the microfluidic circuit 120. Using this data, the tilting module 166 may adjust the tilt of the microfluidic circuit 120 in order to adjust the rate at which micro-objects and/or droplets of medium move in the microfluidic circuit 120. The tilting module 166 may also use this data to iteratively adjust the position of a micro-object and/or droplet of medium in the microfluidic circuit 120.

In the example shown in Figure 1A, the microfluidic circuit 120 is illustrated as comprising a microfluidic channel 122 and sequestration pens 124, 126, 128, 130. Each pen comprises a single opening to channel 122, but otherwise is enclosed such that the pens can substantially isolate micro-objects inside the pen from fluidic medium 180 and/or micro-objects in the flow path 106 of channel 122 or in other pens. The walls of the sequestration pen extend from the inner surface 109 of the base to the inside surface of the cover 110 to provide enclosure. The opening of the pen to the channel 122 is oriented at an angle to the flow 106 of fluidic medium 180 such that flow 106 is not directed into the pens. The flow may be tangential or orthogonal to the plane of the opening of the pen. In some instances, pens 124, 126, 128, 130 are configured to physically corral one or more micro-objects within the microfluidic circuit 120. Sequestration pens in accordance with the present invention can comprise various shapes, surfaces and features that are optimized for use with DEP, OET, OEW, fluid flow, and/or gravitational forces, as will be discussed and shown in detail below.

The microfluidic circuit 120 may comprise any number of microfluidic sequestration pens. Although five sequestration pens are shown, microfluidic circuit 120 may have fewer or more sequestration pens. As shown, microfluidic sequestration pens 124, 126, 128, and 130 of microfluidic circuit 120 each comprise differing features and shapes which may provide one or more benefits useful in screening antibody-producing cells, such as isolating one antibody-producing cell from another antibody-producing cell. Microfluidic sequestration pens 124, 126, 128, and 130 may provide other benefits, such as facilitating single-cell loading and/or growth of colonies (e.g., clonal colonies) of antibody-producing cells. In some embodiments, the microfluidic circuit 120 comprises a plurality of identical microfluidic sequestration pens.

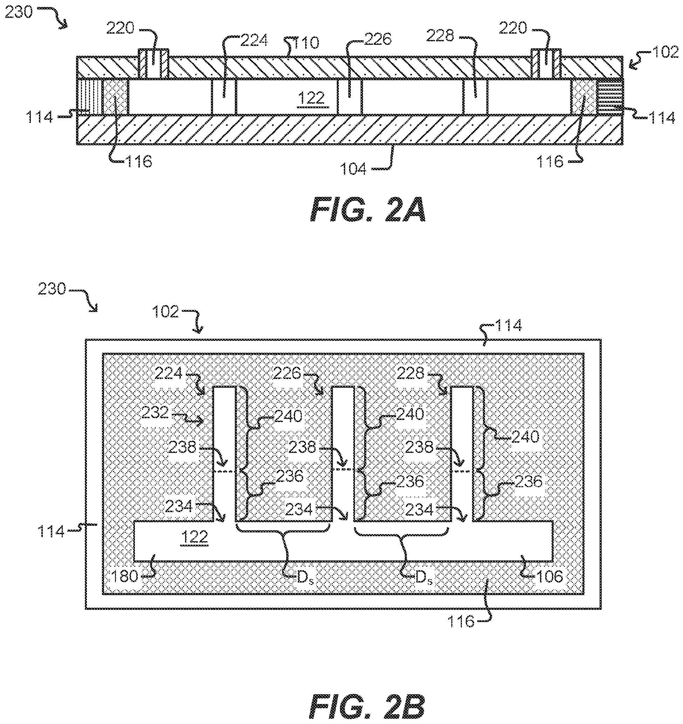

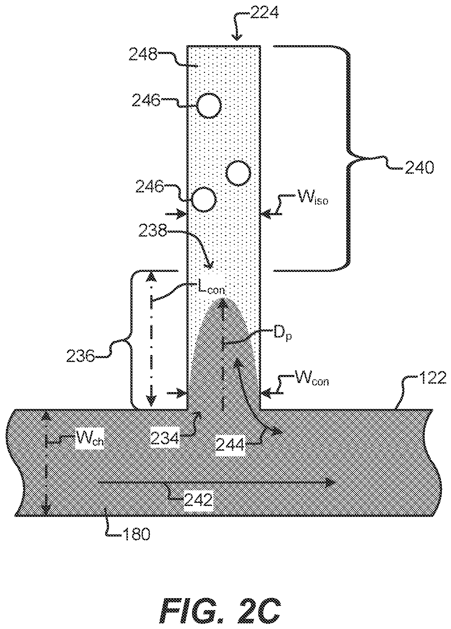

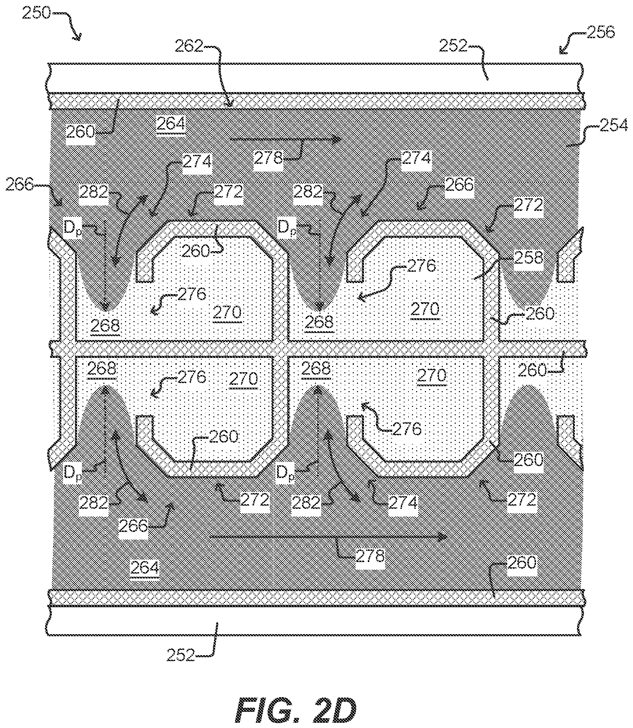

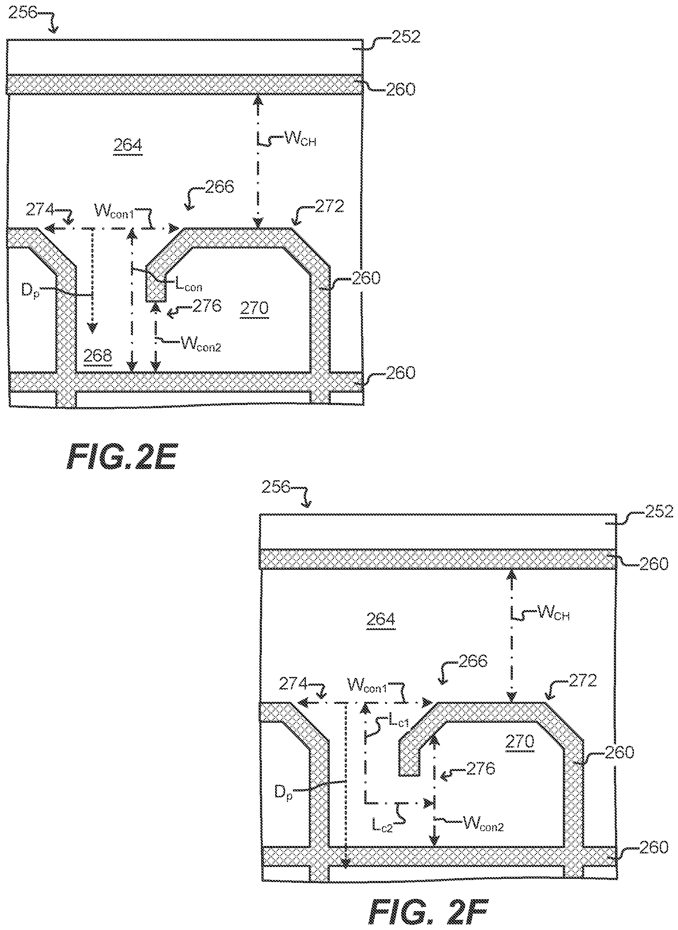

In some embodiments, the microfluidic circuit 120 comprises a plurality of microfluidic sequestration pens, wherein two or more of the sequestration pens comprise differing structures and/or features which provide differing benefits for screening antibody-producing cells. Microfluidic devices useful for screening antibody-producing cells may include any of the sequestration pens 124, 126, 128, and 130 or variations thereof, and/or may include pens configured like those shown in FIGS. 2B, 2C, 2D, 2E and 2F, as discussed below.

In the embodiment illustrated in Figure 1A, a single channel 122 and flow path 106 is shown. However, other embodiments may contain multiple channels 122, each configured to comprise a flow path 106. The microfluidic circuit 120 further comprises an inlet valve or port 107 in fluid communication with the flow path 106 and fluidic medium 180, whereby fluidic medium 180 can access channel 122 via the inlet port 107. In some instances, the flow path 106 comprises a single path. In some instances, the single path is arranged in a zigzag pattern whereby the flow path 106 travels across the microfluidic device 100 two or more times in alternating directions.

In some instances, microfluidic circuit 120 comprises a plurality of parallel channels 122 and flow paths 106, wherein the fluidic medium 180 within each flow path 106 flows in the same direction. In some instances, the fluidic medium within each flow path 106 flows in at least one of a forward or reverse direction. In some instances, a plurality of sequestration pens is configured (e.g., relative to a channel 122) such that the sequestration pens can be loaded with target micro-objects in parallel.

In some embodiments, microfluidic circuit 120 further comprises one or more micro-object traps 132. The traps 132 are generally formed in a wall forming the boundary of a channel 122, and may be positioned opposite an opening of one or more of the microfluidic sequestration pens 124, 126, 128, 130. In some embodiments, the traps 132 are configured to receive or capture a single micro-object from the flow path 106. In some embodiments, the traps 132 are configured to receive or capture a plurality of micro-objects from the flow path 106. In some instances, the traps 132 comprise a volume approximately equal to the volume of a single target micro-object.

The traps 132 may further comprise an opening which is configured to assist the flow of targeted micro-objects into the traps 132. In some instances, the traps 132 comprise an opening having a height and width that is approximately equal to the dimensions of a single target micro-object, whereby larger micro-objects are prevented from entering into the micro-object trap. The traps 132 may further comprise other features configured to assist in retention of targeted micro-objects within the trap 132. In some instances, the trap 132 is aligned with and situated on the opposite side of a channel 122 relative to the opening of a microfluidic sequestration pen, such that upon tilting the microfluidic device 100 about an axis parallel to the channel 122, the trapped micro-object exits the trap 132 at a trajectory that causes the micro-object to fall into the opening of the sequestration pen. In some instances, the trap 132 comprises a side passage 134 that is smaller than the target micro-object in order to facilitate flow through the trap 132 and thereby increase the likelihood of capturing a micro-object in the trap 132.

In some embodiments, dielectrophoretic (DEP) forces are applied across the fluidic medium 180 (e.g., in the flow path and/or in the sequestration pens) via one or more electrodes (not shown) to manipulate, transport, separate and sort micro-objects located therein. For example, in some embodiments, DEP forces are applied to one or more portions of microfluidic circuit 120 in order to transfer a single micro-object from the flow path 106 into a desired microfluidic sequestration pen. In some embodiments, DEP forces are used to prevent a micro-object within a sequestration pen (e.g., sequestration pen 124, 126, 128, or 130) from being displaced therefrom. Further, in some embodiments, DEP forces are used to selectively remove a micro-object from a sequestration pen that was previously collected in accordance with the teachings of the instant invention. In some embodiments, the DEP forces comprise optoelectronic tweezer (OET) forces.

In other embodiments, optoelectrowetting (OEW) forces are applied to one or more positions in the support structure 104 (and/or the cover 110) of the microfluidic device 100 (e.g., positions helping to define the flow path and/or the sequestration pens) via one or more electrodes (not shown) to manipulate, transport, separate and sort droplets located in the microfluidic circuit 120. For example, in some embodiments, OEW forces are applied to one or more positions in the support structure 104 (and/or the cover 110) in order to transfer a single droplet from the flow path 106 into a desired microfluidic sequestration pen. In some embodiments, OEW forces are used to prevent a droplet within a sequestration pen (e.g., sequestration pen 124, 126, 128, or 130) from being displaced therefrom. Further, in some embodiments, OEW forces are used to selectively remove a droplet from a sequestration pen that was previously collected in accordance with the teachings of the instant invention.

In some embodiments, DEP and/or OEW forces are combined with other forces, such as flow and/or gravitational force, so as to manipulate, transport, separate and sort micro-objects and/or droplets within the microfluidic circuit 120. For example, the enclosure 102 can be tilted (e.g., by tilting device 190) to position the flow path 106 and micro-objects located therein above the microfluidic sequestration pens, and the force of gravity can transport the micro-objects and/or droplets into the pens. In some embodiments, the DEP and/or OEW forces can be applied prior to the other forces. In other embodiments, the DEP and/or OEW forces can be applied after the other forces. In still other instances, the DEP and/or OEW forces can be applied at the same time as the other forces or in an alternating manner with the other forces.

Figures 1B, 1C, and 2A-2H illustrates various embodiments of microfluidic devices that can be used in the practice of the present invention. Figure 1B depicts an embodiment in which the microfluidic device 200 is configured as an optically-actuated electrokinetic device. A variety of optically-actuated electrokinetic devices are known in the art, including devices having an optoelectronic tweezer (OET) configuration and devices having an opto-electrowetting (OEW) configuration. Examples of suitable OET configurations are illustrated in the following U.S. patent documents, each of which is incorporated herein by reference in its entirety: U.S. Patent No. RE 44,711 (Wu et al.) (originally issued as U.S. Patent No. 7,612,355); and U.S. Patent No. 7,956,339 (Ohta et al.). Examples of OEW configurations are illustrated in U.S. Patent No. 6,958,132 (Chiou et al.) and U.S. Patent Application Publication No. 2012/0024708 (Chiou et al.), both of which are incorporated by reference herein in their entirety. Yet another example of an optically-actuated electrokinetic device includes a combined OET/OEW configuration, examples of which are shown in U.S. Patent Publication Nos. 20150306598 (Khandros et al.) and 20150306599 (Khandros et al.) and their corresponding PCT Publications WO2015/164846 and WO2015/164847, all of which are incorporated herein by reference in their entirety.

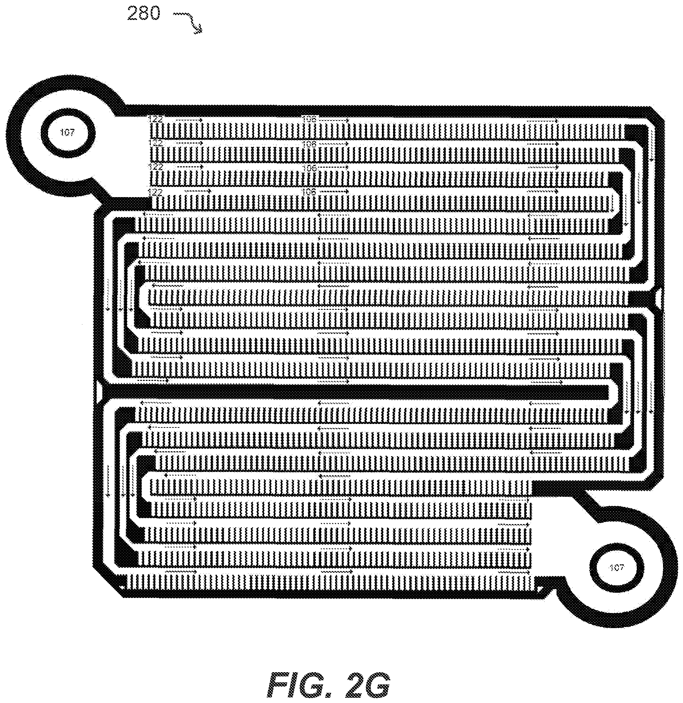

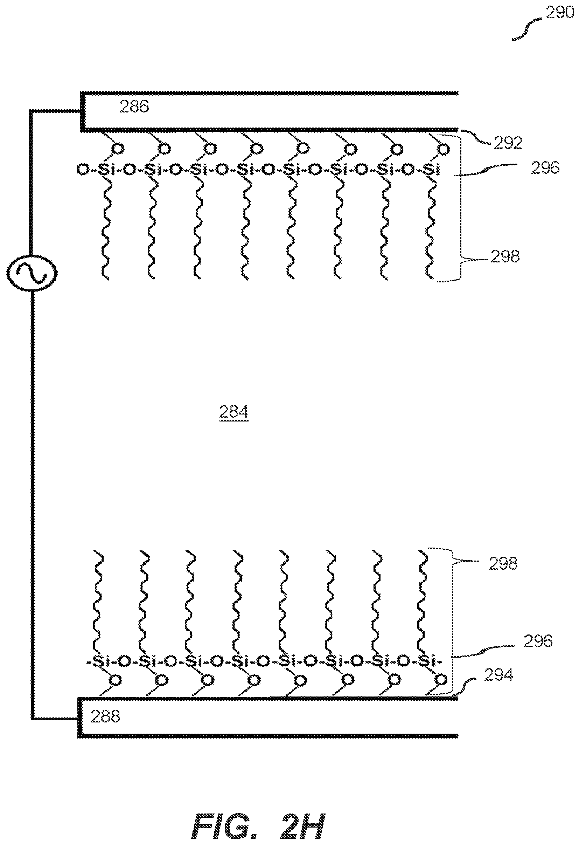

Examples of microfluidic devices having pens in which antibody-producing cells can be placed, cultured, monitored, and/or screened have been described, for example, in US application nos. 14/060,117, 14/520,568 and 14/521,447, each of which is incorporated herein by reference in its entirety. Each of the foregoing applications further describes microfluidic devices configured to produce dielectrophoretic (DEP) forces, such as optoelectronic tweezers (OET) or configured to provide opto-electro wetting (OEW). For example, the optoelectronic tweezers device illustrated in Figure 2 of US application nos. 14/060,117 is an example of a device that can be utilized in embodiments of the present invention to select and move an individual biological micro-object or a group of biological micro-objects.

**Microfluidic device motive configurations.** As described above, the control and monitoring equipment of the system can comprise a motive module for selecting and moving objects, such as micro-objects or droplets, in the microfluidic circuit of a microfluidic device. The microfluidic device can have a variety of motive configurations, depending upon the type of object being moved and other considerations. For example, a dielectrophoresis (DEP) configuration can be utilized to select and move micro-objects in the microfluidic circuit. Thus, the support structure 104 and/or cover 110 of the microfluidic device 100 can comprise a DEP configuration for selectively inducing DEP forces on micro-objects in a fluidic medium 180 in the microfluidic circuit 120 and thereby select, capture, and/or move individual micro-objects or groups of micro-objects. Alternatively, the support structure 104 and/or cover 110 of the microfluidic device 100 can comprise an electrowetting (EW) configuration for selectively inducing EW forces on droplets in a fluidic medium 180 in the microfluidic circuit 120 and thereby select, capture, and/or move individual droplets or groups of droplets.

One example of a microfluidic device 200 comprising a DEP configuration is illustrated in Figures 1B and 1C. While for purposes of simplicity Figures 1B and 1C show a side cross-sectional view and a top cross-sectional view, respectively, of a portion of an enclosure 102 of the microfluidic device 200 having an open region/chamber 202, it should be understood that the region/chamber 202 may be part of a fluidic circuit element having a more detailed structure, such as a growth chamber, a sequestration pen, a flow region, or a flow channel. Furthermore, the microfluidic device 200 may include other fluidic circuit elements. For example, the microfluidic device 200 can include a plurality of growth chambers or sequestration pens and/or one or more flow regions or flow channels, such as those described herein with respect to microfluidic device 100. A DEP configuration may be incorporated into any such fluidic circuit elements of the microfluidic device 200, or select portions thereof. It should be further appreciated that any of the above or below described microfluidic device components and system components may be incorporated in and/or used in combination with the microfluidic device 200. For example, system 150 including control and monitoring equipment 152, described above, may be used with microfluidic device 200, including one or more of the media module 160, motive module 162, imaging module 164, tilting module 166, and other modules 168.

As seen in Figure 1B, the microfluidic device 200 includes a support structure 104 having a bottom electrode 204 and an electrode activation substrate 206 overlying the bottom electrode 204, and a cover 110 having a top electrode 210, with the top electrode 210 spaced apart from the bottom electrode 204. The top electrode 210 and the electrode activation substrate 206 define opposing surfaces of the region/chamber 202. A medium 180 contained in the region/chamber 202 thus provides a resistive connection between the top electrode 210 and the electrode activation substrate 206. A power source 212 configured to be connected to the bottom electrode 204 and the top electrode 210 and create a biasing voltage between the electrodes, as required for the generation of DEP forces in the region/chamber 202, is also shown. The power source 212 can be, for example, an alternating current (AC) power source.

In certain embodiments, the microfluidic device 200 illustrated in Figures 1B and 1C can have an optically-actuated DEP configuration. Accordingly, changing patterns of light 218 from the light source 216, which may be controlled by the motive module 162, can selectively activate and deactivate changing patterns of DEP electrodes at regions 214 of the inner surface 208 of the electrode activation substrate 206. (Hereinafter the regions 214 of a microfluidic device having a DEP configuration are referred to as "DEP electrode regions.") As illustrated in Figure 1C, a light pattern 218 directed onto the inner surface 208 of the electrode activation substrate 206 can illuminate select DEP electrode regions 214a (shown in white) in a pattern, such as a square. The non-illuminated DEP electrode regions 214 (cross-hatched) are hereinafter referred to as "dark" DEP electrode regions 214. The relative electrical impedance through the DEP electrode activation substrate 206 (i.e., from the bottom electrode 204 up to the inner surface 208 of the electrode activation substrate 206 which interfaces with the medium 180 in the flow region 106) is greater than the relative electrical impedance through the medium 180 in the region/chamber 202 (i.e., from the inner surface 208 of the electrode activation substrate 206 to the top electrode 210 of the cover 110) at each dark DEP electrode region 214. An illuminated DEP electrode region 214a, however, exhibits a reduced relative impedance through the electrode activation substrate 206 that is less than the relative impedance through the medium 180 in the region/chamber 202 at each illuminated DEP electrode region 214a.

With the power source 212 activated, the foregoing DEP configuration creates an electric field gradient in the fluidic medium 180 between illuminated DEP electrode regions 214a and adjacent dark DEP electrode regions 214, which in turn creates local DEP forces that attract or repel nearby micro-objects (not shown) in the fluidic medium 180. DEP electrodes that attract or repel micro-objects in the fluidic medium 180 can thus be selectively activated and deactivated at many different such DEP electrode regions 214 at the inner surface 208 of the region/chamber 202 by changing light patterns 218 projected from a light source 216 into the microfluidic device 200. Whether the DEP forces attract or repel nearby micro-objects can depend on such parameters as the frequency of the power source 212 and the dielectric properties of the medium 180 and/or micro-objects (not shown).

The square pattern 220 of illuminated DEP electrode regions 214a illustrated in Figure 1C is an example only. Any pattern of the DEP electrode regions 214 can be illuminated (and thereby activated) by the pattern of light 218 projected into the device 200, and the pattern of illuminated/activated DEP electrode regions 214 can be repeatedly changed by changing or moving the light pattern 218.

In some embodiments, the electrode activation substrate 206 can comprise or consist of a photoconductive material. In such embodiments, the inner surface 208 of the electrode activation substrate 206 can be featureless. For example, the electrode activation substrate 206 can comprise or consist of a layer of hydrogenated amorphous silicon (a-Si:H). The a-Si:H can comprise, for example, about 8% to 40% hydrogen (calculated as 100 * the number of hydrogen atoms / the total number of hydrogen and silicon atoms). The layer of a-Si:H can have a thickness of about 500 nm to about 2.0 µm. In such embodiments, the DEP electrode regions 214 can be created anywhere and in any pattern on the inner surface 208 of the electrode activation substrate 206, in accordance with the light pattern 218. The number and pattern of the DEP electrode regions 214 thus need not be fixed, but can correspond to the light pattern 218. Examples of microfluidic devices having a DEP configuration comprising a photoconductive layer such as discussed above have been described, for example, in U.S. Patent No. RE 44,711 (Wu et al.) (originally issued as U.S. Patent No. 7,612,355), the entire contents of which are incorporated herein by reference.

In other embodiments, the electrode activation substrate 206 can comprise a substrate comprising a plurality of doped layers, electrically insulating layers (or regions), and electrically conductive layers that form semiconductor integrated circuits, such as is known in semiconductor fields. For example, the electrode activation substrate 206 can comprise a plurality of phototransistors, including, for example, lateral bipolar phototransistors, each phototransistor corresponding to a DEP electrode region 214. Alternatively, the electrode activation substrate 206 can comprise electrodes (e.g., conductive metal electrodes) controlled by phototransistor switches, with each such electrode corresponding to a DEP electrode region 214. The electrode activation substrate 206 can include a pattern of such phototransistors or phototransistor-controlled electrodes. The pattern, for example, can be an array of substantially square phototransistors or phototransistor-controlled electrodes arranged in rows and columns, such as shown in Fig. 2B. Alternatively, the pattern can be an array of substantially hexagonal phototransistors or phototransistor-controlled electrodes that form a hexagonal lattice. Regardless of the pattern, electric circuit elements can form electrical connections between the DEP electrode regions 214 at the inner surface 208 of the electrode activation substrate 206 and the bottom electrode 210, and those electrical connections (i.e., phototransistors or electrodes) can be selectively activated and deactivated by the light pattern 218. When not activated, each electrical connection can have high impedance such that the relative impedance through the electrode activation substrate 206 (i.e., from the bottom electrode 204 to the inner surface 208 of the electrode activation substrate 206 which interfaces with the medium 180 in the region/chamber 202) is greater than the relative impedance through the medium 180 (i.e., from the inner surface 208 of the electrode activation substrate 206 to the top electrode 210 of the cover 110) at the corresponding DEP electrode region 214. When activated by light in the light pattern 218, however, the relative impedance through the electrode activation substrate 206 is less than the relative impedance through the medium 180 at each illuminated DEP electrode region 214, thereby activating the DEP electrode at the corresponding DEP electrode region 214 as discussed above. DEP electrodes that attract or repel micro-objects (not shown) in the medium 180 can thus be selectively activated and deactivated at many different DEP electrode regions 214 at the inner surface 208 of the electrode activation substrate 206 in the region/chamber 202 in a manner determined by the light pattern 218.

Examples of microfluidic devices having electrode activation substrates that comprise phototransistors have been described, for example, in U.S. Patent No. 7,956,339 (Ohta et al.) (see, e.g., device 300 illustrated in Figures 21 and 22, and descriptions thereof), the entire contents of which are incorporated herein by reference. Examples of microfluidic devices having electrode activation substrates that comprise electrodes controlled by phototransistor switches have been described, for example, in U.S. Patent Publication No. 2014/0124370 (Short et al.) (see, e.g., devices 200, 400, 500, 600, and 900 illustrated throughout the drawings, and descriptions thereof), the entire contents of which are incorporated herein by reference.

In some embodiments of a DEP configured microfluidic device, the top electrode 210 is part of a first wall (or cover 110) of the enclosure 102, and the electrode activation substrate 206 and bottom electrode 204 are part of a second wall (or support structure 104) of the enclosure 102. The region/chamber 202 can be between the first wall and the second wall. In other embodiments, the electrode 210 is part of the second wall (or support structure 104) and one or both of the electrode activation substrate 206 and/or the electrode 210 are part of the first wall (or cover 110). Moreover, the light source 216 can alternatively be used to illuminate the enclosure 102 from below.

With the microfluidic device 200 of Figures 1B-1C having a DEP configuration, the motive module 162 can select a micro-object (not shown) in the medium 180 in the region/chamber 202 by projecting a light pattern 218 into the device 200 to activate a first set of one or more DEP electrodes at DEP electrode regions 214a of the inner surface 208 of the electrode activation substrate 206 in a pattern (e.g., square pattern 220) that surrounds and captures the micro-object. The motive module 162 can then move the captured micro-object by moving the light pattern 218 relative to the device 200 to activate a second set of one or more DEP electrodes at DEP electrode regions 214. Alternatively, the device 200 can be moved relative to the light pattern 218.

In other embodiments, the microfluidic device 200 can have a DEP configuration that does not rely upon light activation of DEP electrodes at the inner surface 208 of the electrode activation substrate 206. For example, the electrode activation substrate 206 can comprise selectively addressable and energizable electrodes positioned opposite to a surface including at least one electrode (e.g., cover 110). Switches (e.g., transistor switches in a semiconductor substrate) may be selectively opened and closed to activate or inactivate DEP electrodes at DEP electrode regions 214, thereby creating a net DEP force on a micro-object (not shown) in region/chamber 202 in the vicinity of the activated DEP electrodes. Depending on such characteristics as the frequency of the power source 212 and the dielectric properties of the medium (not shown) and/or micro-objects in the region/chamber 202, the DEP force can attract or repel a nearby micro-object. By selectively activating and deactivating a set of DEP electrodes (e.g., at a set of DEP electrodes regions 214 that forms a square pattern 220), one or more micro-objects in region/chamber 202 can be trapped and moved within the region/chamber 202. The motive module 162 in Figure 1A can control such switches and thus activate and deactivate individual ones of the DEP electrodes to select, trap, and move particular micro-objects (not shown) around the region/chamber 202. Microfluidic devices having a DEP configuration that includes selectively addressable and energizable electrodes are known in the art and have been described, for example, in U.S. Patent Nos. 6,294,063 (Becker et al.) and 6,942,776 (Medoro), the entire contents of which are incorporated herein by reference.

As yet another example, the microfluidic device 200 can have an electrowetting (EW) configuration, which can be in place of the DEP configuration or can be located in a portion of the microfluidic device 200 that is separate from the portion which has the DEP configuration. The EW configuration can be an opto-electrowetting configuration or an electrowetting on dielectric (EWOD) configuration, both of which are known in the art. In some EW configurations, the support structure 104 has an electrode activation substrate 206 sandwiched between a dielectric layer (not shown) and the bottom electrode 204. The dielectric layer can comprise a hydrophobic material and/or can be coated with a hydrophobic material. For microfluidic devices 200 that have an EW configuration, the inner surface 208 of the support structure 104 is the inner surface of the dielectric layer or its hydrophobic coating.

The dielectric layer (not shown) can comprise one or more oxide layers, and can have a thickness of about 50 nm to about 250 nm (e.g., about 125 nm to about 175 nm). In certain embodiments, the dielectric layer may comprise a layer of oxide, such as a metal oxide (e.g., aluminum oxide or hafnium oxide). In certain embodiments, the dielectric layer can comprise a dielectric material other than a metal oxide, such as silicon oxide or a nitride. Regardless of the exact composition and thickness, the dielectric layer can have an impedance of about 10 kOhms to about 50 kOhms.

In some embodiments, the surface of the dielectric layer that faces inward toward region/chamber 202 is coated with a hydrophobic material. The hydrophobic material can comprise, for example, fluorinated carbon molecules. Examples of fluorinated carbon molecules include perfluoro-polymers such as polytetrafluoroethylene (e.g., TEFLON^{®}) or poly(2,3-difluoromethylenyl-perfluorotetrahydrofuran) (e.g., CYTOP^{™}). Molecules that make up the hydrophobic material can be covalently bonded to the surface of the dielectric layer. For example, molecules of the hydrophobic material can be covalently bound to the surface of the dielectric layer by means of a linker such as a siloxane group, a phosphonic acid group, or a thiol group. Thus, in some embodiments, the hydrophobic material can comprise alkyl-terminated siloxane, alkyl-termination phosphonic acid, or alkyl-terminated thiol. The alkyl group can be long-chain hydrocarbons (e.g., having a chain of at least 10 carbons, or at least 16, 18, 20, 22, or more carbons). Alternatively, fluorinated (or perfluorinated) carbon chains can be used in place of the alkyl groups. Thus, for example, the hydrophobic material can comprise fluoroalkyl-terminated siloxane, fluoroalkyl-terminated phosphonic acid, or fluoroalkyl-terminated thiol. In some embodiments, the hydrophobic coating has a thickness of about 10 nm to about 50 nm. In other embodiments, the hydrophobic coating has a thickness of less than 10 nm (e.g., less than 5 nm, or about 1.5 to 3.0 nm).

In some embodiments, the cover 110 of a microfluidic device 200 having an electrowetting configuration is coated with a hydrophobic material (not shown) as well. The hydrophobic material can be the same hydrophobic material used to coat the dielectric layer of the support structure 104, and the hydrophobic coating can have a thickness that is substantially the same as the thickness of the hydrophobic coating on the dielectric layer of the support structure 104. Moreover, the cover 110 can comprise an electrode activation substrate 206 sandwiched between a dielectric layer and the top electrode 210, in the manner of the support structure 104. The electrode activation substrate 206 and the dielectric layer of the cover 110 can have the same composition and/or dimensions as the electrode activation substrate 206 and the dielectric layer of the support structure 104. Thus, the microfluidic device 200 can have two electrowetting surfaces.

In some embodiments, the electrode activation substrate 206 can comprise a photoconductive material, such as described above. Accordingly, in certain embodiments, the electrode activation substrate 206 can comprise or consist of a layer of hydrogenated amorphous silicon (a-Si:H). The a-Si:H can comprise, for example, about 8% to 40% hydrogen (calculated as 100 * the number of hydrogen atoms / the total number of hydrogen and silicon atoms). The layer of a-Si:H can have a thickness of about 500 nm to about 2.0 µm. Alternatively, the electrode activation substrate 206 can comprise electrodes (e.g., conductive metal electrodes) controlled by phototransistor switches, as described above. Microfluidic devices having an optoelectrowetting configuration are known in the art and/or can be constructed with electrode activation substrates known in the art. For example, U.S. Patent No. 6,958,132 (Chiou et al.), the entire contents of which are incorporated herein by reference, discloses opto-electrowetting configurations having a photoconductive material such as a-Si:H, while U.S. Patent Publication No. 2014/0124370 (Short et al.), referenced above, discloses electrode activation substrates having electrodes controlled by phototransistor switches.

The microfluidic device 200 thus can have an opto-electrowetting configuration, and light patterns 218 can be used to activate photoconductive EW regions or photoresponsive EW electrodes in the electrode activation substrate 206. Such activated EW regions or EW electrodes of the electrode activation substrate 206 can generate an electrowetting force at the inner surface 208 of the support structure 104 (i.e., the inner surface of the overlaying dielectric layer or its hydrophobic coating). By changing the light patterns 218 (or moving microfluidic device 200 relative to the light source 216) incident on the electrode activation substrate 206, droplets (e.g., containing an aqueous medium, solution, or solvent) contacting the inner surface 208 of the support structure 104 can be moved through an immiscible fluid (e.g., an oil medium) present in the region/chamber 202.

In other embodiments, microfluidic devices 200 can have an EWOD configuration, and the electrode activation substrate 206 can comprise selectively addressable and energizable electrodes that do not rely upon light for activation. The electrode activation substrate 206 thus can include a pattern of such electrowetting (EW) electrodes. The pattern, for example, can be an array of substantially square EW electrodes arranged in rows and columns, such as shown in Fig. 2B. Alternatively, the pattern can be an array of substantially hexagonal EW electrodes that form a hexagonal lattice. Regardless of the pattern, the EW electrodes can be selectively activated (or deactivated) by electrical switches (e.g., transistor switches in a semiconductor substrate). By selectively activating and deactivating EW electrodes in the electrode activation substrate 206, droplets (not shown) contacting the inner surface 208 of the overlaying dielectric layer or its hydrophobic coating can be moved within the region/chamber 202. The motive module 162 in Figure 1A can control such switches and thus activate and deactivate individual EW electrodes to select and move particular droplets around region/chamber 202. Microfluidic devices having a EWOD configuration with selectively addressable and energizable electrodes are known in the art and have been described, for example, in U.S. Patent No. 8,685,344 (Sundarsan et al.), the entire contents of which are incorporated herein by reference.

Regardless of the configuration of the microfluidic device 200, a power source 212 can be used to provide a potential (e.g., an AC voltage potential) that powers the electrical circuits of the microfluidic device 200. The power source 212 can be the same as, or a component of, the power source 192 referenced in Fig. 1. Power source 212 can be configured to provide an AC voltage and/or current to the top electrode 210 and the bottom electrode 204. For an AC voltage, the power source 212 can provide a frequency range and an average or peak power (e.g., voltage or current) range sufficient to generate net DEP forces (or electrowetting forces) strong enough to trap and move individual micro-objects (not shown) in the region/chamber 202, as discussed above, and/or to change the wetting properties of the inner surface 208 of the support structure 104 (i.e., the dielectric layer and/or the hydrophobic coating on the dielectric layer) in the region/chamber 202, as also discussed above. Such frequency ranges and average or peak power ranges are known in the art. See, e.g., US Patent No. 6,958,132 (Chiou et al.), US Patent No. RE44,711 (Wu et al.) (originally issued as US Patent No. 7,612,355), and US Patent Application Publication Nos. US2014/0124370 (Short et al.), US2015/0306598 (Khandros et al.), and US2015/0306599 (Khandros et al.).

**Sequestration pens.** Non-limiting examples of generic sequestration pens 224, 226, and 228 are shown within the microfluidic device 230 depicted in Figures 2A-2C. Each sequestration pen 224, 226, and 228 can comprise an isolation structure 232 defining an isolation region 240 and a connection region 236 fluidically connecting the isolation region 240 to a channel 122. The connection region 236 can comprise a proximal opening 234 to the channel 122 and a distal opening 238 to the isolation region 240. The connection region 236 can be configured so that the maximum penetration depth of a flow of a fluidic medium (not shown) flowing from the channel 122 into the sequestration pen 224, 226, 228 does not extend into the isolation region 240. Thus, due to the connection region 236, a micro-object (not shown) or other material (not shown) disposed in an isolation region 240 of a sequestration pen 224, 226, 228 can thus be isolated from, and not substantially affected by, a flow of medium 180 in the channel 122.

The sequestration pens 224, 226, and 228 of Figures 2A-2C each have a single opening which opens directly to the channel 122. The opening of the sequestration pen opens laterally from the channel 122. The electrode activation substrate 206 underlays both the channel 122 and the sequestration pens 224, 226, and 228. The upper surface of the electrode activation substrate 206 within the enclosure of a sequestration pen, forming the floor of the sequestration pen, is disposed at the same level or substantially the same level of the upper surface the of electrode activation substrate 206 within the channel 122 (or flow region if a channel is not present), forming the floor of the flow channel (or flow region, respectively) of the microfluidic device. The electrode activation substrate 206 may be featureless or may have an irregular or patterned surface that varies from its highest elevation to its lowest depression by less than about 3 microns, 2.5 microns, 2 microns, 1.5 microns, 1 micron, 0.9 microns, 0.8 microns, 0.7 microns, 0.6 microns, 0.5 microns, 0.4 microns, 0.3 microns, 0.2 microns, 0.1 microns, or less. The variation of elevation in the upper surface of the substrate across both the channel 122 (or flow region) and sequestration pens may be less than about 3%, 2%, 1%. 0.9%, 0.8%, 0.5%, 0.3% or 0.1% of the height of the walls of the sequestration pen or walls of the microfluidic device. While described in detail for the microfluidic device 200, this also applies to any of the microfluidic devices 100, 230, 250, 280, 290 described herein.

The channel 122 can thus be an example of a swept region, and the isolation regions 240 of the sequestration pens 224, 226, 228 can be examples of unswept regions. As noted, the channel 122 and sequestration pens 224, 226, 228 can be configured to contain one or more fluidic media 180. In the example shown in Figures 2A-2B, the ports 222 are connected to the channel 122 and allow a fluidic medium 180 to be introduced into or removed from the microfluidic device 230. Prior to introduction of the fluidic medium 180, the microfluidic device may be primed with a gas such as carbon dioxide gas. Once the microfluidic device 230 contains the fluidic medium 180, the flow 242 of fluidic medium 180 in the channel 122 can be selectively generated and stopped. For example, as shown, the ports 222 can be disposed at different locations (e.g., opposite ends) of the channel 122, and a flow 242 of medium can be created from one port 222 functioning as an inlet to another port 222 functioning as an outlet.

Figure 2C illustrates a detailed view of an example of a sequestration pen 224 according to the present invention. Examples of micro-objects 246 are also shown.

As is known, a flow 242 of fluidic medium 180 in a microfluidic channel 122 past a proximal opening 234 of sequestration pen 224 can cause a secondary flow 244 of the medium 180 into and/or out of the sequestration pen 224. To isolate micro-objects 246 in the isolation region 240 of a sequestration pen 224 from the secondary flow 244, the length L_{con} of the connection region 236 of the sequestration pen 224 (i.e., from the proximal opening 234 to the distal opening 238) should be greater than the penetration depth Dₚ of the secondary flow 244 into the connection region 236. The penetration depth Dₚ of the secondary flow 244 depends upon the velocity of the fluidic medium 180 flowing in the channel 122 and various parameters relating to the configuration of the channel 122 and the proximal opening 234 of the connection region 236 to the channel 122. For a given microfluidic device, the configurations of the channel 122 and the opening 234 will be fixed, whereas the rate of flow 242 of fluidic medium 180 in the channel 122 will be variable. Accordingly, for each sequestration pen 224, a maximal velocity Vₘₐₓ for the flow 242 of fluidic medium 180 in channel 122 can be identified that ensures that the penetration depth Dₚ of the secondary flow 244 does not exceed the length L_{con} of the connection region 236. As long as the rate of the flow 242 of fluidic medium 180 in the channel 122 does not exceed the maximum velocity Vₘₐₓ, the resulting secondary flow 244 can be limited to the channel 122 and the connection region 236 and kept out of the isolation region 240. The flow 242 of medium 180 in the channel 122 will thus not draw micro-objects 246 out of the isolation region 240. Rather, micro-objects 246 located in the isolation region 240 will stay in the isolation region 240 regardless of the flow 242 of fluidic medium 180 in the channel 122.

Moreover, as long as the rate of flow 242 of medium 180 in the channel 122 does not exceed Vₘₐₓ, the flow 242 of fluidic medium 180 in the channel 122 will not move miscellaneous particles (e.g., microparticles and/or nanoparticles) from the channel 122 into the isolation region 240 of a sequestration pen 224. Having the length L_{con} of the connection region 236 be greater than the maximum penetration depth Dₚ of the secondary flow 244 can thus prevent contamination of one sequestration pen 224 with miscellaneous particles from the channel 122 or another sequestration pen (e.g., sequestration pens 226, 228 in Fig. 2D).

Because the channel 122 and the connection regions 236 of the sequestration pens 224, 226, 228 can be affected by the flow 242 of medium 180 in the channel 122, the channel 122 and connection regions 236 can be deemed swept (or flow) regions of the microfluidic device 230. The isolation regions 240 of the sequestration pens 224, 226, 228, on the other hand, can be deemed unswept (or non-flow) regions. For example, components (not shown) in a first fluidic medium 180 in the channel 122 can mix with a second fluidic medium 248 in the isolation region 240 substantially only by diffusion of components of the first medium 180 from the channel 122 through the connection region 236 and into the second fluidic medium 248 in the isolation region 240. Similarly, components (not shown) of the second medium 248 in the isolation region 240 can mix with the first medium 180 in the channel 122 substantially only by diffusion of components of the second medium 248 from the isolation region 240 through the connection region 236 and into the first medium 180 in the channel 122. In some embodiments, the extent of fluidic medium exchange between the isolation region of a sequestration pen and the flow region by diffusion is about 90%, 91%, 92%, 93%, 94% 95%, 96%, 97%, 98%, 99%, or greater than the amount of total fluidic exchange. The first medium 180 can be the same medium or a different medium than the second medium 248. Moreover, the first medium 180 and the second medium 248 can start out being the same, then become different (e.g., through conditioning of the second medium 248 by one or more cells in the isolation region 240, or by changing the medium 180 flowing through the channel 122).

The maximum penetration depth Dₚ of the secondary flow 244 caused by the flow 242 of fluidic medium 180 in the channel 122 can depend on a number of parameters, as mentioned above. Examples of such parameters include: the shape of the channel 122 (e.g., the channel can direct medium into the connection region 236, divert medium away from the connection region 236, or direct medium in a direction substantially perpendicular to the proximal opening 234 of the connection region 236 to the channel 122); a width W_{ch} (or cross-sectional area) of the channel 122 at the proximal opening 234; and a width W_{con} (or cross-sectional area) of the connection region 236 at the proximal opening 234; the velocity V of the flow 242 of fluidic medium 180 in the channel 122; the viscosity of the first medium 180 and/or the second medium 248, or the like.

In some embodiments, the dimensions of the channel 122 and sequestration pens 224, 226, 228 can be oriented as follows with respect to the vector of the flow 242 of fluidic medium 180 in the channel 122: the channel width W_{ch} (or cross-sectional area of the channel 122) can be substantially perpendicular to the flow 242 of medium 180; the width W_{con} (or cross-sectional area) of the connection region 236 at opening 234 can be substantially parallel to the flow 242 of medium 180 in the channel 122; and/or the length L_{con} of the connection region can be substantially perpendicular to the flow 242 of medium 180 in the channel 122. The foregoing are examples only, and the relative position of the channel 122 and sequestration pens 224, 226, 228 can be in other orientations with respect to each other.

As illustrated in Figure 2C, the width W_{con} of the connection region 236 can be uniform from the proximal opening 234 to the distal opening 238. The width W_{con} of the connection region 236 at the distal opening 238 can thus be in any of the ranges identified herein for the width W_{con} of the connection region 236 at the proximal opening 234. Alternatively, the width W_{con} of the connection region 236 at the distal opening 238 can be larger than the width W_{con} of the connection region 236 at the proximal opening 234.

As illustrated in Figure 2C, the width Wiso of the isolation region 240 at the distal opening 238 can be substantially the same as the width W_{con} of the connection region 236 at the proximal opening 234. The width Wiso of the isolation region 240 at the distal opening 238 can thus be in any of the ranges identified herein for the width W_{con} of the connection region 236 at the proximal opening 234. Alternatively, the width Wiso of the isolation region 240 at the distal opening 238 can be larger or smaller than the width W_{con} of the connection region 236 at the proximal opening 234. Moreover, the distal opening 238 may be smaller than the proximal opening 234 and the width W_{con} of the connection region 236 may be narrowed between the proximal opening 234 and distal opening 238. For example, the connection region 236 may be narrowed between the proximal opening and the distal opening, using a variety of different geometries (e.g. chamfering the connection region, beveling the connection region). Further, any part or subpart of the connection region 236 may be narrowed (e.g. a portion of the connection region adjacent to the proximal opening 234).

Figures 2D-2F depict another exemplary embodiment of a microfluidic device 250 containing a microfluidic circuit 262 and flow channels 264, which are variations of the respective microfluidic device 100, circuit 132 and channel 134 of Figure 1. The microfluidic device 250 also has a plurality of sequestration pens 266 that are additional variations of the above-described sequestration pens 124, 126, 128, 130, 224, 226 or 228. In particular, it should be appreciated that the sequestration pens 266 of device 250 shown in Figures 2D-2F can replace any of the above-described sequestration pens 124, 126, 128, 130, 224, 226 or 228 in devices 100, 200, 230, 280, 290, or 320. Likewise, the microfluidic device 250 is another variant of the microfluidic device 100, and may also have the same or a different DEP configuration as the above-described microfluidic device 100, 200, 230, 280, 290, 320 as well as any of the other microfluidic system components described herein.

The microfluidic device 250 of Figures 2D-2F comprises a support structure (not visible in Figures 2D-2F, but can be the same or generally similar to the support structure 104 of device 100 depicted in Figure 1A), a microfluidic circuit structure 256, and a cover (not visible in Figures 2D-2F, but can be the same or generally similar to the cover 122 of device 100 depicted in Figure 1A). The microfluidic circuit structure 256 includes a frame 252 and microfluidic circuit material 260, which can be the same as or generally similar to the frame 114 and microfluidic circuit material 116 of device 100 shown in Figure 1A. As shown in Figure 2D, the microfluidic circuit 262 defined by the microfluidic circuit material 260 can comprise multiple channels 264 (two are shown but there can be more) to which multiple sequestration pens 266 are fluidically connected.

Each sequestration pen 266 can comprise an isolation structure 272, an isolation region 270 within the isolation structure 272, and a connection region 268. From a proximal opening 274 at the channel 264 to a distal opening 276 at the isolation structure 272, the connection region 268 fluidically connects the channel 264 to the isolation region 270. Generally, in accordance with the above discussion of Figures 2B and 2C, a flow 278 of a first fluidic medium 254 in a channel 264 can create secondary flows 282 of the first medium 254 from the channel 264 into and/or out of the respective connection regions 268 of the sequestration pens 266.

As illustrated in Figure 2E, the connection region 268 of each sequestration pen 266 generally includes the area extending between the proximal opening 274 to a channel 264 and the distal opening 276 to an isolation structure 272. The length L_{con} of the connection region 268 can be greater than the maximum penetration depth Dₚ of secondary flow 282, in which case the secondary flow 282 will extend into the connection region 268 without being redirected toward the isolation region 270 (as shown in Figure 2D). Alternatively, at illustrated in Figure 2F, the connection region 268 can have a length L_{con} that is less than the maximum penetration depth Dₚ, in which case the secondary flow 282 will extend through the connection region 268 and be redirected toward the isolation region 270. In this latter situation, the sum of lengths L_{c1} and L_{c2} of connection region 268 is greater than the maximum penetration depth Dₚ, so that secondary flow 282 will not extend into isolation region 270. Whether length L_{con} of connection region 268 is greater than the penetration depth Dₚ, or the sum of lengths L_{c1} and L_{c2} of connection region 268 is greater than the penetration depth Dₚ, a flow 278 of a first medium 254 in channel 264 that does not exceed a maximum velocity Vₘₐₓ will produce a secondary flow having a penetration depth Dₚ, and micro-objects (not shown but can be the same or generally similar to the micro-objects 246 shown in Figure 2C) in the isolation region 270 of a sequestration pen 266 will not be drawn out of the isolation region 270 by a flow 278 of first medium 254 in channel 264. Nor will the flow 278 in channel 264 draw miscellaneous materials (not shown) from channel 264 into the isolation region 270 of a sequestration pen 266. As such, diffusion is the only mechanism by which components in a first medium 254 in the channel 264 can move from the channel 264 into a second medium 258 in an isolation region 270 of a sequestration pen 266. Likewise, diffusion is the only mechanism by which components in a second medium 258 in an isolation region 270 of a sequestration pen 266 can move from the isolation region 270 to a first medium 254 in the channel 264. The first medium 254 can be the same medium as the second medium 258, or the first medium 254 can be a different medium than the second medium 258. Alternatively, the first medium 254 and the second medium 258 can start out being the same, then become different, e.g., through conditioning of the second medium by one or more cells in the isolation region 270, or by changing the medium flowing through the channel 264.

As illustrated in Figure 2E, the width W_{ch} of the channels 264 (i.e., taken transverse to the direction of a fluid medium flow through the channel indicated by arrows 278 in Figure 2D) in the channel 264 can be substantially perpendicular to a width W_{con1} of the proximal opening 274 and thus substantially parallel to a width W_{con2} of the distal opening 276. The width W_{con1} of the proximal opening 274 and the width W_{con2} of the distal opening 276, however, need not be substantially perpendicular to each other. For example, an angle between an axis (not shown) on which the width W_{con1} of the proximal opening 274 is oriented and another axis on which the width W_{con2} of the distal opening 276 is oriented can be other than perpendicular and thus other than 90°. Examples of alternatively oriented angles include angles in any of the following ranges: from about 30° to about 90°, from about 45° to about 90°, from about 60° to about 90°, or the like.

In various embodiments of sequestration pens (e.g. 124, 126, 128, 130, 224, 226, 228, or 266), the isolation region (e.g. 240 or 270) is configured to contain a plurality of micro-objects. In other embodiments, the isolation region can be configured to contain only one, two, three, four, five, or a similar relatively small number of micro-objects. Accordingly, the volume of an isolation region can be, for example, at least 5x10⁵, 8x10⁵, 1x10⁶, 2x10⁶, 4x10⁶, 6x10⁶ cubic microns, or more.

In various embodiments of sequestration pens, the width W_{ch} of the channel (e.g., 122) at a proximal opening (e.g. 234) can be within any of the following ranges: about 50-1000 microns, 50-500 microns, 50-400 microns, 50-300 microns, 50-250 microns, 50-200 microns, 50-150 microns, 50-100 microns, 70-500 microns, 70-400 microns, 70-300 microns, 70-250 microns, 70-200 microns, 70-150 microns, 90-400 microns, 90-300 microns, 90-250 microns, 90-200 microns, 90-150 microns, 100-300 microns, 100-250 microns, 100-200 microns, 100-150 microns, and 100-120 microns. In some other embodiments, the width W_{ch} of the channel (e.g., 122) at a proximal opening (e.g. 234) can be in a range of about 200-800 microns, 200-700 microns, or 200-600 microns. The foregoing are examples only, and the width W_{ch} of the channel 122 can be in other ranges (e.g., a range defined by any of the endpoints listed above). Moreover, the W_{ch} of the channel 122 can be selected to be in any of these ranges in regions of the channel other than at a proximal opening of a sequestration pen.

In some embodiments, a sequestration pen has a height of about 30 to about 200 microns, or about 50 to about 150 microns. In some embodiments, the sequestration pen has a cross-sectional area of about 1 x 10⁴ to about 3 x 10⁶ square microns, about 2 x 10⁴ to about 2 x 10⁶ square microns, about 4 x 10⁴ to about 1 x 10⁶ square microns, about 2 x 10⁴ to about 5 x 10⁵ square microns, about 2 x 10⁴ to about 1 x 10⁵ square microns, or about 2 x 10⁵ to about 2 x 10⁶ square microns. In some embodiments, the connection region has a cross-sectional width of about 20 to about 100 microns, about 30 to about 80 microns or about 40 to about 60 microns.

In various embodiments of sequestration pens, the height H_{ch} of the channel (e.g., 122) at a proximal opening (e.g., 234) can be within any of the following ranges: 20-100 microns, 20-90 microns, 20-80 microns, 20-70 microns, 20-60 microns, 20-50 microns, 30-100 microns, 30-90 microns, 30-80 microns, 30-70 microns, 30-60 microns, 30-50 microns, 40-100 microns, 40-90 microns, 40-80 microns, 40-70 microns, 40-60 microns, or 40-50 microns. The foregoing are examples only, and the height H_{ch} of the channel (e.g., 122) can be in other ranges (e.g., a range defined by any of the endpoints listed above). The height H_{ch} of the channel 122 can be selected to be in any of these ranges in regions of the channel other than at a proximal opening of an sequestration pen.

In various embodiments of sequestration pens a cross-sectional area of the channel (e.g., 122) at a proximal opening (e.g., 234) can be within any of the following ranges: 500-50,000 square microns, 500-40,000 square microns, 500-30,000 square microns, 500-25,000 square microns, 500-20,000 square microns, 500-15,000 square microns, 500-10,000 square microns, 500-7,500 square microns, 500-5,000 square microns, 1,000-25,000 square microns, 1,000-20,000 square microns, 1,000-15,000 square microns, 1,000-10,000 square microns, 1,000-7,500 square microns, 1,000-5,000 square microns, 2,000-20,000 square microns, 2,000-15,000 square microns, 2,000-10,000 square microns, 2,000-7,500 square microns, 2,000-6,000 square microns, 3,000-20,000 square microns, 3,000-15,000 square microns, 3,000-10,000 square microns, 3,000-7,500 square microns, or 3,000 to 6,000 square microns. The foregoing are examples only, and the cross-sectional area of the channel (e.g., 122) at a proximal opening (e.g., 234) can be in other ranges (e.g., a range defined by any of the endpoints listed above).

In various embodiments of sequestration pens, the length L_{con} of the connection region (e.g., 236) can be in any of the following ranges: about 20 to about 300 microns, about 40 to about 250 microns, about 60 to about 200 microns, about 80 to about 150 microns, about 20 to about 500 microns, about 40 to about 400 microns, about 60 to about 300 microns, about 80 to about 200 microns, or about 100 to about 150 microns. The foregoing are examples only, and length L_{con} of a connection region (e.g., 236) can be in a different range than the foregoing examples (e.g., a range defined by any of the endpoints listed above).

In various embodiments of sequestration pens the width W_{con} of a connection region (e.g., 236) at a proximal opening (e.g., 234) can be in any of the following ranges: about 20 to about 150 microns, about 20 to about 100 microns, about 20 to about 80 microns, about 20 to about 60 microns, about 30 to about 150 microns, about 30 to about 100 microns, about 30 to about 80 microns, about 30 to about 60 microns, about 40 to about 150 microns, about 40 to about 100 microns, about 40 to about 80 microns, about 40 to about 60 microns, about 50 to about 150 microns, about 50 to about 100 microns, about 50 to about 80 microns, about 60 to about 150 microns, about 60 to about 100 microns, about 60 to about 80 microns, about 70 to about 150 microns, about 70 to about 100 microns, about 80 to about 150 microns, and about 80 to about 100 microns. The foregoing are examples only, and the width W_{con} of a connection region (e.g., 236) at a proximal opening (e.g., 234) can be different than the foregoing examples (e.g., a range defined by any of the endpoints listed above).

In various embodiments of sequestration pens, the width W_{con} of a connection region (e.g., 236) at a proximal opening (e.g., 234) can be at least as large as the largest dimension of a micro-object (e.g., a biological cell, which may be a immunological cell, such as B cell or a T cell, or a hybridoma cell, or the like) that the sequestration pen is intended for. For example, the width W_{con} of a connection region 236 at a proximal opening 234 of an sequestration pen that an immunological cell (e.g., B cell) will be placed into can be any of the following: about 20 microns, about 25 microns, about 30 microns, about 35 microns, about 40 microns, about 45 microns, about 50 microns, about 55 microns, about 60 microns, about 65 microns, about 70 microns, about 75 microns, or about 80 microns. The foregoing are examples only, and the width W_{con} of a connection region (e.g., 236) at a proximal opening (e.g., 234) can be different than the foregoing examples (e.g., a range defined by any of the endpoints listed above).

In various embodiments of sequestration pens, a ratio of the length L_{con} of a connection region (e.g., 236) to a width W_{con} of the connection region (e.g., 236) at the proximal opening 234 can be greater than or equal to any of the following ratios: 0.5, 1.0, 1.5, 2.0, 2.5, 3.0, 3.5, 4.0, 4.5, 5.0, 6.0, 7.0, 8.0, 9.0, 10.0, or more. The foregoing are examples only, and the ratio of the length L_{con} of a connection region 236 to a width W_{con} of the connection region 236 at the proximal opening 234 can be different than the foregoing examples.

In various embodiments of microfluidic devices 100, 200, 230, 250, 280, 290, 320 Vₘₐₓ can be set around 0.2, 0.3, 0.4, 0.5, 0.6, 0.7, 0.8, 0.9, 1.0, 1.1, 1.2, 1.3, 1.4, 1.5, 2.0, 2.5, 3.0, 3.5, 4.0, 4.5, or 5.0 µL/sec.

In various embodiments of microfluidic devices having sequestration pens, the volume of an isolation region (e.g., 240) of a sequestration pen can be, for example, at least 5x10⁵, 8x10⁵, 1x10⁶, 2x10⁶, 4x10⁶, 6x10⁶, 8x10⁶, 1x10⁷ cubic microns, or more. In various embodiments of microfluidic devices having sequestration pens, the volume of a sequestration pen may be about 5x10⁵, 6x10⁵, 8x10⁵, 1x10⁶, 2x10⁶, 4x10⁶, 8x10⁶, 1x10⁷ cubic microns, or more. In some other embodiments, the volume of a sequestration pen may be about 0.5 nanoliter to about 10 nanoliters, about 1.0 nanoliters to about 5.0 nanoliters, about 1.5 nanoliters to about 4.0 nanoliters, about 2.0 nanoliters to about 3.0 nanoliters, about 2.5 nanoliters, or any range defined by two of the foregoing endpoints.

In various embodiment, the microfluidic device has sequestration pens configured as in any of the embodiments discussed herein where the microfluidic device has about 5 to about 10 sequestration pens, about 10 to about 50 sequestration pens, about 100 to about 500 sequestration pens; about 200 to about 1000 sequestration pens, about 500 to about 1500 sequestration pens, about 1000 to about 2000 sequestration pens, or about 1000 to about 3500 sequestration pens. The sequestration pens need not all be the same size and may include a variety of configurations (e.g., different widths, different features within the sequestration pen.

In some other embodiments, the microfluidic device has sequestration pens configured as in any of the embodiments discussed herein where the microfluidic device has about 1500 to about 3000 sequestration pens, about 2000 to about 3500 sequestration pens, about 2500 to about 4000 sequestration pens about 3000 to about 4500 sequestration pens, about 3500 to about 5000 sequestration pens, about 4000 to about 5500 sequestration pens, about 4500 to about 6000 sequestration pens, about 5000 to about 6500 sequestration pens, about 5500 to about 7000 sequestration pens, about 6000 to about 7500 sequestration pens, about 6500 to about 8000 sequestration pens, about 7000 to about 8500 sequestration pens, about 7500 to about 9000 sequestration pens, about 8000 to about 9500 sequestration pens, about 8500 to about 10,000 sequestration pens, about 9000 to about 10,500 sequestration pens, about 9500 to about 11,000 sequestration pens, about 10,000 to about 11,500 sequestration pens, about 10,500 to about 12,000 sequestration pens, about 11,000 to about 12,500 sequestration pens, about 11,500 to about 13,000 sequestration pens, about 12,000 to about 13,500 sequestration pens, about 12,500 to about 14,000 sequestration pens, about 13,000 to about 14,500 sequestration pens, about 13,500 to about 15,000 sequestration pens, about 14,000 to about 15,500 sequestration pens, about 14,500 to about 16,000 sequestration pens, about 15,000 to about 16,500 sequestration pens, about 15,500 to about 17,000 sequestration pens, about 16,000 to about 17,500 sequestration pens, about 16,500 to about 18,000 sequestration pens, about 17,000 to about 18,500 sequestration pens, about 17,500 to about 19,000 sequestration pens, about 18,000 to about 19,500 sequestration pens, about 18,500 to about 20,000 sequestration pens, about 19,000 to about 20,500 sequestration pens, about 19,500 to about 21,000 sequestration pens, or about 20,000 to about 21,500 sequestration pens.

Figure 2G illustrates a microfluidic device 280 according to one embodiment. The microfluidic device 280 is illustrated in Figure 2G is a stylized diagram of a microfluidic device 100. In practice the microfluidic device 280 and its constituent circuit elements (e.g. channels 122 and sequestration pens 128) would have the dimensions discussed herein. The microfluidic circuit 120 illustrated in Figure 2G has two ports 107, four distinct channels 122 and four distinct flow paths 106. The microfluidic device 280 further comprises a plurality of sequestration pens opening off of each channel 122. In the microfluidic device illustrated in Figure 2G, the sequestration pens have a geometry similar to the pens illustrated in Figure 2C and thus, have both connection regions and isolation regions. Accordingly, the microfluidic circuit 120 includes both swept regions (e.g. channels 122 and portions of the connection regions 236 within the maximum penetration depth Dₚ of the secondary flow 244) and non-swept regions (e.g. isolation regions 240 and portions of the connection regions 236 not within the maximum penetration depth Dₚ of the secondary flow 244).

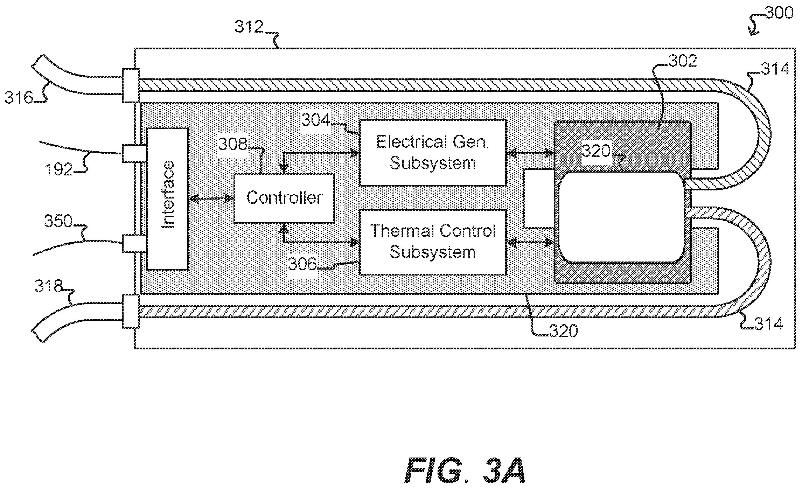

Figures 3A through 3B shows various embodiments of system 150 which can be used to operate and observe microfluidic devices (e.g. 100, 200, 230, 280, 250, 290, 320) according to the present invention. As illustrated in Figure 3A, the system 150 can include a structure ("nest") 300 configured to hold a microfluidic device 100 (not shown), or any other microfluidic device described herein. The nest 300 can include a socket 302 capable of interfacing with the microfluidic device 320 (e.g., an optically-actuated electrokinetic device 100) and providing electrical connections from power source 192 to microfluidic device 320. The nest 300 can further include an integrated electrical signal generation subsystem 304. The electrical signal generation subsystem 304 can be configured to supply a biasing voltage to socket 302 such that the biasing voltage is applied across a pair of electrodes in the microfluidic device 320 when it is being held by socket 302. Thus, the electrical signal generation subsystem 304 can be part of power source 192. The ability to apply a biasing voltage to microfluidic device 320 does not mean that a biasing voltage will be applied at all times when the microfluidic device 320 is held by the socket 302. Rather, in most cases, the biasing voltage will be applied intermittently, e.g., only as needed to facilitate the generation of electrokinetic forces, such as dielectrophoresis or electro-wetting, in the microfluidic device 320.

As illustrated in Figure 3A, the nest 300 can include a printed circuit board assembly (PCBA) 322. The electrical signal generation subsystem 304 can be mounted on and electrically integrated into the PCBA 322. The exemplary support includes socket 302 mounted on PCBA 322, as well.

Typically, the electrical signal generation subsystem 304 will include a waveform generator (not shown). The electrical signal generation subsystem 304 can further include an oscilloscope (not shown) and/or a waveform amplification circuit (not shown) configured to amplify a waveform received from the waveform generator. The oscilloscope, if present, can be configured to measure the waveform supplied to the microfluidic device 320 held by the socket 302. In certain embodiments, the oscilloscope measures the waveform at a location proximal to the microfluidic device 320 (and distal to the waveform generator), thus ensuring greater accuracy in measuring the waveform actually applied to the device. Data obtained from the oscilloscope measurement can be, for example, provided as feedback to the waveform generator, and the waveform generator can be configured to adjust its output based on such feedback. An example of a suitable combined waveform generator and oscilloscope is the Red Pitaya^{™}.

In certain embodiments, the nest 300 further comprises a controller 308, such as a microprocessor used to sense and/or control the electrical signal generation subsystem 304. Examples of suitable microprocessors include the Arduino^{™} microprocessors, such as the Arduino Nano^{™}. The controller 308 may be used to perform functions and analysis or may communicate with an external master controller 154 (shown in Figure 1A) to perform functions and analysis. In the embodiment illustrated in Figure 3A the controller 308 communicates with a master controller 154 through an interface 310 (e.g., a plug or connector).

In some embodiments, the nest 300 can comprise an electrical signal generation subsystem 304 comprising a Red Pitaya^{™} waveform generator/oscilloscope unit ("Red Pitaya unit") and a waveform amplification circuit that amplifies the waveform generated by the Red Pitaya unit and passes the amplified voltage to the microfluidic device 100. In some embodiments, the Red Pitaya unit is configured to measure the amplified voltage at the microfluidic device 320 and then adjust its own output voltage as needed such that the measured voltage at the microfluidic device 320 is the desired value. In some embodiments, the waveform amplification circuit can have a +6.5V to -6.5V power supply generated by a pair of DC-DC converters mounted on the PCBA 322, resulting in a signal of up to 13 Vpp at the microfluidic device 100.

As illustrated in Figure 3A, the support structure 300 can further include a thermal control subsystem 306. The thermal control subsystem 306 can be configured to regulate the temperature of microfluidic device 320 held by the support structure 300. For example, the thermal control subsystem 306 can include a Peltier thermoelectric device (not shown) and a cooling unit (not shown). The Peltier thermoelectric device can have a first surface configured to interface with at least one surface of the microfluidic device 320. The cooling unit can be, for example, a cooling block (not shown), such as a liquid-cooled aluminum block. A second surface of the Peltier thermoelectric device (e.g., a surface opposite the first surface) can be configured to interface with a surface of such a cooling block. The cooling block can be connected to a fluidic path 314 configured to circulate cooled fluid through the cooling block. In the embodiment illustrated in Figure 3A, the support structure 300 comprises an inlet 316 and an outlet 318 to receive cooled fluid from an external reservoir (not shown), introduce the cooled fluid into the fluidic path 314 and through the cooling block, and then return the cooled fluid to the external reservoir. In some embodiments, the Peltier thermoelectric device, the cooling unit, and/or the fluidic path 314 can be mounted on a casing 312of the support structure 300. In some embodiments, the thermal control subsystem 306 is configured to regulate the temperature of the Peltier thermoelectric device so as to achieve a target temperature for the microfluidic device 320. Temperature regulation of the Peltier thermoelectric device can be achieved, for example, by a thermoelectric power supply, such as a Pololu^{™} thermoelectric power supply (Pololu Robotics and Electronics Corp.). The thermal control subsystem 306 can include a feedback circuit, such as a temperature value provided by an analog circuit. Alternatively, the feedback circuit can be provided by a digital circuit.

In some embodiments, the nest 300 can include a thermal control subsystem 306 with a feedback circuit that is an analog voltage divider circuit (not shown) which includes a resistor (e.g., with resistance 1 kOhm+/-0.1 %, temperature coefficient +/-0.02 ppm/C0) and a NTC thermistor (e.g., with nominal resistance 1 kOhm+/-0.01 %). In some instances, the thermal control subsystem 306 measures the voltage from the feedback circuit and then uses the calculated temperature value as input to an on-board PID control loop algorithm. Output from the PID control loop algorithm can drive, for example, both a directional and a pulse-width-modulated signal pin on a Pololu^{™} motor drive (not shown) to actuate the thermoelectric power supply, thereby controlling the Peltier thermoelectric device.

The nest 300 can include a serial port 324 which allows the microprocessor of the controller 308 to communicate with an external master controller 154 via the interface 310 (not shown). In addition, the microprocessor of the controller 308 can communicate (e.g., via a Plink tool (not shown)) with the electrical signal generation subsystem 304 and thermal control subsystem 306. Thus, via the combination of the controller 308, the interface 310, and the serial port 324, the electrical signal generation subsystem 304 and the thermal control subsystem 306 can communicate with the external master controller 154. In this manner, the master controller 154 can, among other things, assist the electrical signal generation subsystem 304 by performing scaling calculations for output voltage adjustments. A Graphical User Interface (GUI) (not shown) provided via a display device 170 coupled to the external master controller 154, can be configured to plot temperature and waveform data obtained from the thermal control subsystem 306 and the electrical signal generation subsystem 304, respectively. Alternatively, or in addition, the GUI can allow for updates to the controller 308, the thermal control subsystem 306, and the electrical signal generation subsystem 304.

As discussed above, system 150 can include an imaging device 194. In some embodiments, the imaging device 194 comprises a light modulating subsystem 330 (See Figure 3B). The light modulating subsystem 330 can include a digital mirror device (DMD) or a microshutter array system (MSA), either of which can be configured to receive light from a light source 332 and transmits a subset of the received light into an optical train of microscope 350. Alternatively, the light modulating subsystem 330 can include a device that produces its own light (and thus dispenses with the need for a light source 332), such as an organic light emitting diode display (OLED), a liquid crystal on silicon (LCOS) device, a ferroelectric liquid crystal on silicon device (FLCOS), or a transmissive liquid crystal display (LCD). The light modulating subsystem 330 can be, for example, a projector. Thus, the light modulating subsystem 330 can be capable of emitting both structured and unstructured light. One example of a suitable light modulating subsystem 330 is the Mosaic^{™} system from Andor Technologies^{™}. In certain embodiments, imaging module 164 and/or motive module 162 of system 150 can control the light modulating subsystem 330.

In certain embodiments, the imaging device 194 further comprises a microscope 350. In such embodiments, the nest 300 and light modulating subsystem 330 can be individually configured to be mounted on the microscope 350. The microscope 350 can be, for example, a standard research-grade light microscope or fluorescence microscope. Thus, the nest 300 can be configured to be mounted on the stage 344of the microscope 350 and/or the light modulating subsystem 330 can be configured to mount on a port of microscope 350. In other embodiments, the nest 300 and the light modulating subsystem 330 described herein can be integral components of microscope 350.

In certain embodiments, the microscope 350 can further include one or more detectors 348. In some embodiments, the detector 348 is controlled by the imaging module 164. The detector 348 can include an eye piece, a charge-coupled device (CCD), a camera (e.g., a digital camera), or any combination thereof. If at least two detectors 348 are present, one detector can be, for example, a fast-frame-rate camera while the other detector can be a high sensitivity camera. Furthermore, the microscope 350 can include an optical train configured to receive reflected and/or emitted light from the microfluidic device 320 and focus at least a portion of the reflected and/or emitted light on the one or more detectors 348. The optical train of the microscope can also include different tube lenses (not shown) for the different detectors, such that the final magnification on each detector can be different.

In certain embodiments, imaging device 194 is configured to use at least two light sources. For example, a first light source 332 can be used to produce structured light (e.g., via the light modulating subsystem 330) and a second light source 334 can be used to provide unstructured light. The first light source 332 can produce structured light for optically-actuated electrokinesis and/or fluorescent excitation, and the second light source 334 can be used to provide bright field illumination. In these embodiments, the motive module 164 can be used to control the first light source 332 and the imaging module 164 can be used to control the second light source 334. The optical train of the microscope 350 can be configured to (1) receive structured light from the light modulating subsystem 330 and focus the structured light on at least a first region in a microfluidic device, such as an optically-actuated electrokinetic device, when the device is being held by the nest 300, and (2) receive reflected and/or emitted light from the microfluidic device and focus at least a portion of such reflected and/or emitted light onto detector 348. The optical train can be further configured to receive unstructured light from a second light source and focus the unstructured light on at least a second region of the microfluidic device, when the device is held by the nest 300. In certain embodiments, the first and second regions of the microfluidic device can be overlapping regions. For example, the first region can be a subset of the second region.

In Figure 3B, the first light source 332 is shown supplying light to a light modulating subsystem 330, which provides structured light to the optical train of the microscope 350 of system 355 (not shown). The second light source 334 is shown providing unstructured light to the optical train via a beam splitter 336. Structured light from the light modulating subsystem 330 and unstructured light from the second light source 334 travel from the beam splitter 336 through the optical train together to reach a second beam splitter (or dichroic filter 338, depending on the light provided by the light modulating subsystem 330), where the light gets reflected down through the objective 336 to the sample plane 342. Reflected and/or emitted light from the sample plane 342 then travels back up through the objective 340, through the beam splitter and/or dichroic filter 338, and to a dichroic filter 346. Only a fraction of the light reaching dichroic filter 346 passes through and reaches the detector 348.

In some embodiments, the second light source 334 emits blue light. With an appropriate dichroic filter 346, blue light reflected from the sample plane 342 is able to pass through dichroic filter 346 and reach the detector 348. In contrast, structured light coming from the light modulating subsystem 330 gets reflected from the sample plane 342, but does not pass through the dichroic filter 346. In this example, the dichroic filter 346 is filtering out visible light having a wavelength longer than 495 nm. Such filtering out of the light from the light modulating subsystem 330 would only be complete (as shown) if the light emitted from the light modulating subsystem did not include any wavelengths shorter than 495 nm. In practice, if the light coming from the light modulating subsystem 330 includes wavelengths shorter than 495 nm (e.g., blue wavelengths), then some of the light from the light modulating subsystem would pass through filter 346 to reach the detector 348. In such an embodiment, the filter 346 acts to change the balance between the amount of light that reaches the detector 348 from the first light source 332 and the second light source 334. This can be beneficial if the first light source 332 is significantly stronger than the second light source 334. In other embodiments, the second light source 334 can emit red light, and the dichroic filter 346 can filter out visible light other than red light (e.g., visible light having a wavelength shorter than 650 nm).

**Coating solutions and coating agents.** Without intending to be limited by theory, the culturing of a micro-object, such as a biological cell (e.g., an immunological cell such as a B cell or a T cell) within a microfluidic device may be facilitated (i.e., the micro-object exhibits increased viability, greater expansion, and/or greater portability within the microfluidic device) when one or more inner surfaces of the microfluidic device have been conditioned or coated so as to present a layer of organic and/or hydrophilic molecules that provides the primary interface between the microfluidic device and the micro-object (e.g., biological cell) maintained therein. In some embodiments, one or more of the inner surfaces of the microfluidic device (e.g. the inner surface of the electrode activation substrate of a DEP-configured microfluidic device, the cover of the microfluidic device, and/or the surfaces of the circuit material) are treated with a coating solution and/or coating agent to generate the desired layer of organic and/or hydrophilic molecules. In some embodiments, the micro-object(s) (e,g, biological cell(s)) that are to be cultured and, optionally, allowed to expand in the microfluidic device are imported in a coating solution that includes one or more coating agents.

In other embodiments, the inner surface(s) of the microfluidic device (e.g., a DEP-configured microfluidic device) are treated or "primed" with a coating solution comprising a coating agent prior to introduction of the micro-object(s) (e,g, biological cell(s)) into the microfluidic device. Any convenient coating agent/coating solution can be used, including but not limited to: serum or serum factors, bovine serum albumin (BSA), polymers, detergents, enzymes, and any combination thereof. In some specific embodiments, a coating agent will be used to treat the inner surface(s) of the microfluidic device. In one example, a polymer comprising alkylene ether moieties can be included as a coating agent in the coating solution. A wide variety of alkylene ether containing polymers may be suitable. One non-limiting exemplary class of alkylene ether containing polymers are amphiphilic nonionic block copolymers which include blocks of polyethylene oxide (PEO) and polypropylene oxide (PPO) subunits in differing ratios and locations within the polymer chain. Pluronic^{®} polymers (BASF) are block copolymers of this type and are known in the art to be suitable for use when in contact with living cells. The polymers range in average molecular mass M_{w} from about 2000Da to about 20KDa. In some embodiments, the PEO-PPO block copolymer can have a hydrophilic-lipophilic balance (HLB) greater than about 10 (e.g. 12-18). Specific Pluronic^{®} polymers useful for yielding a coated surface include Pluronic^{®} L44, L64, P85, and F127 (including F127NF). Another class of alkylene ether containing polymers is polyethylene glycol (PEG M_{w} <100,000Da) or alternatively polyethylene oxide (PEO, M_{w}>100,000). In some embodiments, a PEG may have an M_{w} of about 1000Da, 5000Da, 10,000Da or 20,000Da.

In some embodiments, a coating solution can comprise various proteins and/or peptides as coating agents. In a specific embodiment, a coating solution that finds use in the present disclosure includes a protein such as albumin (e.g. BSA) and/or serum (or a combination of multiple different sera) comprising albumin and/or one or more other similar proteins as coating agents. The serum can be from any convenient source, including but not limited to fetal calf serum, sheep serum, goat serum, horse serum, and the like. In certain embodiments, BSA in a blocking solution is present in a range of form about 1 mg/mL to about 100 mg/mL, including 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, or more or anywhere in between. In certain embodiments, serum in a coating solution is present in a range of from about 20% (v/v) to about 50% v/v, including 25%, 30%, 35%, 40%, 45%, or more or anywhere in between. In some embodiments, BSA is present as a coating agent in a coating solution at 5 mg/mL, whereas in other embodiments, BSA is present as a coating agent in a coating solution at 70 mg/mL. In certain embodiments, serum is present as a coating agent in a coating solution at 30%.

**Coating materials.** Depending on the embodiment, any of the foregoing coating agents/coating solutions can be replaced by or used in combination with various coating materials used to coat one or more of the inner surface(s) of the microfluidic device (e.g., a DEP-configured and/or EW-configured microfluidic device). In some embodiments, at least one surface of the microfluidic device includes a coating material that provides a layer of organic and/or hydrophilic molecules suitable for maintenance and/or expansion of biological micro-objects (e.g., cells, such as immunological cells (e.g., B cells) or hybridoma cells). In some embodiments, substantially all the inner surfaces of the microfluidic device include the coating material. The coated inner surface(s) may include the surface of a flow region (e.g., channel), chamber, or sequestration pen, or a combination thereof. In some embodiments, each of a plurality of sequestration pens has at least one inner surface coated with coating materials. In other embodiments, each of a plurality of flow regions or channels has at least one inner surface coated with coating materials. In some embodiments, at least one inner surface of each of a plurality of sequestration pens and each of a plurality of channels is coated with coating materials.

**Polymer-based coating materials.** The at least one inner surface may include a coating material that comprises a polymer. The polymer may be covalently or non-covalently bound (or linked) to the at least one surface. The polymer may have a variety of structural motifs, such as found in block polymers (and copolymers), star polymers (star copolymers), and graft or comb polymers (graft copolymers), all of which may be suitable for the methods disclosed herein.

The polymer may include a polymer including alkylene ether moieties. A wide variety of alkylene ether containing polymers may be suitable for use in the microfluidic devices described herein. One non-limiting exemplary class of alkylene ether containing polymers are amphiphilic nonionic block copolymers which include blocks of polyethylene oxide (PEO) and polypropylene oxide (PPO) subunits in differing ratios and locations within the polymer chain. Pluronic^{®} polymers (BASF) are block copolymers of this type and are known in the art to be suitable for use when in contact with living cells. The polymers range in average molecular mass M_{w} from about 2000Da to about 20KDa. In some embodiments, the PEO-PPO block copolymer can have a hydrophilic-lipophilic balance (HLB) greater than about 10 (e.g. 12-18). Specific Pluronic^{®} polymers useful for yielding a coated surface include Pluronic^{®} L44, L64, P85, and F127 (including F127NF). Another class of alkylene ether containing polymers is polyethylene glycol (PEG M_{w} <100,000Da) or alternatively polyethylene oxide (PEO, M_{w}>100,000). In some embodiments, a PEG may have an M_{w} of about 1000Da, 5000Da, 10,000Da or 20,000Da.

In other embodiments, the coating material may include a polymer containing carboxylic acid moieties. The carboxylic acid subunit may be an alkyl, alkenyl or aromatic moiety containing subunit. One non-limiting example is polylactic acid (PLA).

In other embodiments, the coating material may include a polymer containing sulfonic acid moieties. The sulfonic acid subunit may be an alkyl, alkenyl or aromatic moiety containing subunit. One non-limiting example is polystyrene sulfonic acid (PSSA) or polyanethole sulfonic acid. These latter exemplary polymers are polyelectrolytes and may alter the characteristics of the surface to provides a layer of organic and/or hydrophilic molecules suitable for maintenance and/or expansion of biological micro-objects (e.g., cells, such as immunological cells (e.g., B cells) or hybridoma cells).

In some embodiments, the coating material may include a polymer containing urethane moieties, such as, but not limited to polyurethane.

In other embodiments, the coating material may include a polymer containing phosphate moieties, either at a terminus of the polymer backbone or pendant from the backbone of the polymer.

In other embodiments, the coating material may include a polymer containing saccharide moieties. In a non-limiting example, polysaccharides such as those derived from algal or fungal polysaccharides such as xanthan gum or dextran may be suitable to form a material which may reduce or prevent cell sticking in the microfluidic device. For example, a dextran polymer having a size about 3Kda may be used to provide a coating material for a surface within a microfluidic device.

In other embodiments, the coating material may include a polymer containing nucleotide moieties, i.e. a nucleic acid, which may have ribonucleotide moieties or deoxyribonucleotide moieties. The nucleic acid may contain only natural nucleotide moieties or may contain unnatural nucleotide moieties which comprise nucleobase, ribose or phosphate moiety analogs such as 7-deazaadenine, pentose, methyl phosphonate or phosphorothioate moieties without limitation. A nucleic acid containing polymer may include a polyelectrolyte which may provide a layer of organic and/or hydrophilic molecules suitable for maintenance and/or expansion of biological micro-objects (e.g., cells, such as immunological cells (e.g., B cells) or hybridoma cells).

In yet other embodiments, the coating material may include a polymer containing amino acid moieties. The polymer containing amino acid moieties may include a natural amino acid containing polymer or an unnatural amino acid containing polymer, either of which may include a peptide, a polypeptide or a protein. In one non-limiting example, the protein may be bovine serum albumin (BSA). In some embodiments, an extracellular matrix (ECM) protein may be provided within the coating material for optimized cell adhesion to foster cell growth. A cell matrix protein, which may be included in a coating material, can include, but is not limited to, a collagen, an elastin, an RGD-containing peptide (e.g. a fibronectin), or a laminin. In yet other embodiments, growth factors, cytokines, hormones or other cell signaling species may be provided within the coating material of the microfluidic device.

In further embodiments, the coating material may include a polymer including amine moieties. The polyamino polymer may include a natural polyamine polymer or a synthetic polyamine polymer. Examples of natural polyamines include spermine, spermidine, and putrescine.

In some embodiments, the coating material may include a polymer containing more than one of alkylene oxide moieties, carboxylic acid moieties, sulfonic acid moieties, phosphate moieties, saccharide moieties, nucleotide moieties, or amino acid moieties. In other embodiments, the polymer conditioned surface may include a mixture of more than one polymer each having alkylene oxide moieties, carboxylic acid moieties, sulfonic acid moieties, phosphate moieties, saccharide moieties, nucleotide moieties, and/or amino acid moieties, which may be independently or simultaneously incorporated into the coating material.

**Covalently linked coating materials.** In some embodiments, the at least one inner surface includes covalently linked molecules that provide a layer of organic and/or hydrophilic molecules suitable for maintenance and/or expansion of biological micro-objects (e.g., cells, such as immunological cells (e.g., B cells) or hybridoma cells) within the microfluidic device, providing a conditioned surface for such cells. The covalently linked molecules include a linking group, wherein the linking group is covalently linked to one or more surfaces of the microfluidic device. The linking group is also covalently linked to a moiety configured to provide a layer of organic and/or hydrophilic molecules suitable for maintenance and/or expansion of biological micro-objects (e.g., cells, such as immunological cells (e.g., B cells) or hybridoma cells). The surface to which the linking group links may include a surface of the substrate of the microfluidic device which, for embodiments in which the microfluidic device includes a DEP configuration, can include silicon and/or silicon dioxide. In some embodiments, the covalently linked coating materials coat substantially all of the inner surfaces of the microfluidic device.

In some embodiments, the covalently linked moiety configured to provide a layer of organic and/or hydrophilic molecules suitable for maintenance and/or expansion of biological micro-objects (e.g., cells, such as immunological cells (e.g., B cells) or hybridoma cells) may include alkyl or fluoroalkyl (which includes perfluoroalkyl) moieties; mono- or polysaccharides (which may include but is not limited to dextran); alcohols (including but not limited to propargyl alcohol); polyalcohols, including but not limited to polyvinyl alcohol; alkylene ethers, including but not limited to polyethylene glycol; polyelectrolytes ( including but not limited to polyacrylic acid or polyvinyl phosphonic acid); amino groups (including derivatives thereof, such as, but not limited to alkylated amines, hydroxyalkylated amino group, guanidinium, and heterocylic groups containing an unaromatized nitrogen ring atom, such as, but not limited to morpholinyl or piperazinyl); carboxylic acids including but not limited to propiolic acid (which may provide a carboxylate anionic surface); phosphonic acids, including but not limited to ethynyl phosphonic acid (which may provide a phosphonate anionic surface); sulfonate anions; carboxybetaines; sulfobetaines; sulfamic acids; or amino acids.

The covalently linked moiety configured to provide a layer of organic and/or hydrophilic molecules suitable for maintenance and/or expansion of biological micro-objects (e.g., cells, such as immunological cells (e.g., B cells) or hybridoma cells) in the microfluidic device may be any polymer as described herein, and may include one or more polymers containing alkylene oxide moieties, carboxylic acid moieties, saccharide moieties, sulfonic acid moieties, phosphate moieties, amino acid moieties, nucleic acid moieties, or amino moieties.

In other embodiments, the covalently linked moiety configured to provide a layer of organic and/or hydrophilic molecules suitable for maintenance and/or expansion of biological micro-objects (e.g., cells, such as immunological cells (e.g., B cells) or hybridoma cells) in the microfluidic device may include non-polymeric moieties such as an alkyl moiety, a substituted alkyl moiety, such as a fluoroalkyl moiety (including but not limited to a perfluoroalkyl moiety), amino acid moiety, alcohol moiety, amino moiety, carboxylic acid moiety, phosphonic acid moiety, sulfonic acid moiety, sulfamic acid moiety, or saccharide moiety.

In some embodiments, the covalently linked moiety may be an alkyl group that comprises carbon atoms that form a linear chain (e.g., a linear chain of at least 10 carbons, or at least 14, 16, 18, 20, 22, or more carbons). Thus, the alkyl group may be an unbranched alkyl. In some embodiments, the alkyl group may include a substituted alkyl group (e.g., some of the carbons in the alkyl group can be fluorinated or perfluorinated). The alkyl group may comprise a linear chain of substituted (e.g., fluorinated or perfluorinated) carbons joined to a linear chain of non-substituted carbons. For example, the alkyl group may include a first segment, which may include a perfluoroalkyl group, joined to a second segment, which may include a non-substituted alkyl group. The first and second segments may be joined directly or indirectly (e.g., by means of an ether linkage). The first segment of the alkyl group may be located distal to the linking group, and the second segment of the alkyl group may be located proximal to the linking group. In other embodiment, the alkyl group may include a branched alkyl group and may further have one or more arylene group interrupting the alkyl backbone of the alkyl group. In some embodiments, a branched or arylene-interrupted portion of the alkyl or fluorinated alkyl group is located at a point distal to the linking group and the covalent linkage to the surface.

In other embodiments, the covalently linked moiety may include at least one amino acid, which may include more than one type of amino acid. Thus, the covalently linked moiety may include a peptide or a protein. In some embodiments, the covalently linked moiety may include an amino acid which may provide a zwitterionic surface to support cell growth, viability, portability, or any combination thereof.

The covalently linked moiety may include one or more saccharides. The covalently linked saccharides may be mono-, di-, or polysaccharides. The covalently linked saccharides may be modified to introduce a reactive pairing moiety which permits coupling or elaboration for attachment to the surface. Exemplary reactive pairing moieties may include aldehyde, alkyne or halo moieties. A polysaccharide may be modified in a random fashion, wherein each of the saccharide monomers may be modified or only a portion of the saccharide monomers within the polysaccharide are modified to provide a reactive pairing moiety that may be coupled directly or indirectly to a surface. One exemplar may include a dextran polysaccharide, which may be coupled indirectly to a surface via an unbranched linker.

The covalently linked moiety may include one or more amino groups. The amino group may be a substituted amine moiety, guanidine moiety, nitrogen-containing heterocyclic moiety or heteroaryl moiety. The amino containing moieties may have structures permitting pH modification of the environment within the microfluidic device, and optionally, within the sequestration pens and/or flow regions (e.g., channels).

The coating material may comprise only one kind of covalently linked moiety or may include more than one different kind of covalently linked moiety. For example, the fluoroalkyl conditioned surfaces (including perfluoroalkyl) may have a plurality of covalently linked moieties which are all the same, e.g., having the same linking group and covalent attachment to the surface, the same overall length, and the same number of fluoromethylene units comprising the fluoroalkyl moiety. Alternatively, the coating material may have more than one kind of covalently linked moiety attached to the surface. For example, the coating material may include molecules having covalently linked alkyl or fluoroalkyl moieties having a specified number of methylene or fluoromethylene units and may further include a further set of molecules having covalently charged moieties attached to an alkyl or fluoroalkyl chain having a greater number of methylene or fluoromethylene units. In some embodiments, the coating material having more than one kind of covalently linked moiety may be designed such that a first set of molecules which have a greater number of backbone atoms, and thus a greater length from the covalent attachment to the surface, may provide capacity to present bulkier moieties at the coated surface, while a second set of molecules having different, less sterically demanding termini and fewer backbone atoms can help to functionalize the entire substrate surface and thereby prevent undesired adhesion or contact with silicon or alumina making up the substrate itself. In another example, the covalently linked moieties may provide a zwitterionic surface presenting alternating charges in a random fashion on the surface.

**Conditioned surface properties.** In some embodiments, the covalently linked moieties may form a monolayer when covalently linked to the surface of the microfluidic device (e.g., a DEP configured substrate surface). In some embodiments, the conditioned surface formed by the covalently linked moieties may have a thickness of less than 10 nm (e.g., less than 5 nm, or about 1.5 to 3.0 nm). In other embodiments, the conditioned surface formed by the covalently linked moieties may have a thickness of about 10 nm to about 50 nm. In some embodiments, the conditioned surface does not require a perfectly formed monolayer to be suitably functional for operation within a DEP-configured microfluidic device.

In various embodiments, the coating material of the microfluidic device may provide desirable electrical properties. Without intending to be limited by theory, one factor that impacts robustness of a surface coated with a particular coating material is intrinsic charge trapping. Different coating materials may trap electrons, which can lead to breakdown of the coating material. Defects in the coating material may increase charge trapping and lead to further breakdown of the coating material. Similarly, different coating materials have different dielectric strengths (i.e. the minimum applied electric field that results in dielectric breakdown), which may impact charge trapping. In certain embodiments, the coating material can have an overall structure (e.g., a densely-packed monolayer structure) that reduces or limits that amount of charge trapping.

Aside from the composition of the coating material, other factors such as physical (and electrical) thickness of the coating material can impact the generation of DEP force and/or electrowetting force by a substrate in a microfluidic device. Various factors can alter the physical and electrical thickness of the coating material, including the manner in which the coating material is deposited on the substrate (e.g. vapor deposition, liquid phase deposition, spin coating, or electrostatic coating). The physical thickness and uniformity of the coating material can be measured using an ellipsometer.

Besides their electrical properties, the coating material may have properties that are beneficial in use with biological molecules. For example, coating materials that contain fluorinated (or perfluorinated) alkyl groups may provide a benefit relative to unsubstituted alkyl groups in reducing the amount of surface fouling. Surface fouling, as used herein, refers to the amount of material indiscriminately deposited on the surface of the microfluidic device, which may include permanent or semi-permanent deposition of biomaterials such as protein and degradation products, nucleic acids, and respective degradation products. Such fouling can increase the amount of adhesion of biological micro-objects to the surface.

Aside from the composition of the conditioned surface, other factors such as physical thickness of the hydrophobic material can impact DEP force. Various factors can alter the physical thickness of the conditioned surface, such as the manner in which the conditioned surface is formed on the substrate (e.g. vapor deposition, liquid phase deposition, spin coating, flooding, and electrostatic coating). The physical thickness and uniformity of the conditioned surface can be measured using an ellipsometer.

In addition to its electrical properties, the conditioned surface may also have properties that are beneficial in use with biological molecules. For example, a conditioned surface that contains fluorinated (or perfluorinated) carbon chains may provide a benefit relative to alkyl-terminated chains in reducing the amount of surface fouling. Surface fouling, as used herein, refers to the amount of indiscriminate material deposition on the surface of the microfluidic device, which may include permanent or semi-permanent deposition of biomaterials such as protein and its degradation products, nucleic acids and respective degradation products and the like.

Various properties for conditioned surfaces which may be used in DEP configurations are included in the table below. As can be seen, for entries 1 to 7, which were all covalently linked conditioned surfaces as described herein, the thickness as measured by ellipsometry were consistently thinner than that of entry 8, a CYTOP surface which was formed by non-covalent spin coating (N/A represents data not available throughout the table). Fouling was found to be more dependent upon the chemical nature of the surface than upon the mode of formation as the fluorinated surfaces were typically less fouling than that of alkyl (hydrocarbon) conditioned surfaces.

**Table 1. Properties of various conditioned surfaces prepared by covalently modifying a surface, compared to CYTOP, a non-covalently formed surface.**

| **Surface modification type** | **Formula of surface modifying reagent** | **Thickness** | **Fouling** |
|---|---|---|---|
| Alkyl terminated siloxane (C₁₆) | CH₃-(CH₂)₁₅-Si-(OCH₃)3 | N/A | More fouling than fluorinated layers. |
| Alkyl terminated siloxane (C₁₈) | CH₃-(CH₂)₁₇-Si-(OCH₃)₃ | ∼ 2nm | More fouling than fluorinated layers. |
| Alkyl-terminated phosphonate ester CisPA | CH₃-(CH₂)₁₇-P=O(OH)2 | N/A | More fouling than fluorinated layers. |
| Alkyl terminated siloxane (C22) | CH₃-(CH₂)₂₁-Si-(OCH2CH3)3 | -2-2.5 nm | More fouling than fluorinated layers. |
| Fluoro-alkyl-terminated alkylsiloxane C₁₀F | CF₃-(CF₂)₇-(CH₂)₂-Si-(OCH₃)₃ | ∼1 nm | More resistant to fouling than alkyl-terminated layers |
| Fluoro-alkyl-terminated alkylsiloxane (C₁₆F) | CF₃-(CF₂)₁₃-(CH₂)₂-Si-(OCH₃)₃ | ∼2 nm | More resistant to fouling than alkyl-terminated layers |
| Fluoro-alkyl-terminated alkoxyalkyl-siloxane C₆FC₁₃ | CF₃-(CF₂)₅-(CH₂)₂-O-(CH₂)₁₁-Si(OCH₃)₃ | ∼2 nm | N/A |
| CYTOP Fluoropolymer¹ | | -30 nm | More resistant to fouling than alkyl-terminated layers |

| | | | |
|---|---|---|---|
| 1. Spin coated, not covalent. | | | |

**Linking group to surface.** The covalently linked moieties forming the coating material are attached to the surface via a linking group. The linking group may be a siloxy linking group formed by the reaction of a siloxane-containing reagent with oxides of the substrate surface, which can include silicon oxide (e.g., for a DEP-configured substrate) or aluminum oxide or hafnium oxide (e.g., for a EW-configured substrate). In some other embodiments, the linking group may be a phosphonate ester formed by the reaction of a phosphonic acid containing reagent with the oxides of the substrate surface.

**Multi-part conditioned surface.** The covalently linked coating material may be formed by reaction of a molecule which already contains the moiety configured to provide a layer of organic and/or hydrophilic molecules suitable for maintenance and/or expansion of biological micro-objects (e.g., cells, such as immunological cells (e.g., B cells) or hybridoma cells)in the microfluidic device (e.g., an alkyl siloxane reagent or a fluoro-substituted alkyl siloxane reagent, which may include a perfluoroalkyl siloxane reagent), as is described below. Alternatively, the covalently linked coating material may be formed by coupling the moiety configured provide a layer of organic and/or hydrophilic molecules suitable for maintenance and/or expansion of biological micro-objects (e.g., cells, such as immunological cells (e.g., B cells) or hybridoma cells) to a surface modifying ligand that itself is covalently linked to the surface.

**Methods of preparing a covalently linked coating material.** In some embodiments, a coating material that is covalently linked to the surface of a microfluidic device (e.g., including at least one surface of the sequestration pens and/or flow regions) has a structure of Formula 1.

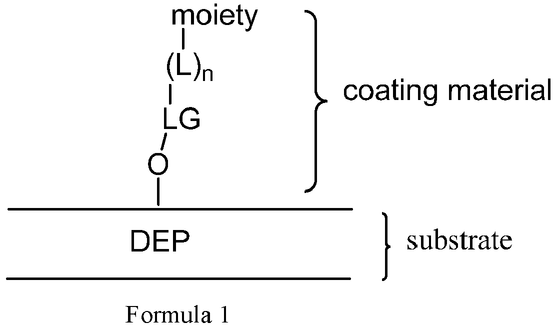

The coating material may be linked covalently to oxides of the surface of a DEP-configured substrate. The DEP-configured substrate may comprise silicon or alumina or hafnium oxide, and oxides may be present as part of the native chemical structure of the substrate or may be introduced as discussed below.

The coating material may be attached to the oxides via a linking group ("LG"), which may be a siloxy or phosphonate ester group formed from the reaction of a siloxane or phosphonic acid group with the oxides. The moiety configured to provide a layer of organic and/or hydrophilic molecules suitable for maintenance and/or expansion of biological micro-objects (e.g., cells, such as immunological cells (e.g., B cells) or hybridoma cells) in the microfluidic device can be any of the moieties described herein. The linking group LG may be directly or indirectly connected to the moiety configured to provide a layer of organic and/or hydrophilic molecules suitable for maintenance and/or expansion of biological micro-objects (e.g., cells, such as immunological cells (e.g., B cells) or hybridoma cells) in the microfluidic device. When the linking group LG is directly connected to the moiety, optional linker ("L") is not present and n is 0. When the linking group LG is indirectly connected to the moiety, linker L is present and n is 1. The linker L may have a linear portion where a backbone of the linear portion may include 1 to 200 non-hydrogen atoms selected from any combination of silicon, carbon, nitrogen, oxygen, sulfur and phosphorus atoms, subject to chemical bonding limitations as is known in the art. It may be interrupted with any combination of one or more moieties selected from the group consisting of ether, amino, carbonyl, amido, or phosphonate groups, in some non-limiting examples. Additionally, the linker L may have one or more arylene, heteroarylene, or heterocyclic groups interrupting the backbone of the linker. In some embodiments, the backbone of the linker L may include 10 to 20 atoms. In other embodiments, the backbone of the linker L may include about 5 atoms to about 200 atoms; about 10 atoms to about 80 atoms; about 10 atoms to about 50 atoms; or about 10 atoms to about 40 atoms. In some embodiments, the backbone atoms are all carbon atoms. In other embodiments, the backbone atoms are not all carbons, and may include any possible combination of silicon, carbon, nitrogen, oxygen, sulfur or phosphorus atoms, subject to chemical bonding limitations as is known in the art.

When the moiety configured to provide a layer of organic and/or hydrophilic molecules suitable for maintenance and/or expansion of biological micro-objects (e.g., cells, such as immunological cells (e.g., B cells) or hybridoma cells) in the microfluidic device is added to the surface of the substrate in a one step process, a molecule of Formula 2 may be used to introduce the coating material:

moiety - (L)n - LG. Formula 2

In some embodiments, the moiety configured to provide a layer of organic and/or hydrophilic molecules suitable for maintenance and/or expansion of biological micro-objects (e.g., cells, such as immunological cells (e.g., B cells) or hybridoma cells) in the microfluidic device may be added to the surface of the substrate in a multi-step process. When the moiety configured to provide a layer of organic and/or hydrophilic molecules suitable for maintenance and/or expansion of biological micro-objects (e.g., cells, such as immunological cells (e.g., B cells) or hybridoma cells) is coupled to the surface in a step wise fashion, the linker L may further include a coupling group CG, as shown in Formula 3.

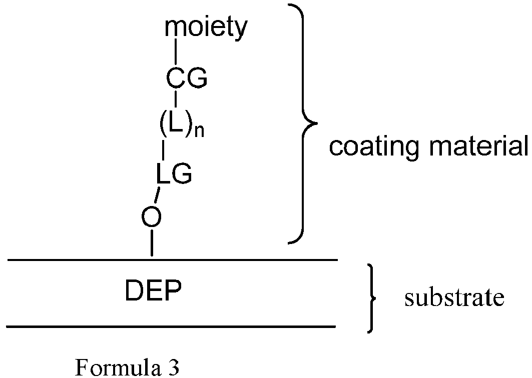

In some embodiments, the coupling group CG represents the resultant group from reaction of a reactive moiety Rₓ and a reactive pairing moiety Rₚₓ (i.e., a moiety configured to react with the reactive moiety Rₓ). For example, one typical coupling group CG may include a carboxamidyl group, which is the result of the reaction of an amino group with a derivative of a carboxylic acid, such as an activated ester, an acid chloride or the like. Other CG may include a triazolylene group, a carboxamidyl, thioamidyl, an oxime, a mercaptyl, a disulfide, an ether, or alkenyl group, or any other suitable group that may be formed upon reaction of a reactive moiety with its respective reactive pairing moiety. The coupling group CG may be located at the second end (i.e., the end proximal to the moiety configured to provide a layer of organic and/or hydrophilic molecules suitable for maintenance and/or expansion of biological micro-objects (e.g., cells, such as immunological cells (e.g., B cells) or hybridoma cells) in the microfluidic device) of a linker L. In some other embodiments, the coupling group CG may interrupt the backbone of the linker L. In some embodiments, the coupling group CG is triazolylene, which is the result of a reaction between an alkyne group and an azide group, either of which may be the reactive moiety Rₓ or the reactive pairing moiety Rₚₓ, as is known in the art for use in Click coupling reactions. A triazolylene group may also be further substituted. For example, a dibenzocylcooctenyl fused triazolylene group may result from the reaction of a moiety bound to a dibenzocyclooctynyl reactive pairing moiety Rₚₓ with an azido reactive moiety Rₓ of the surface modifying molecule, which are described in more detail in the following paragraphs. A variety of dibenzocyclooctynyl modified molecules are known in the art or may be synthesized to incorporate a moiety configured to provide a layer of organic and/or hydrophilic molecules suitable for maintenance and/or expansion of biological micro-objects (e.g., cells, such as immunological cells (e.g., B cells) or hybridoma cells).

When the coating material is formed in a multi-step process, the moiety configured to provide a layer of organic and/or hydrophilic molecules suitable for maintenance and/or expansion of biological micro-objects (e.g., cells, such as immunological cells (e.g., B cells) or hybridoma cells) in the microfluidic device may be introduced by reaction of a moiety-containing reagent (Formula 5) with a substrate having a surface modifying ligand covalently linked thereto (Formula 6).

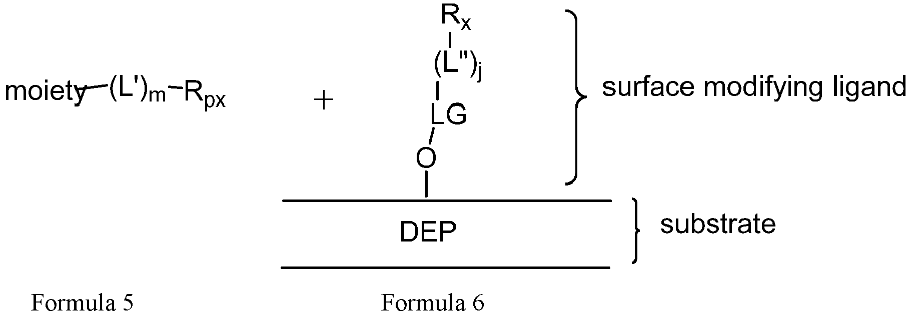

The modified surface of Formula 4 has a surface modifying ligand attached thereto, which has a formula of -LG-(L")j- Rₓ, ,which is linked to the oxide of the substrate and is formed similarly as described above for the conditioned surface of Formula 1. The surface of the substrate can be a DEP-configured substrate surface as described above, and can include oxides either native to the substrate or introduced therein. The linking group LG is as described above. A linker L" may be present (j=1) or absent (j= 0). The linker L" may have a linear portion where a backbone of the linear portion may include 1 to 100 non-hydrogen atoms selected from of any combination of silicon, carbon, nitrogen, oxygen, sulfur and phosphorus atoms, subject to chemical bonding limitations as is known in the art. It may be interrupted with any combination of ether, amino, carbonyl, amido, or phosphonate groups, in some non-limiting examples. Additionally, the linker L" may have one or more arylene, heteroarylene, or heterocyclic groups interrupting the backbone of the linker. In some embodiments, the backbone of the linker L" may include 10 to 20 carbon atoms. In other embodiments, the backbone of the linker L" may include about 5 atoms to about 100 atoms; about 10 atoms to about 80 atoms, about 10 atoms to about 50 atoms, or about 10 atoms to about 40 atoms. In some embodiments, the backbone atoms are all carbon atoms. In other embodiments, the backbone atoms are not all carbons, and may include any possible combination of silicon, carbon, nitrogen, oxygen, sulfur or phosphorus atoms, subject to chemical bonding limitations as is known in the art.

A reactive moiety Rₓ is present at the terminus of the surface modifying ligand distal to the covalent linkage of the surface modifying ligand with the surface. The reactive moiety Rₓ is any suitable reactive moiety useful for coupling reactions to introduce the moiety provide a layer of organic and/or hydrophilic molecules suitable for maintenance and/or expansion of biological micro-objects (e.g., cells, such as immunological cells (e.g., B cells) or hybridoma cells) in the microfluidic device. In some embodiments, the reactive moiety Rₓ may be an azido, amino, bromo, a thiol, an activated ester, a succinimidyl or alkynyl moiety.

**Moiety-containing reagent.** The moiety-containing reagent (Formula 5) is configured to supply the moiety configured to provide a layer of organic and/or hydrophilic molecules suitable for maintenance and/or expansion of biological micro-objects (e.g., cells, such as immunological cells (e.g., B cells) or hybridoma cells) in the microfluidic device.

Moiety-(L')m-Rpx Formula 5

The moiety configured to provide a layer of organic and/or hydrophilic molecules suitable for maintenance and/or expansion of biological micro-objects (e.g., cells, such as immunological cells (e.g., B cells) or hybridoma cells) in the moiety-containing reagent is linked to the surface modifying ligand by reaction of a reactive pairing moiety Rₚₓ with the reactive moiety Rₓ. The reactive pairing moiety Rₚₓ is any suitable reactive group configured to react with the respective reactive moiety Rₓ. In one non-limiting example, one suitable reactive pairing moiety Rₚₓ may be an alkyne and the reactive moiety Rₓ may be an azide. The reactive pairing moiety Rₚₓ may alternatively be an azide moiety and the respective reactive moiety Rₓ may be alkyne. In other embodiments, the reactive pairing moiety Rₚₓ may be an active ester functionality and the reactive moiety Rₓ may be an amino group. In other embodiments, the reactive pairing moiety Rₚₓ may be aldehyde and the reactive moiety Rₓ may be amino. Other reactive moiety-reactive pairing moiety combinations are possible, and these examples are in no way limiting.

The moiety configured to provide a layer of organic and/or hydrophilic molecules suitable for maintenance and/or expansion of biological micro-objects (e.g., cells, such as immunological cells (e.g., B cells) or hybridoma cells) of the moiety-containing reagent of Formula 5 may include any of the moieties described herein, including alkyl or fluoroalkyl (which includes perfluoroalkyl) moieties; mono- or polysaccharides (which may include but is not limited to dextran); alcohols (including but not limited to propargyl alcohol); polyalcohols, including but not limited to polyvinyl alcohol; alkylene ethers, including but not limited to polyethylene glycol; polyelectrolytes ( including but not limited to polyacrylic acid or polyvinyl phosphonic acid); amino groups (including derivatives thereof, such as, but not limited to alkylated amines, hydroxyalkylated amino group, guanidinium, and heterocylic groups containing an unaromatized nitrogen ring atom, such as, but not limited to morpholinyl or piperazinyl); carboxylic acids including but not limited to propiolic acid (which may provide a carboxylate anionic surface); phosphonic acids, including but not limited to ethynyl phosphonic acid (which may provide a phosphonate anionic surface); sulfonate anions; carboxybetaines; sulfobetaines; sulfamic acids; or amino acids.

The moiety configured to provide a layer of organic and/or hydrophilic molecules suitable for maintenance and/or expansion of biological micro-objects (e.g., cells, such as immunological cells (e.g., B cells) or hybridoma cells) of the moiety-containing reagent of Formula 5 may be directly connected (i.e., L', where m =0) or indirectly connected to the reactive pairing moiety Rₚₓ. When the reactive pairing moiety Rₚₓ is connected indirectly to the moiety configured to provide a layer of organic and/or hydrophilic molecules suitable for maintenance and/or expansion of biological micro-objects (e.g., cells, such as immunological cells (e.g., B cells) or hybridoma cells), the reactive pairing moiety Rₚₓ may be connected to a linker L' (m = 1). The reactive pairing moiety Rₚₓ may be connected to a first end of the linker L', and the moiety configured to reduce surface fouling and/or prevent or reduce cell sticking may be connected to a second end of the linker L'. Linker L' may have a linear portion wherein a backbone of the linear portion includes 1 to 100 non-hydrogen atoms selected from of any combination of silicon, carbon, nitrogen, oxygen, sulfur and phosphorus atoms, subject to chemical bonding limitations as is known in the art. It may be interrupted with any combination of ether, amino, carbonyl, amido, or phosphonate groups, in some non-limiting examples. Additionally, the linker L' may have one or more arylene, heteroarylene, or heterocyclic groups interrupting the backbone of the linker L'. In some embodiments, the backbone of the linker L' may include 10 to 20 atoms. In other embodiments, the backbone of the linker L' may include about 5 atoms to about 100 atoms; about 10 atoms to about 80 atoms; about 10 atoms to about 50 atoms; or about 10 atoms to about 40 atoms. In some embodiments, the backbone atoms are all carbon atoms. In other embodiments, the backbone atoms are not all carbons, and may include any possible combination of silicon, carbon, nitrogen, oxygen, sulfur or phosphorus atoms, subject to chemical bonding limitations as is known in the art.

When the moiety-containing reagent (Formula 5) reacts with the surface having a surface modifying ligand (Formula 3), a substrate having a conditioned surface of Formula 2 is formed. Linker L' and linker L" then are formally part of linker L, and the reaction of the reactive pairing moiety Rₚₓ with the reactive moiety Rₓ yields the coupling group CG of Formula 2.

**Surface modifying reagent.** The surface modifying reagent is a compound having a structure LG-(L")j- Rₓ (Formula 4). The linking group LG links covalently to the oxides of the surface of the substrate. The substrate may be a DEP-configured substrate and may include silicon or alumina or hafnium oxide, and oxides may be present as part of the native chemical structure of the substrate or may be introduced as discussed herein. The linking group LG may be any linking group described herein, such as a siloxy or phosphonate ester group, formed from the reaction of a siloxane or phosphonic acid group with the oxide on the surface of the substrate. The reactive moiety Rₓ is described above. The reactive moiety Rₓ may be connected directly (L", j = 0) or indirectly via a linker L" (j=1) to the linking group LG. The linking group LG may be attached to a first end of the linker L" and the reactive moiety Rₓ may be connected to a second end of the linker L", which will be distal to the surface of the substrate once the surface modifying reagent has been attached to the surface as in Formula 6.

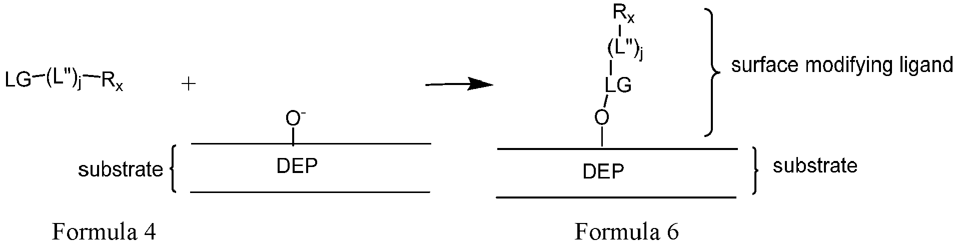

Linker L" may have a linear portion wherein a backbone of the linear portion includes 1 to 100 non-hydrogen atoms selected from of any combination of silicon, carbon, nitrogen, oxygen, sulfur and phosphorus atoms. It may be interrupted with any combination of ether, amino, carbonyl, amido, or phosphonate groups, in some non-limiting examples. Additionally, the linker L" may have one or more arylene, heteroarylene, or heterocyclic groups interrupting the backbone of the linker L". In some embodiments, the backbone of the linker L" may include 10 to 20 atoms. In other embodiments, the backbone of the linker L" may include about 5 atoms to about 100 atoms; about 10 atoms to about 80 atoms, about 10 atoms to about 50 atoms, or about 10 atoms to about 40 atoms. In some embodiments, the backbone atoms are all carbon atoms. In other embodiments, the backbone atoms are not all carbons, and may include any possible combination of silicon, carbon, nitrogen, oxygen, sulfur or phosphorus atoms, subject to chemical bonding limitations as is known in the art.

In some embodiments, the coating material (or surface modifying ligand) is deposited on the inner surfaces of the microfluidic device using chemical vapor deposition. Through chemical vapor deposition, the coating material can achieve densely-packed monolayers in which the molecules comprising the coating material are covalently bonded to the molecules of the inner surfaces of the microfluidic device. To achieve a desirable packing density, molecules comprising, for example, alkyl-terminated siloxane can be vapor deposited at a temperature of at least 110°C (e.g., at least 120°C, 130°C, 140°C, 150°C, 160°C, etc.), for a period of at least 15 hours (e.g., at least 20, 25, 30, 35, 40, 45, or more hours). Such vapor deposition is typically performed under vacuum and in the presence of a water source, such as a hydrated sulfate salt (e.g., MgSO₄·7H₂0). Typically, increasing the temperature and duration of the vapor deposition produces improved characteristics of the hydrophobic coating material.

The vapor deposition process can be optionally improved, for example, by pre-cleaning the cover 110, the microfluidic circuit material 116, and/or the substrate (e.g., the inner surface 208 of the electrode activation substrate 206 of a DEP-configured substrate, or a dielectric layer of the support structure 104 of an EW-configured substrate). For example, such pre-cleaning can include a solvent bath, such as an acetone bath, an ethanol bath, or a combination thereof. The solvent bath can include sonication. Alternatively, or in addition, such pre-cleaning can include treating the cover 110, the microfluidic circuit material 116, and/or the substrate in an oxygen plasma cleaner, which can remove various impurities, while at the same time introducing an oxidized surface (e.g. oxides at the surface, which may be covalently modified as described herein). The oxygen plasma cleaner can be operated, for example, under vacuum conditions, at 100W for 60 seconds. Alternatively, liquid-phase treatments, which include oxidizing agents such as hydrogen peroxide to oxidize the surface, may be used in place of an oxygen plasma cleaner. For example, a mixture of hydrochloric acid and hydrogen peroxide or a mixture of sulfuric acid and hydrogen peroxide (e.g., piranha solution, which may have a ratio of sulfuric acid to hydrogen peroxide in a range from about 3:1 to about 7:1) may be used in place of an oxygen plasma cleaner.

In some embodiments, vapor deposition is used to coat the inner surfaces of the microfluidic device 200 after the microfluidic device 200 has been assembled to form an enclosure 102 defining a microfluidic circuit 120. Deposition of a coating material comprising a densely-packed monolayer on a fully-assembled microfluidic circuit 120 may be beneficial in providing various functional properties. Without intending to be limited by theory, depositing such a coating material on a fully-assembled microfluidic circuit 120 may be beneficial in preventing delamination caused by a weakened bond between the microfluidic circuit material 116 and the electrode activation substrate 206 dielectric layer and/or the cover 110.

Figure 2H depicts a cross-sectional views of a microfluidic device 290 comprising exemplary classes of coating materials. As illustrated, the coating materials 298 (shown schematically) can comprise a monolayer of densely-packed molecules covalently bound to both the inner surface 294 of the substrate 286 and the inner surface 292 of the cover 288 of the microfluidic device 290. The coating material 298 can be disposed on all inner surfaces 294, 292 proximal to, and facing inwards towards, the enclosure 284 of the microfluidic device 290, including, in some embodiments and as discussed above, the surfaces of microfluidic circuit material (not shown) used to define circuit elements and/or structures within the microfluidic device 290. In alternate embodiments, the coating material 298 can be disposed on only one or some of the inner surfaces of the microfluidic device 290.

In the embodiment shown in Figure 2H, the coating material 298 comprises a monolayer of alkyl-terminated siloxane molecules, each molecule covalently bonded to the inner surfaces 292, 294 of the microfluidic device 290 via a siloxy linker 296. However, any of the above-discussed coating materials 298 can be used (e.g. alkyl-terminated phosphonate ester molecules). More specifically, the alkyl group can comprise a linear chain of at least 10 carbon atoms (e.g. 10, 12, 14, 16, 18, 20, 22, or more carbon atoms) and, optionally, may be a substituted alkyl group. As discussed above, coating materials 298 that comprise a monolayer of densely-packed molecules can have beneficial functional characteristics for use in DEP configured microfluidic devices 290, such as minimal charge trapping, reduced physical/electrical thickness, and a substantially uniform surface.

In another specific embodiment, the coating material 298 can comprise a fluoroalkyl group (e.g. a fluorinated alkyl group or a perfluorinated alkyl group) at its enclosure-facing terminus (i.e. the portion of the monolayer of the coating material 298 that is not bound to the inner surfaces 292, 294 and is proximal to the enclosure 284). As discussed above, the coating material 298 can comprise a monolayer of fluoroalkyl-terminated siloxane or fluoroalkyl-terminated phosphonate ester, wherein the fluoroalkyl group is present at the enclosure-facing terminus of the coating material 298. Such a coating material 298 provides a functional benefit in providing for improved maintenance and/or expansion of biological micro-objects (e.g., cells, such as immunological cells (e.g., B cells) or hybridoma cells) by separating or "shielding" the biological micro-object from the non-biological molecules (e.g., the silicon and/or silicon oxide of the substrate)

In another specific embodiment, the coating material 298 used to coat the inner surface(s) 292, 294 of the microfluidic device 290 can include anionic, cationic, or zwitterionic moieties, or any combination thereof. Without intending to be limited by theory, by presenting cationic moieties, anionic moieties, and/or zwitterionic moieties at the inner surfaces of the enclosure 284 of the microfluidic circuit 120, the coating material 298 can form strong hydrogen bonds with water molecules such that the resulting water of hydration acts as a layer (or "shield") that separates the nuclei from interactions with non-biological molecules (e.g., the silicon and/or silicon oxide of the substrate). In addition, in embodiments in which the coating material 298 is used in conjunction with blocking agents, the anions, cations, and/or zwitterions of the coating material 298 can form ionic bonds with the charged portions of non-covalent coating agents (e.g. proteins in solution) that are present in a medium 180 (e.g. a coating solution) in the enclosure 284.

In still another specific embodiment, the coating material may comprise or be chemically modified to present a hydrophilic coating agent at its enclosure-facing terminus. In some embodiments, the coating agent may be an alkylene ether containing polymer, such as PEG. In some embodiments, the coating agent may be a polysaccharide, such as dextran. Like the charged moieties discussed above (e.g., anionic, cationic, and zwitterionic moieties), the hydrophilic coating agent can form strong hydrogen bonds with water molecules such that the resulting water of hydration acts as a layer (or "shield") that separates the nuclei from interactions with non-biological molecules (e.g., the silicon and/or silicon oxide of the substrate).

**Methods of detecting antibody expression.** Methods disclosed herein include a method of detecting or identifying a biological cell expressing an antibody that specifically binds to an antigen of interest. The antigen of interest can be a protein, a carbohydrate group or chain, a biological or chemical agent other than a protein or carbohydrate, or any combination thereof. The antigen of interest can be, for example, an antigen associated with a pathogen, such as a virus, a bacterial pathogen, a fungal pathogen, a protozoan pathogen, or the like. Alternatively, the antigen of interest can be associated with a cancer, such as lung cancer, breast cancer, melanoma, and the like. In yet another alternative, the antigen can be associated with an auto-immune disease, such as multiple sclerosis or type I diabetes. As used herein, the term "associated with a pathogen," when used in reference to an antigen of interest, means that the antigen of interest is produced directly by the pathogen or results from an interaction between the pathogen and the host.

The methods of detecting a biological cell expressing an antibody that specifically binds to an antigen of interest can be performed in a microfluidic device described herein. In particular, the microfluidic device can include an enclosure having a flow region, which may include one or more microfluidic channels, and a sequestration pen (or a plurality of sequestration pens). The sequestration pen can include an isolation region and a connection region, the connection region providing a fluidic connection between the isolation region and the flow region/microfluidic channel. The sequestration pen can have a volume of about 0.5 nL to about 5.0 nl, or any range therein (e.g., about 0.5 nl to about 1.0 nl, about 0.5 nl to about 1.5 nl, about 0.5 nl to about 2.0 nl, about 1.0 nl to about 1.5 nl, about 1.0 nl to about 2.0 nl, about 1.0 nl to about 2.5 nl, about 1.5 nl to about 2.0 nl, about 1.5 nl to about 2.5 nl, about 1.5 nl to about 3.0 nl, about 2.0 nl to about 2.5 nl, about 2.0 nl to about 3.0 nl, about 2.0 nl to about 3.5 nl, about 2.5 nl to about 3.0 nl, about 2.5 nl to about 3.5 nl, about 2.5 nl to about 4.0 nl, about 3.0 nl to about 3.5 nl, about 3.0 nl to about 4.0 nl, about 3.0 nl to about 4.5 nl, about 3.5 nl to about 4.0 nl, about 3.5 nl to about 4.5 nl, about 3.5 nl to about 5.0 nl, about 4.0 nl to about 4.5 nl, about 4.0 nl to about 5.0 nl, about 4.5 nl to about 5.0 nl, or any range defined by one of the foregoing endpoints). The connection region can have a width W_{con} as generally described herein (e.g., about 20 microns to about 100 microns, or about 30 microns to about 60 microns). The isolation region can have a width Wiso that is greater than the width W_{con} of said connection region. In certain embodiments, the isolation region has a width Wiso that is about 50 microns to about 250 microns.

The flow region, the sequestration pen, and or the isolation region of the sequestration pen can include at least one surface coated with a coating material that promotes the viability of and/or reduces interactions with a biological cell. Thus, for example, the coating material can promote the viability of a hybridoma cell, and/or promote the viability of a B cell lymphocyte (e.g., a memory B cell or a plasma cell), and/or the ability to move any such cells within the microfluidic device. As used in this context, "promote the viability" means that the viability of the antibody expressing biological cell is better on the coated surface as compared to an equivalent surface that is non-coated. In certain embodiments, the flow region, the sequestration pen, and/or the isolation region has a plurality of surfaces each coated with a coating material that promotes the viability of and/or reduces interactions with the antibody expressing cell. The coating material can be any suitable coating material known in the art and/or described herein. The coating material can, for example, comprise hydrophilic molecules. The hydrophilic molecules can be selected from the group consisting of polymers comprising polyethylene glycol (PEG), polymers comprising carbohydrate groups, polymers comprising amino acids (e.g., proteins, such as BSA), and combinations thereof.

The flow region, the sequestration pen, and or the isolation region of the sequestration pen can include at least one conditioned surface that promotes the viability of and/or reduces interactions with the antibody expressing biological cell. Thus, for example, the conditioned surface can promote the viability of a hybridoma cell, and/or promote the viability of a B cell lymphocyte (e.g., a memory B cell or a plasma cell), and/or promote the ability to move any such cells within the microfluidic device. As used in this context, "promote the viability" means that the viability of the antibody expressing biological cell is better on the conditioned surface as compared to an equivalent surface that is not conditioned. In certain embodiments, the flow region, the sequestration pen, and/or the isolation region has a plurality of conditioned surfaces each of which is capable of promoting the viability of and/or reducing interactions with the antibody expressing cell. The conditioned surface(s) can comprise covalently linked molecules. The covalently linked molecules can be any suitable molecules known in the art and/or disclosed herein, including, for example, covalently linked hydrophilic molecules. The hydrophilic molecules can be selected from the group consisting of polymers comprising polyethylene glycol (PEG), polymers comprising carbohydrate groups, polymers comprising amino acids, and combinations thereof. The hydrophilic molecules can form a layer of covalently linked hydrophilic molecules, as described herein. Alternatively, the covalently linked molecules can comprise perfluoroalkanes (e.g., a layer of covalently linked perfluoroalkanes).

The methods of detecting a biological cell expressing an antibody that specifically binds to an antigen of interest can include the steps of: introducing a sample containing the antibody expressing biological cell into the microfluidic device; loading the antibody expressing biological cell into an isolation region of a sequestration pen in a microfluidic device; introducing the antigen of interest into the microfluidic device such that the antigen of interest is located proximal to the antibody expressing biological cell; and monitoring binding of the antigen of interest to the antibody expressed by the biological cell.

The antibody expressing biological cell can be, for example, a hybridoma cell. Alternatively, the antibody expressing biological cell can be a B cell lymphocyte. The B cell lymphocyte can be, for example, a CD27⁺ B cell or a CD138⁺ B cell. In some embodiments, the B cell is a memory B cell. In other embodiments, the B cell is a plasma cell.

Introducing the antibody expressing biological cell into the microfluidic device can involve obtaining the sample that contains the antibody expressing cell. For embodiments in which the antibody expressing biological cell is a B cell lymphocyte, the sample containing the B cell lymphocyte can be obtained from a mammal, such as a human, a rodent (e.g., a mouse, rat, guinea pig, gerbil, hamster), a rabbit, a ferret, livestock (e.g., goats, sheep, pigs, horses, cows), a llama, a camel, a monkey, or obtained from avian species, such as chickens and turkey. In some embodiments, the mammal has been immunized against the antigen of interest. In some embodiments, the animal has been exposed to or infected with a pathogen associated with the antigen of interest. In some embodiments, the animal has a cancer that is associate with the antigen of interest. In other embodiments, the animal has an auto-immune disease that is associated with the antigen of interest. The sample containing the B cell lymphocyte can be a peripheral blood sample (e.g., PBMCs), a spleen biopsy, a bone marrow biopsy, a lymph node biopsy, a tumor biopsy, or any combination thereof.

The sample containing the B cell lymphocyte can be treated (e.g., sorted, negatively and/or positively) to enrich for desired B cell lymphocytes. In some embodiments, the desired B cell lymphocytes are memory B cells. In other embodiments, the desired B cell lymphocytes are plasma cells. In some embodiments, the desired B cell lymphocytes express an IgG-type antibody. Thus, for example, the sample can be depleted of cell types other than B cell lymphocytes. Methods of depleting non-B cell cell types from samples are well known in the art, and include, for example, treating the sample with the DYNABEADS^{™} Untouched Human B Cells reagent (Thermo Fisher), the B Cell Isolation Kit (Miltenyi), the EasySep B Cell Enrichment Kit (EasySep), the RosetteSep Human B Cell Enrichment Cocktain (Stem Cell Technologies), or the like. Alternatively, or in addition, the sample containing the B cell lymphocyte can be sorted by fluorescence-associated cell sorting (FACS) to remove unwanted cell types and enriched for the desired cell types. The FACS sorting can be negative and/or positive. For example, the FACS sorting can deplete the sample of B cell lymphocytes expressing IgM antibodies, IgA antibodies, IgD antibodies, IgG antibodies, or any combination thereof. Alternatively, or in addition, the FACS sorting can enrich the sample for B cell lymphocytes that express CD27 (or some other memory B cell marker) or for B cell lymphocytes that express CD138 (or some other plasma cell marker). The sample containing the B cell lymphocyte can be provided in an enriched state (i.e., pre-treated) such that no treatment to enrich for desired B cell lymphocytes is required as part of the method. Alternatively, treating the sample containing the B cell lymphocyte to enrich for desired B cell lymphocytes can be performed as part of the methods of the invention.

The sample containing the B cell lymphocyte can be treated to reduce sticking of cells in the sample to the microfluidic device. For example, the sample can be treated with a DNase, such as Benzonase^{®} Nuclease (Millipore). Preferably, the DNase contains minimal protease activity.

Introducing the antibody expressing biological cell into the microfluidic device can be performed by flowing a sample containing the biological cell into an inlet in the microfluidic device and through a portion of the flow region of the microfluidic device. Flow of the sample through the microfluidic device can then be stopped to allow for loading the antibody expressing biological cell (e.g., B cell lymphocyte) into the isolation region of a sequestration pen. Loading of the antibody expressing cell into the isolation region can be performed by any technique known in the art or disclosed herein, such as using gravity and/or DEP force. In certain embodiments, a single antibody expressing cell (e.g., B cell lymphocyte) is loaded into the isolation region. In certain embodiments, a single antibody expressing cell (e.g., B cell lymphocyte) is loaded into the isolation region of each of a plurality of sequestration pens in the microfluidic device.

The methods of detecting a biological cell expressing an antibody that specifically binds to an antigen of interest can include the step of contacting a B cell lymphocyte with a stimulating agent that stimulates B cell activation. The stimulating agent can be a CD40 agonist, such as CD40L, a derivative thereof, or an anti-CD40 antibody. The stimulating agent can comprise, consist essentially of, or consist of CD40L⁺ feeder cells. The CD40L⁺ feeder cells can be T cells (e.g., Jurkat D1.1 cells), or a derivative thereof. Alternatively, the feeder cells can be a cell line (e.g., NIH-3T3 cells) transfected/transformed with a CD40L-expressing construct. The stimulating agent can further comprise a B Cell Receptor (BCR) superantigen, such as Protein A, Protein G, or any other BCR superantigen. The BCR superantigen can be attached to a micro-object, such as a bead, lipid vesicle, lipid nanoraft, or the like. Thus, micro-objects coated with a superantigen can be mixed with CD40L⁺ feeder cells. The mixture can have a ratio of about 1:1 feeder cells-to-micro-objects, or a ratio of about 1:5 feeder cells-to-micro-objects, or any ratio therebetween. Alternatively, the mixture can have a ratio of about 1:2 feeder cells-to-micro-objects, or a ratio of about 2:10 feeder cells-to-micro-objects, or any ratio therebetween. The stimulating agent can further comprise a toll-like receptor (TLR) agonist (e.g., a TLR9 agonist), which may be in combination with the CD40 agonist and, optionally, the BCR superantigen. The TLR agonist can be, for example, a CpG oligonucleotide (e.g., CpG2006). The CpG oligonucleotide can be used at a concentration of about 1 microgram/mL to about 20 micrograms/mL (e.g., about 1.5 to about 15 micrograms/mL, about 2.0 to about 10 micrograms/mL, or about 2.5 to about 5.0 micrograms/mL). The B cell lymphocyte can be contacted (e.g., substantially continuously, or periodically/intermittently) with the stimulating agent for a period of one to ten days (e.g., two to eight days, three to seven days, or four to six days). The B cell lymphocyte can be contacted with the stimulating agent within the sequestration pen into which the B cell lymphocyte is loaded. Such contacting can occur after the B cell lymphocyte is loaded in the sequestration pen.

The methods of detecting a biological cell expressing an antibody that specifically binds to an antigen of interest can further include the step of providing the antibody expressing biological cell (e.g., B cell lymphocyte) with culture/activation medium comprises one or more growth-inducing agents that promote B cell activation and/or expansion. The one or more growth-inducing agents can include at least one agent selected from the group consisting of CpG oligonucleotide, IL-2, IL-4, IL-6, IL-10, IL-21, BAFF, and April. The IL-2 can be provided at a concentration of about 2 ng/mL to about 5 ug/mL, or about 50 ng/mL to about 2 ug/mL, or about 100 ng/mL to about 1.5 ug/mL, or about 500 ng/mL to about 1 ug/mL, or about 1 ug/mL. The IL-4 can be provided at a concentration of about 2 ng/mL to about 20 ng/mL, or about 5 ng/mL to about 10 ng/mL, or about 5 ng/mL. The IL-6, IL-10, and/or IL-21 can be provided at a concentration of about 2 ng/mL to about 50 ng/mL, or about 5 ng/mL to about 20 ng/mL, or about 10 ng/mL. The BAFF and/or the April can be provided at a concentration of about 10 ng/mL to about 100 ng/mL, or about 10 ng/mL to about 50 ng/mL, or about 10 ng/mL to about 20 ng/mL, or about 10 ng/mL. The CpG oligonucleotide can be used at a concentration of about 1 microgram/mL to about 20 micrograms/mL, about 1.5 to about 15 micrograms/mL, about 2.0 to about 10 micrograms/mL, or about 2.0 micrograms/mL. In certain embodiments, the culture medium is provided to the antibody expressing biological cell of a period of one to ten days (e.g., two to eight days, three to seven days, or four to six days). The culture medium can comprise the stimulating agent (e.g., CD40 agonist and/or BCR superantigen). Thus, for example, when the antibody producing cell is a B cell lymphocyte, providing the culture medium to the B cell lymphocyte can be performed at the same time as contacting the B cell lymphocyte with the activating agent. In certain embodiments, the steps of contacting the B cell lymphocyte with a stimulating agent and providing culture medium to the B cell lymphocyte are preformed at overlapping times (e.g., over a substantially coextensive period of time).

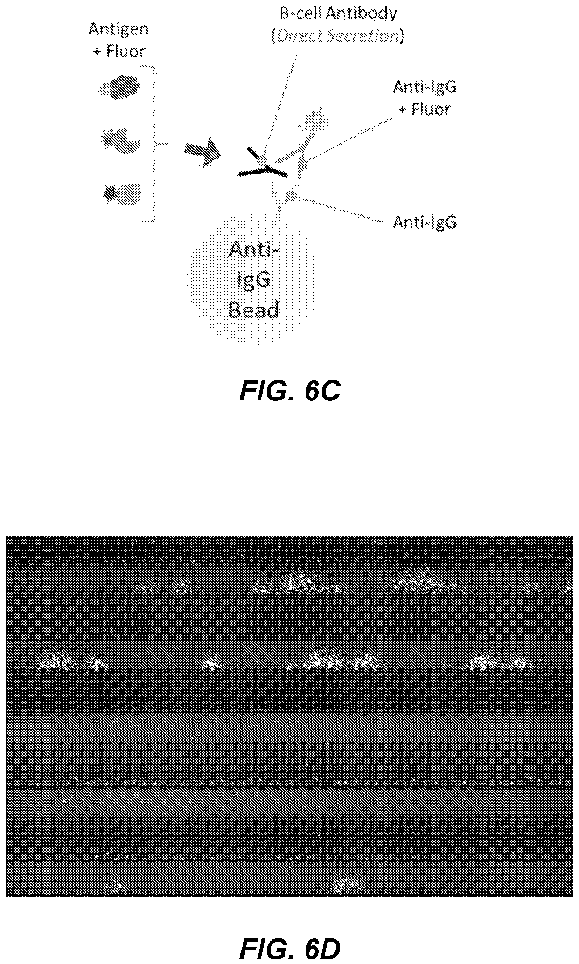

In certain embodiments, introducing the antigen of interest into the microfluidic device such that the antigen of interest is located proximal to the antibody expressing biological cell comprises positioning the antigen of interest within 1 millimeter (mm) of the biological cell (e.g., within 750 microns, within 600 microns, within 500 microns, within 400 microns, within 300 microns, within 200 microns, within 100 microns, or within 50 microns of the biological cell). In certain embodiments, the methods can include introducing a micro-object, or a plurality of micro-objects, into the flow region/microfluidic channel connected to the sequestration pen. The micro-objects can comprise an antibody-specific binding agent, such as an anti-IgG antibody or other IgG-binding agent. See, for example, Fig. 6C. In such embodiments, monitoring of binding of the antigen of interest to the antibody expressed by the biological cell comprises detecting indirect binding of labeled antigen of interest to the micro-object(s) via the antibody expressed by the antibody expressing biological cell. The labeled antigen of interest can be soluble and can include a detectable label, such as a fluorescent label. The micro-object can be any suitable micro-object known in the art and/or described herein (e.g., a cell, a liposome, a lipid nanoraft, or a bead). The step of providing the antigen of interest can include positioning such a micro-object adjacent to or within the connection region of the sequestration pen in which the antibody expressing biological cell is located. Alternatively, the step of providing the antigen of interest can include loading such a micro-object into the isolation region of the sequestration pen in which the antibody expressing biological call is located. The micro-object and antigen of interest can be provided simultaneously, as a mixture, or sequentially (if the micro-object is first positioned within the sequestration pen).

Alternatively, in certain embodiments, the methods can include introducing a micro-object, or a plurality of micro-objects, into the flow region/microfluidic channel connected to the sequestration pen, wherein the antigen of interest is coupled to the micro-object. In such embodiments, a soluble labeled antibody-specific binding agent, such as an anti-IgG antibody or other IgG-binding agent, can also be provided, and monitoring of binding of the antigen of interest to the antibody expressed by the biological cell comprises detecting indirect binding of the labeled antibody-specific binding agent to the micro-object(s) via the antibody expressed by the antibody expressing biological cell. The labeled antibody-specific binding agent can include a detectable label, such as a fluorescent label. The micro-object can be any suitable micro-object known in the art and/or described herein (e.g., a cell, a liposome, a lipid nanoraft, or a bead). The step of providing the antigen of interest can include positioning such a micro-object adjacent to or within the connection region of the sequestration pen in which the antibody expressing biological cell is located. The step of providing the antigen of interest can further include loading such a micro-object into the isolation region of the sequestration pen in which the antibody expressing biological call is located. The micro-object and antibody-specific binding agent can be provided simultaneously, as a mixture, or sequentially (if the micro-object is first positioned within the sequestration pen). Methods of screening for expression of a molecule of interest, such as an antibody, have been described, for example, in U.S. Patent Publication No. US2015/0151298, the entire contents of which are incorporated herein by reference.

In some embodiments, the methods further comprise providing a second antibody-specific binding agent prior to or concurrently with said first antibody-specific binding agent. See, for example, Fig. 6C. The second antibody-binding agent can be an anti-IgG antibody or other type of antibody-binding agent, and may be labeled (e.g., with a fluorescent label). In certain embodiments, the labeled second antibody-specific binding agent is provided in a mixture with the antigen of interest and first antibody-specific binding agent. In other embodiments, the labeled second antibody-specific binding agent is provided after providing the antigen of interest and/or first antibody-specific binding agent.

In certain embodiments, providing the antigen of interest can involve flowing a solution comprising soluble antigen of interest through the flow region of the microfluidic device and allowing the soluble antigen to diffuse into the sequestration pen in which the antibody expressing biological cell is located. Such soluble antigen can be covalently bound to a detectable label (e.g., a fluorescent label). General methods of screening for expression of a molecule of interest, including an antibody, in this manner have been described, for example, in International Application PCT/US2017/027795, filed April 14, 2017, the entire contents of which are incorporated herein by reference.

In certain embodiments, the methods can further comprise detecting binding of the antigen of interest to antibody expressed by the biological cell (e.g., B cell lymphocyte), and identifying the antibody expressing biological cell (e.g., B cell lymphocyte) as expressing an antibody that specifically binds to said antigen of interest.

**Obtaining antibody sequences from identified B cell lymphocytes.** Methods of providing sequencing libraries and/or obtaining heavy and light chain antibody sequences from antibody expressing cells are also disclosed herein. Additionally, obtaining a sequencing library from B cell lymphocytes of interest may be performed by methods other than the methods described herein. Other suitable, but non-limiting methods are described in PCT/US2017/054628, filed on September 29, 2017, and hereby incorporated by reference for all purposes in its entirety.

Capture/priming oligonucleotide. A capture/priming oligonucleotide may include a first priming sequence and a capture sequence. The capture/priming oligonucleotide may include a 5'-most nucleotide and a 3'-most nucleotide.

Capture sequence. The capture sequence is an oligonucleotide sequence configured to capture nucleic acid from a lysed cell. In various embodiments, the capture sequence may be adjacent to or comprises the 3'-most nucleotide of the capture/priming oligonucleotide. The capture sequence may have from about 6 to about 50 nucleotides. In some embodiments, the capture sequence captures a nucleic acid by hybridizing to a nucleic acid released from a cell of interest. In some of the methods described herein, the nucleic acid released from a B cell of interest may be mRNA. A capture sequence which may capture and hybridize to mRNA, which has a PolyA sequence at the 3' end of the mRNA, may include a polyT sequence. The polyT sequence may have from about 20 T nucleotides to more than 100 T nucleotides. In some embodiments, the polyT sequence may have about 30 to about 40 nucleotides. The polyT sequence may further contain two nucleotides VI at its 3' end.

First Priming sequence. The first priming sequence of the capture/priming oligonucleotide may be: 5' to the capture sequence, adjacent to the 5'-most nucleotide of the capture/priming oligonucleotide; or comprises the 5'-most nucleotide of the capture/priming oligonucleotide. The first priming sequence may be a generic or a sequence-specific priming sequence. The first priming sequence may bind to a primer that, upon binding, primes a reverse transcriptase. The first priming sequence may include about 10 to about 50 nucleotides.

Additional priming and/or adaptor sequences. The capture oligonucleotide may optionally have one or more additional priming/adaptor sequences, which either provide a landing site for primer extension (which can include extension by a polymerase) or a site for immobilization to complementary hybridizing anchor sites within a massively parallel sequencing array or flow cell. In the methods here, the second (or additional) priming sequence may be a P1 sequence (e.g., as used in Illumina sequencing chemistries, AAGCAGTGGTATCAACGCAGAGT (SEQ ID NO. 1)), but the methods are not so limited. Any suitable priming sequences may be included for other types of NGS library preparation. In some embodiments, when a P1 sequence is included as an additional priming sequence, it may be 5' to the first priming sequence. The PI additional priming sequence may also be 5' to the capture sequence.

Template switching oligonucleotide. A template switching oligonucleotide as used herein, refers to an oligonucleotide that permits the terminal transferase activity of an appropriate reverse transcriptase, such as, but not limited to Moloney murine leukemia virus (MMLV), to use the deoxycytidine nucleotides added to anchor a template switching oligonucleotide. Upon base pairing between the template switching oligonucleotide and the appended deoxycytidines, the reverse transcriptase "switches" template strands from the captured RNA to the template switching oligonucleotide and continues replication to the 5'end of the template switching oligonucleotide. Thus, a complete 5' end of the transcripted RNA is included and additional priming sequences for further amplification may be introduced. Further the cDNA is transcribed in a sequence independent manner.

BCR gene sequences. The B cell receptor gene sequence include several sub-regions including variable (V), diversity (D), joining (J) and constant (C) segments, in that order 5' to 3' in the released RNA. The constant region is just 5' to the polyA sequence. In a number of approaches to sequencing BCR, it may be desirable to construct selection strategies to obtain amplicons for sequencing that do not contain the poly A sequence (tail). Further it may be desirable to produce amplicons which retain little of the constant region. Limiting amplification to exclude these sections of the released nucleic acid sequence can permit more robust sequencing of the V, D (if present), and J segments of the BCR.

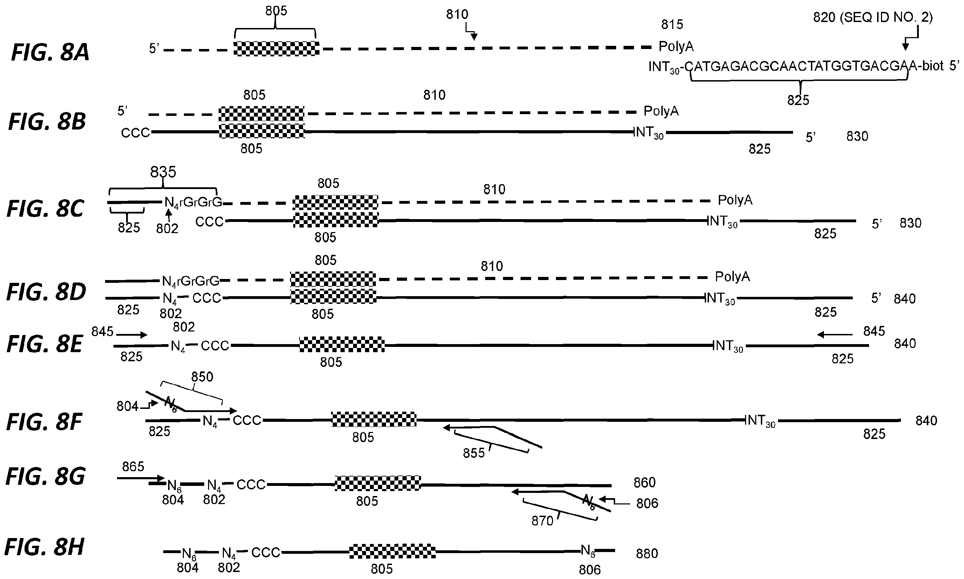

Turning to Figures 8A- 8H for a better understanding of the method, each of Figures 8A-8H represent a single species or set of associated duplexed species present at differing points in the process of obtaining a BCR sequencing library from a single cell. The process may be multiplexed such that a number of single cells may be processed to provide sequencing libraries that may be tracked back to specific originating sites within a well plate. The knowledge of this location may further be trackable back to an individual sequestration pen within a microfluidic device from which the cell has been exported. Therefore, a biological cell may be assayed for production of a desired product or for a desired ability to stimulate other cells, then trackably exported, trackably processed to provide a sequencing library, and the resultant genomic data derived from the sequencing may be correlated identifiably back to the source cell in the microfluidic device.

In Figure 8A, upon lysis of the biological cell, an mRNA 810 is released. The released mRNA 810 may include a gene sequence of interest 805 and has a poly A segment 815 at its 3' end. Capture/priming oligonucleotide 820 may include a P1 priming sequence 825 and a PolyT capture sequence (shown in Figure 8A as T30NI, which represents a sequence of 30 T nucleotides and has a two-nucleotide sequence NI at the 3' end of the capture/priming oligonucleotide 820). In some embodiments, the N nucleotide in the T30NI sequence of the PolyT capture sequence can be selected from G, C, and A (e.g., can exclude T nucleotides). The capture/priming oligonucleotide can bind to polyA sequence 815 of the released mRNA 810.

In Figure 8B, the initial process of reverse transcription is represented, where the reverse transcriptase extends the capture/priming oligonucleotide, using mRNA 810 as the template, thereby incorporating the gene of interest 805 into the transcript. Reaching the 3' terminus of mRNA 810, the reverse transcriptase adds several C (shown here as three C) nucleotides.

In Figure 8C, a transcript switching oligonucleotide (TSO) 835 is present within the reverse transcription reaction mixture, where the TSO include a PI sequence 825 and may further include a four nucleotide (N4) first barcode 802. In the example described below the TSO 835 may be an oligonucleotide having a sequence of SEQ ID NO. 3. The first barcode 802 may be used to multiplex several experiments during sequencing, and the method is not limited to requiring that first barcode 802 be present. The first barcode 802 is not limited to having four nucleotides but may have any suitable number of nucleotides to render the sequencing library product identifiable. In some embodiments, the first (multiplex) barcode may have from about 3 nucleotides to about 10 nucleotides. The TSO may further include biotin linked to its 5' end of the oligonucleotide to increase efficiency.

In the reverse transcription, the TSO aligns with the 5' end of mRNA 810 and permits the reverse transcriptase to "switch templates" and use the deoxynucleotides of the TSO as a template to extend the cDNA 830 past the three C nucleotides at its 3' end, to incorporate the first four nucleotide barcode 802 (N4) and the PI sequence 825 of the TSO, as shown in Figure 8D. The fully extended cDNA product 840 now includes a PI priming sequence at both ends, the gene of interest 805 and, optionally first barcode 802 (N4). cDNA product 840 also still includes the polyT-NI sequence incorporated from the capture sequence of the capture/priming oligonucleotide 820.

In Fig. 8E, the cDNA 840 can be amplified using PI primer 845 for both forward and reverse, in order to amplify the whole mRNA captured. In the example described below, the PI primer may have a biotin at its 5' end and may have a sequence of SEQ ID NO. 4. The sequence of the product of the amplification, amplified cDNA 840, retains all the features of the transcript arising from the reverse transcription step, including the PI sequences at both the 5' and 3' termini, gene sequence of interest 805 and first barcode 802. The first barcode is incorporated to the 5' of the gene sequence of interest 805 and to the 3' of the P1 priming sequence at the 5' end of amplified product 840.

A first polymerase chain reaction (PCR) is then performed. Figure 8F shows the schematic representation of the primer arrangement used to selectively amplify a focused region of the BCR region. Forward primer 850 is designed to bind to portions of the 5' region of the cDNA amplified product 840 that are 3' to the PI sequence 825 that had been used in the amplification of Fig. 8E. The forward primer 850 also may include a second, 6 nucleotide barcode 804 which can be used as part of the system for identifying the source well of the well plate. While a 6 nucleotide second barcode 804 is illustrated in Figures 8E- 8H, the second barcode may have any suitable number of nucleotides, and may have from about 3 nucleotides to about 10 nucleotides. The reverse primer 855 is directed to bind to a sub-region of the large constant region of the BCR, close to the 5' terminus of the constant region, where it follows the 3' terminus of the join (J) region of the BCR. This ensures that all of the variable (V), diversity (D) if present, and join (J) sub-regions fall within the sequenced portion of the amplicon. This removes the T30NI sequence and the PI sequence 825 introduced from the capture/priming sequence 820. To ensure coverage of heavy chain and of both kappa and lambda light chains, a mixture of reverse primers 855 are employed.

A second PCR amplification is performed on the selected and truncated amplification product 860 as shown in Fig. 8G. The amplification product 860 contains the gene sequence of interest 805, optional first (multiplex) barcode sequence 802 (N4), and second (well plate) barcode 804 (N6). The forward primer 865 for the second PCR binds to the sequence, 5' to the second (well plate) barcode 804, which had been introduced by the forward primer 850. The reverse primer 870 binds to the shared sequence introduced by the reverse primer(s) 855 (the 5' portion of each of the reverse primers 855). The reverse primer 870 also includes a third (well plate) barcode 806 having 6 nucleotides (N6). While a 6 nucleotide third (well plate) barcode 806 is illustrated in Figs. 8G- 8H, the third barcode may have any suitable number of nucleotides, and may have from about 3 nucleotides to about 10 nucleotides.

The final amplicon 880 is shown in Figure 8H, and contains the first, optional multiplex barcode 802, the gene sequence of interest 805, a second (well plate) barcode 804, and a third (well plate) barcode 806. Additional adaptors may be present for use in specific sequencing chemistries.

Barcodes 2 and 3 are employed across the wells of the export well plate to unequivocally identify each source well, and hence the cell from which the sequencing library has been generated. An economical approach may be to use an 8X12 distribution of unique barcodes across the wells of the well plate such only 20 unique barcodes total are necessary to identify each well. The first (multiplex) barcode may be used if multiple well plate samples are combined in a sequencing run, but is not necessary if only one well plate is sequenced in a sequencing run.

### EXAMPLES

### Example 1 - Screening Mouse Splenocytes for Secretion of IgG antibodies Capable of Binding Human CD45.

A screen was performed to identify mouse splenocytes that secrete IgG-type antibodies that bind to human CD45. The experimental design included the following steps:
1. Generation of CD45 antigen coated beads;
2. Harvest mouse splenocytes;
3. Load cells into a microfluidic device; and
4. Assay for antigen specificity.

**Table 1 - Reagents for Example 1.**

| | Name | Vendor | Catalog Number | Lot Number |
|---|---|---|---|---|
| 1 | Slide-A-Lyzer^{™} MINI Dialysis Device, 7K MWCO, 0.1mL | Thermo Pierce | 69560 | OJ189254 |
| 2 | CD45 Protein | R&D Systems | 1430-CD | 112722 |
| 3 | PBS pH 7.2 with Mg2+ and Ca2+ | Fisher | BP29404 | |
| 4 | Streptavidin Coated Beads (8 µm) | Spherotech | SVP-60-5 | AC01 |
| 5 | EZ-Link NHS-PEG4-Biotin, No-Weigh Format | Pierce | 21329 | |
| 6 | Hybridoma SFM Media | Life Tech | 12045-076 | |
| 7 | Fetal Bovine Serum | Hyclone | #SH30084.03 | |
| 8 | Penicillin-Streptomycin (10,000 U/mL) | Life | 15140-122 | |
| 9 | Goat anti-mouse F(ab')2-Alexa 568 | Life | Cat# A11019 | Lot#1073003 |
| 10 | streptavidin-488 | Life | Catalog #S32354 | Lot #1078760 |
| 11 | Mouse anti CD45 IgG₁ | R&D Systems | MAB1430 | ILP0612061 |
| 12 | BD Falcon^{™} Cell Strainers, 40µm, Blue | BD | 352340 | |

**Generation of CD45 antigen coated beads.** CD45 antigen coated microbeads were generated in the following manner:

50 micrograms carrier free CD45 was resuspended in 500 microliters PBS (pH 7.2).

A Slide-A-Lyzer dialysis mini cup was rinsed with 500 microliters PBS, then added to a microfuge tube.

50 microliters of the 0.1 microgram/ microliter CD45 solution was added to the rinsed dialysis mini cup.

170 microliters PBS was added to 2 mg of NHS-PEG4-Biotin, after which 4.1 microliters of NHS-PEG4-Biotin was added to the dialysis mini cup containing the CD45 antigen.

The NGS-PEG4-Biotin was incubated with the CD45 antigen for 1 hour at room temperature.

Following the incubation, the dialysis mini cup was removed from the microfuge tube, placed into 1.3 mls PBS (pH 7.2) in a second microfuge tube, and incubated at 4°C with rocking, for a first 1 hour period. The dialysis mini cup was subsequently transferred to a third microfuge tube containing 1.3 mls of fresh PBS (pH 7.2), and incubated at 4°C with rocking, for a second 1 hour period. This last step was repeated three more times, for a total of five 1 hour incubations.

100 microliters of biotinylated CD45 solution (∼50 ng/ microliter) was pipetted into labeled tubes.

500 microliters Spherotech streptavidin coated beads were pipetted into a microfuge tube, washed 3 times (1000 microliters /wash) in PBS (pH 7.4), then centrifuged for 5 min at 3000 RCF.

The beads were resuspended in 500 microliters PBS (pH 7.4), resulting in a bead concentration of 5 mg/ml.

The biotinylated CD45 protein solution (50 microliters) was mixed with the resuspended Spherotech streptavidin coated beads. The mixture was incubated at 4°C, with rocking, for 2 hours, then centrifuged 4° for 5 min at 3000 RCF. The supernatant was discarded and the CD45 coated beads were washed 3 times in 1 mL PBS (pH 7.4). The beads were then centrifuges at 4°C for another 5 min at 3000 RCF. Finally, the CD45 beads were resuspended in 500 microliters PBS pH 7.4 and stored at 4° C.

**Mouse Splenocyte Harvest.** The spleen from a mouse immunized with CD45 was harvested and placed into DMEM media + 10% FBS. Scissors were used to mince the spleen.

Minced spleen was placed into a 40 micron cell strainer. Single cells were washed through the cell strainer with a 10ml pipette. A glass rod was used to break up the spleen further and force single cells through the cell strainer, after which single cells were again washed through the cell strainer with a 10ml pipette.

Red blood cells were lysed with a commercial kit.

Cells were spun down at 200xG and raw splenocytes were resuspended in DMEM media + 10% FBS with 10 ml pipette at a concentration of 2e⁸ cells/ml.

**Loading Cells into Microfluidic Device.** The microfluidic device was an OptoSelect^{™} device (Berkeley Lights, Inc.), configured with OptoElectroPositioning (OEP^{™}) technology. The microfluidic device included a flow region and a plurality of NanoPen^{™} chambers fluidically connected thereto, with the chambers having a volume of about 7x10⁵ cubic microns. The microfluidic device was operated on a prototype system (Berkeley Lights, Inc.) included at least a flow controller, temperature controller, fluidic medium conditioning and pump component, light source for light activated DEP configurations, mounting stage for the microfluidic device, and a camera.

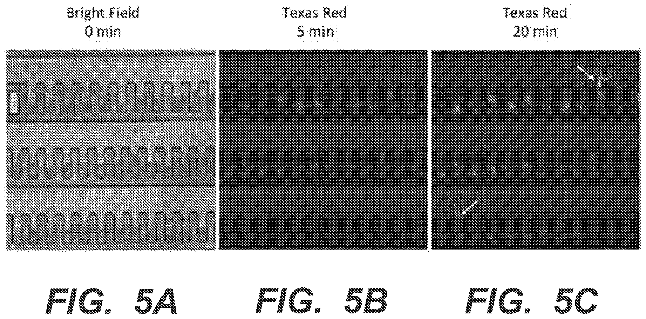

Splenocytes were imported into the microfluidic device and loaded into NanoPen chambers containing 20-30 cells per NanoPen chamber. 100 microliters of media were flowed through the device at 1microliter/sec to remove unwanted cells. Temperature was set to 36°C, and culture media was perfused for 30 minutes at 0.1 microliters/sec. Brightfield imaging as shown in Figure 5A showed the location of the cells within the NanoPen chambers.

**Antigen Specificity Assay.** Media containing 1:2500 goat anti-mouse F(ab')2-Alexa 568 was prepared.

100 microliters of CD45 beads were re-suspended in 22 microliters of the media containing the 1:2500 dilution of goat anti-mouse F(ab')2-Alexa 568 secondary antibody.

The resuspended CD45 beads were next flowed into the main channel of the microfluidic chip at a rate of 1 microliter/sec until they were located adjacent to, but just outside the NanoPen chambers containing splenocytes. Fluid flow was then stopped.

The microfluidic chip was then imaged in bright field to determine the location of the beads (not shown). Next, a Texas Red Filter was used to capture images of the cells and beads. Images were taken every 5 minutes for 1 hr, with each exposure lasting 1000 ms and a gain of 5. As shown in Figure 5B, imaging a timepoint 5 min after introduction of the beads/labeled secondary antibody mixture showed fluorescent signal becoming evident at the site of cells within some pens. Labelling of the cells indicated presence of IgG on the surface of cells. Faint labelling of beads was observed at this timepoint.

**Results.** Positive signal was observed developing on the beads at a timepoint of 20 minutes post bead/antibody mixture introduction, reflecting the diffusion of IgG-isotype antibodies diffusing out of certain pens and into the main channel of the microfluidic device, where they were able to bind the CD45-coated beads. Binding of anti-CD45 antibody to the beads allowed for the secondary goat anti-mouse IgG-568 to associate with the beads and produce a detectable signal. See Figure 5C, white arrows.

Using the methods of the invention, each group of splenocytes associated with positive signal could be separated and moved into new pens as a single cell and reassayed. In this manner, single cells expressing anti-CD45 IgG antibodies could be detected and isolated.

### Example 2: Activation and Screening of Memory B Cells in a Microfluidic Device

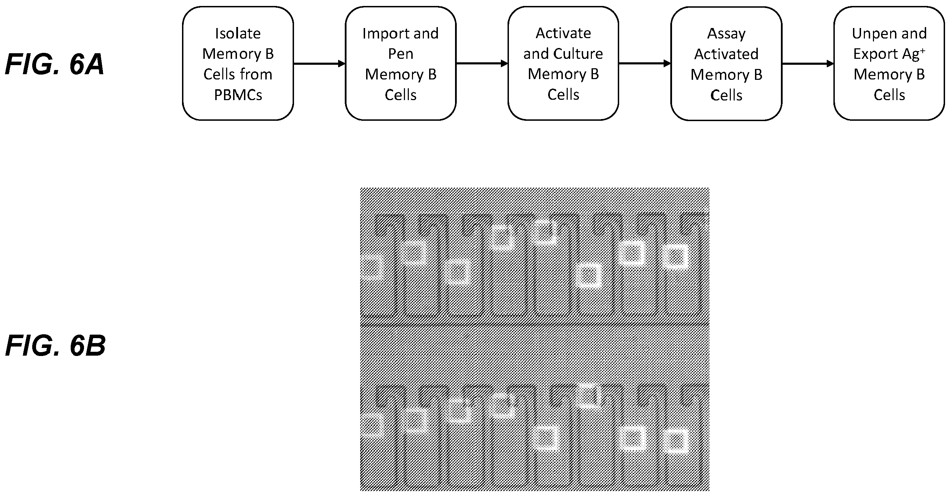

A general method for screening memory B cells in a microfluidic device is outlined in Fig. 6A. The foregoing method is focused on human memory B cells, but the method can be used to screen B cells from other animals.

**Harvest Memory B Cells.** Frozen human peripheral blood mononuclear cells (PBMCs) are thawed and mixed with a 6X volume of RPMI 1640 (Gibco) supplemented with 10% FBS (Seradigm), counted, and centrifuged at 500g for 5 min. The supernatant is aspirated away and the cell pellet is resuspended to a concentration of 5x10⁷ cells/mL in FACS buffer (PBS, 2% BSA, ImM EDTA).

Next, a B cell enrichment is performed using an EasySep Human B cell Enrichment Kit (EasySep, #19054). 50 microliters of B cell enrichment cocktail is added for each mL of human PBMCs and the resulting mixture is incubated at room temperature for 10 minutes. 75 microliters of magnetic particles for each mL of human PBMCs is then added, and the mixture is mixed well and incubated at room temperature for 10 minutes. An approximately 1.1 uL volume of the PBMC cell suspension is brought to 2.4 mL by adding FACS buffer, then mixed well by pipetting up and down. A tube containing the PBMC suspension is then placed into the EasySep magnet (without lid) and incubated for 5 minutes. While maintaining the tube in the EasySep magnet, an enriched B cell suspension is poured into a new, clean tube. A cell count of the enriched B cell suspension is performed, after which the cells are centrifuged at 300g for 5 minutes. The supernatant is aspirated away.

The enriched B cell pellet is resuspended to a concentration of 5x10⁷ cells/mL in FACS buffer containing anti-CD27 antibody and then incubated at 4°C for 20 minutes in the dark. After the incubation, the cells are washed 2X with 3 mL FACS buffer and centrifuging the suspension at 300g for 5 minutes. The final enriched B cell pellet is resuspended in FACS buffer to a concentration of 5x10⁷ cells/mL and then passed through a single-cell strainer, with the pipette tip pressed against (and perpendicular to) the mesh of the strainer. The strained cell suspension is maintained on ice until FACS sorting. Using a FACS Aria instrument, CD27⁺ B cells are sorted into B Cell Activation/Culture Medium (RPMI 1640 (Gibco), 10% FBS (Seradigm), 2 ug/mL CpG (Invivogen), 1 ug/mL IL-2 (Peprotek), 5 ng/mL IL-4 (Peprotek), 10 ng/mL IL-6 (Peprotek), 10 ng/mL IL-21 (Peprotek), and 10 ng/mL BAFF (Peprotek)).

The isolated memory B cells are adjusted to a concentration of 2x10⁶ cells/mL and then incubated at 37°C until import into the microfluidic device, which is performed as soon as possible.

**Preparation of microfluidic device and import of memory B cells.** The microfluidic device is an OptoSelect^{™} device (Berkeley Lights, Inc.), configured with OptoElectroPositioning (OEP^{™}) technology and having conditioned internal surfaces that include a layer of covalently-linked polyethylene glycol (PEG) polymers. The microfluidic device includes a flow region having a plurality of microfluidic channels and a plurality of sequestration pens (or NanoPen^{™} chambers) fluidically connected to each microfluidic channel, with the sequestration pens having a volume of about 5x10⁵ cubic microns. The microfluidic device is operated on a Beacon platform (Berkeley Lights, Inc.) or a prototype Alpha platform (Berkeley Lights, Inc.), with the platform including a flow controller, temperature controller, fluidic medium conditioning and pump component, light source for light activated DEP configurations, mounting stage for the microfluidic device, and a camera.

250 microliters of 100% carbon dioxide are flowed into the microfluidic device at a rate of 12 microliters/sec. This is followed by 250 microliters of a priming medium containing 1000 ml Iscove's Modified Dulbecco's Medium (ATCC), 200 ml Fetal Bovine Serum (ATCC), 10 ml pen-strep (Life Technologies), and 10 mL Pluronic F-127 (Life Technologies). Introduction of B cell culture medium containing RPMI 1640 (Gibco) supplemented with 10% FBS (Seradigm), 1X Pen-Strep (Gibco), and 1X Kanamycin Sulfate (Gibco) follows.

The isolated memory B cell suspension prepared as above is next imported into the microfluidic device by flowing the suspension into an inlet and stopping the flow when the memory B cells are located within the flow region/microfluidic channels. Memory B cells are then loaded into the sequestration pens, with a target of one B cell per pen. The memory B cells are moved from the flow region/microfluidic channels into the isolation regions of the sequestration pens using light-activated DEP force (OEP technology). The parameters for operating OEP include applying an AC potential across the microfluidic device (voltage 3.5 V, frequency 2 MHz), using structured light to form light traps that trap individual cells (as shown in Fig. 6B), and moving the light traps at 8 microns/sec. Cells remining in the flow region/microfluidic channels after penning are flushed from the microfluidic device.

**Memory B cell activation.** Beads coated with Protein A (Spherotech) are mixed with irradiated Jurkat D1.1 feeder cells at a ratio of about 1:1. The bead/feeder cell mixture is then flowed into the microfluidic device, and feeder cells and beads are bulk loaded into each sequestration pen containing a memory B cell. Bulk loading is achieved by tilting the microfluidic device on end and allowing gravity to pull the cells and beads down into the sequestration pens. The bead/feeder cell mixture is flowed into the microfluidic device at a concentration of about 1.5x10⁷ per mL of each of feeder cells and beads, and sequestration pens bulk loaded in this manner receive an expected average of about 10 feeder cells and about 10 beads per pen.

The microfluidic device is then moved to a culture station and B Cell Activation/Culture Medium (above) is perfused through the flow path of the microfluidic device for a period of four (4) days. The microfluidic device is maintained in a tilted, on end position while maintained on the culture station. The perfusion method is as follows: perfuse B Cell Activation/Culture Medium at 0.02 microliters/sec for 100 seconds; stop flow for 500 seconds; perfuse B Cell Activation/Culture Medium at 2 microliters/sec for 64 seconds; and repeat.

**Assaying the activated memory B cells.** After 4 days of culture/activation, the microfluidic device is removed from the culture station and returned to the Beacon/Alpha system, whereupon a multiplex assay is performed to detect IgG secretion and antigen specificity. The multiplex assay, illustrated in Fig. 6C, includes anti-human IgG antibody-coated capture beads (Spherotech), an anti-human IgG-Alexa Fluor 488 secondary antibody (Invitrogen), and an antigen of interest labeled using Alexa Fluor 647 carboxylic acid succinimydyl ester. The capture beads, secondary antibody, and labeled antigen of interest are mixed together in B Cell Activation/Culture Medium and flowed into the flow region/microfluidic channels of the microfluidic device. Flow is stopped, and images of the sequestration pens are taken periodically for a period of 10 to 25 minutes, using appropriate filters for visualizing the fluorescent labels. Memory B cells that secrete an antibody which binds to the antigen of interest will induce an association between the labeled antigen of interest and the IgG antibody-coated capture beads. As a result, a "plume" of fluorescently-labeled capture beads will appear at the opening between the flow region/microfluidic channel and the sequestration pen in which the memory B cell is located. Fig. 6D shows typical assay results for memory B cells.

**Export and further processing**. Memory B cells identified as secreting an antibody which binds to antigen of interest are next unpenned one pen at a time using DEP force (using OEP parameters discussed above), and exported from the microfluidic device into a well of a 96-well plate by flowing export medium (DPBS with Ca2+ and Mg2+ (Lonza), 5mg/mL BSA (Sigma), and 1:100 Pluronic^{™} F-127 (Thermo Fisher)) through the flow path of the microfluidic device. Following export, the memory B cells are lysed and transcripts encoding the heavy and light chain antibody sequences are reverse transcribed into cDNAs and sequenced.

**Results:** Using protocols substantially the same as the foregoing protocol, rates of B cell activation (as measured by detection of IgG secretion) have reached about 12% for human memory B cells. Rates for cell activation of non-human mammalian memory B cells have reached as high as 40%. Rates for detection of activated memory B cells expressing an antibody that binds to an antigen of interest are dependent upon the antigen of interest, but are typically around 1% or less. In one experiment to test the relevancy of putative Ag⁺ antibodies obtained from human memory B cells by such screening protocols, a set of 20 memory B cells identified as secreting Ag-binding antibodies were exported from a microfluidic device and their antibody heavy chains and light chains sequences were determined. Following re-expression in HEK 393T cells and ELISA analysis with the antigen of interest used in the on chip assay, 16 of 20 (or 80%) of the antibodies detected the antigen of interest in the ELISA assay. This confirms the relevancy of activating and screening memory B cells according to the presently disclosed methods.

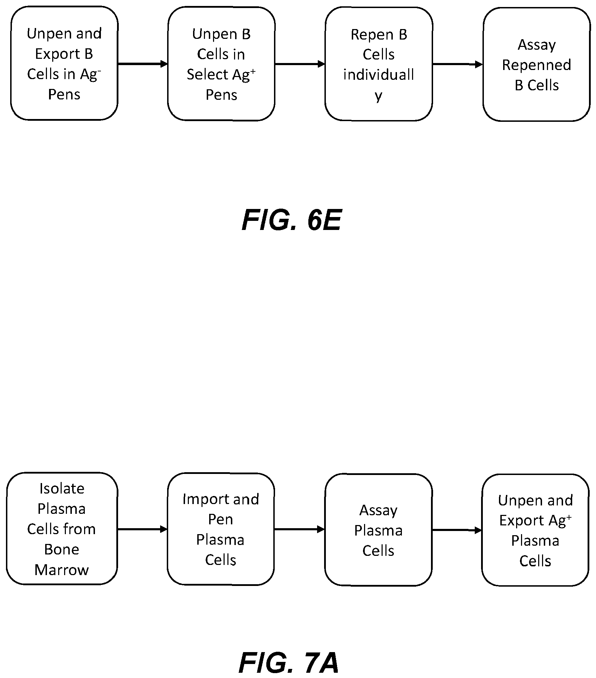

**Variations.** The foregoing method can be varied in many ways and still achieve the goal of direct screening of memory B cells. These variations include:
1. Screening of memory B cells isolated from animals other than humans, including other mammalian species, such as rodents (e.g., mouse, rat, guinea pig, gerbil, hamster), rabbits, ferrets, livestock (e.g., goats, sheep, pigs, horses, cows), llama, camel, and avian species, such as chickens and turkey.
2. The OEP operating parameters used to pen and unpen memory B cells can be varied. For example, AC potential across the microfluidic device can be set at about 2 to about 5 volts, with a frequency of about 1 to about 3 MHz. Specific examples include (i) a voltage of about 2.5 V and a frequency of about 3 MHz, and (ii) a voltage of about 4.5 V and a frequency of about 1 MHz. In addition, the speed at which the structured light (or light cage) is moved can be varied between about 5 to about 10 microns/sec.
3. As described above, the microfluidic device is maintained on a culture station, with the chip tilted on end. Alternatively, the microfluidic device can be placed back on the Beacon/Alpha system in the standard position (i.e., with the microfluidic device laid substantially flat). Memory B cell culture/activation medium is then perfused through the flow region of the microfluidic device according to the following protocol: perfuse B cell culture/activation medium at 0.01 microliters/sec. for 2 hours; perfuse B cell culture/activation medium at 2 microliters/sec. for 64 seconds; and repeat.
4. The assay can be further multiplexed to include second antigens of interest or even second and third antigens of interest. See, for example, Fig. 6C. In this manner, the memory B cells can be screened simultaneously for antibodies that bind to different epitopes on the same target protein/molecule, antibodies that exhibit different levels of cross-species reactivity to the target protein/molecule, or simply antibodies that bind to completely different antigens of interest. In addition, the use of second and/or third antigens of interest allows for high throughput screening.
5. The size of the sequestration pens in the microfluidic device can be increased, for example, to about 1.1x10⁶ cubic microns. Typically, the pens will have a volume of about 5x10⁶ cubic microns or less (e.g., about 4x10⁶ cubic microns, about 3x10⁶ cubic microns, about 2.5x10⁶ cubic microns, about 2x10⁶ cubic microns, about 1.5x10⁶ cubic microns, or less). Larger size can be useful for the initially assaying polyclonal groups of memory B cells, but the larger size also delays the multiplex assay and can potentially negatively impact memory B cell activation and growth.
6. The assay can start as a polyclonal assay, then shift to a monoclonal assay. In this approach, the sequestration pens are initially loaded with a plurality of memory B cells (e.g., 2 to 10, or 4 to 10). When this approach is taken, sequestration pens that show up as Ag⁺ in the initial assay must be further analyzed to determine which memory B cell in the sequestration pen is producing that Ag⁺ antibody. To do this, cells in Ag⁻ pens are unpenned and exported (e.g., by flowing export medium through the flow region/microfluidic channels to a discard tube). Next, the cells in Ag+ pens are unpenned and single memory B cells are re-penned into empty pens located adjacent to or nearby the source pen on the microfluidic device. The multiplex assay for Ag+ pens is then repeated, and memory B cells located in any pens identified as Ag+ in the repeated assay are exported for further processing. This higher throughput polyclonal-to-monoclonal approach adds additional steps, which are outlined in Fig. 6E, to the method outlined in Fig. 6A.

### Example 3: Screening of Plasma Cells in a Microfluidic Device

A general method for screening plasma cells in a microfluidic device is outlined in Fig. 7A. The foregoing method is focused on human plasma cells, but the method can be used to screen plasma cells from other animals.

**Harvest Plasma Cells**. Frozen human bone marrow (BM) cells are thawed rapidly in a 37°C water bath, then added dropwise to 5 mL of pre-heated (37°C) Plasma Cell Culture Medium (RPMI 1640 (Gibco), 10% FCS (Hyclone), 1X non-essential amino acid (NEAA) solution (Gibco), 1X sodium pyruvate (Gibco), 50 uM beta mercaptoethanol (Gibco), and 1X pen-strep (Gibco)) supplemented with 1X DNase (Benzonase^{®} Nuclease 1000X stock containing 25,000 U/mL, Millipore). The resulting mixture is centrifuged at 300g for 10 minutes, and the cell pellet is washed 2X with FACS buffer (PBS, 2% BSA, ImM EDTA).

The cell pellet obtained after the washes in FACS buffer is resuspended to a concentration of 1x10⁷ cells/mL in FACS buffer containing anti-CD138 antibody and then incubated at 4°C for 20 minutes in the dark. After the incubation, the cells are washed 2X in FACS buffer, and resuspended in FACS buffer to a concentration of 1x10⁷ cells/mL. The cell suspension is maintained on ice until FACS sorting. Using a FACS Aria instrument, CD138⁺ plasma cells are sorted into a Plasma Cell Culture Medium (above) supplemented with 40 ug/mL IL-6 (R&D Systems).

The isolated plasma cells are adjusted to a concentration of 2x10⁶ cells/mL and then incubated at 37°C until import into the microfluidic device, which is performed as soon as possible.

**Preparation of microfluidic device and import of plasma cells**. The microfluidic device is an OptoSelect^{™} device (Berkeley Lights, Inc.), configured with OptoElectroPositioning (OEP^{™}) technology and having conditioned internal surfaces that include a layer of covalently-linked polyethylene glycol (PEG) polymers. The microfluidic device includes a flow region having a plurality of microfluidic channels and a plurality of sequestration pens (or NanoPen^{™} chambers) fluidically connected to each microfluidic channel, with the sequestration pens having a volume of about 5x10⁵ cubic microns. The microfluidic device is operated on a Beacon platform (Berkeley Lights, Inc.) or a prototype Alpha platform (Berkeley Lights, Inc.), with the platform including a flow controller, temperature controller, fluidic medium conditioning and pump component, light source for light activated DEP configurations, mounting stage for the microfluidic device, and a camera.

250 microliters of 100% carbon dioxide are flowed into the microfluidic device at a rate of 12 microliters/sec. This is followed by 250 microliters of a priming medium containing 1000 ml Iscove's Modified Dulbecco's Medium (ATCC), 200 ml Fetal Calf Serum (Hyclone), 10 ml pen-strep (Life Technologies), and 10 mL Pluronic F-127 (Life Technologies). Introduction of Plasma Cell Culture Medium (above) supplemented with 40 ug/mL IL-6 (R&D Systems) follows.

The isolated plasma cell suspension prepared as above is next imported into the microfluidic device by flowing the suspension into an inlet and stopping the flow when the plasma cells are located within the flow region/microfluidic channels. Plasma cells are then loaded into the sequestration pens, with a target of one plasma cell per pen. The plasma cells are moved from the flow region/microfluidic channels into the isolation regions of the sequestration pens using light-activated DEP force (OEP technology). The parameters for operating OEP include applying an AC potential across the microfluidic device (voltage 2.5 V, frequency 3 MHz), using structured light to form light traps that trap individual cells (similar to as shown in Fig. 6B), and moving the light traps at 8 microns/sec. Cells remining in the flow region/microfluidic channels after penning are flushed from the microfluidic device.

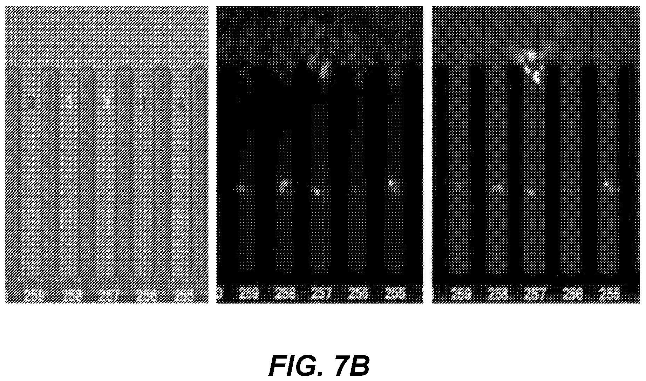

**Assaying the plasma cells**. Immediately following penning, the plasma cells are assayed to detect IgG secretion and antigen specificity. The multiplex assay, illustrated in Fig. 6C, includes anti-human IgG antibody-coated capture beads (Spherotech), an anti-human IgG-Alexa Fluor 488 secondary antibody (Invitrogen), and an antigen of interest labeled using Alexa Fluor 647 carboxylic acid succinimydyl ester. The capture beads, secondary antibody, and labeled antigen of interest are mixed together in Plasma Cell Culture Medium (above) supplemented with 40 ug/mL IL-6 (R&D Systems), and flowed into the flow region/microfluidic channels of the microfluidic device. Flow is stopped, and images of the sequestration pens are taken periodically for a period of 10 to 25 minutes, using appropriate filters for visualizing the fluorescent labels. Plasma cells that secrete an antibody which binds to the antigen of interest will induce an association between the labeled antigen of interest and the IgG antibody-coated capture beads. As a result, a "plume" of fluorescently-labeled capture beads will appear at the opening between the flow region/microfluidic channel and the sequestration pen in which the plasma cell is located. Fig. 7B shows typical assay results for plasma cells, with a brightfield image in the left panel, a fluorescent image of the anti-human IgG secondary antibody in the middle panel, and a fluorescent image of the labeled antigen of interest in the right panel.

**Export and further processing.** Plasma cells identified as secreting an antibody which binds to antigen of interest are next unpenned one pen at a time using DEP force (using OEP parameters discussed above), and exported from the microfluidic device into a well of a 96-well plate by flowing export medium (DPBS with Ca2+ and Mg2+ (Lonza), 5mg/mL BSA (Sigma), and 1:100 Pluronic^{™} F-127 (Thermo Fisher)) through the flow path of the microfluidic device. Following export, the plasma cells are lysed and transcripts encoding the heavy and light chain antibody sequences are reverse transcribed into cDNAs and sequenced.

**Results:** The foregoing protocol is performed in less than a day. Using protocols substantially the same as the foregoing, rates for detection of plasma cells expressing an antibody that binds to an antigen of interest (which are dependent upon the antigen of interest) are typically around 1% or less, and putative Ag⁺ antibodies obtained from plasma cells by such protocols have exhibited Ag-specific binding upon re-expression at a rate as high as 82% in one study.

**Variations.** The foregoing method can be varied in many ways and still achieve the goal of direct screening of plasma cells. These variations include:
1. Screening of plasma cells isolated from animals other than humans, including other mammalian species, such as rodents (e.g., mouse, rat, guinea pig, gerbil, hamster), rabbits, ferrets, livestock (e.g., goats, sheep, pigs, horses, cows), llama, ad avian species, such as chickens and turkey.
2. A B cell enrichment can be performed prior to FACS isolation of the plasma cells, for example, using an EasySep Human B cell Enrichment Kit (EasySep, #19054).
3. The OEP operating parameters used to pen and unpen plasma cells can be varied. For example, AC potential across the microfluidic device can be set at about 2 to about 5 volts, with a frequency of about 1 to about 3 MHz. Specific examples include (i) a voltage of about 3.5 V and a frequency of about 2 MHz, and (ii) a voltage of about 4.5 V and a frequency of about 1 MHz. In addition, the speed at which the structured light (or light cage) is moved can be varied between about 5 to about 10 microns/sec.
4. The sequestration pens into which the plasma cells are loaded can differ in size. For example, the pens can have a volume of about 1.1x106 cubic microns. Typically, the pens will have a volume of about 5x10⁶ cubic microns or less (e.g., about 4x10⁶ cubic microns, about 3x10⁶ cubic microns, about 2.5x10⁶ cubic microns, about 2x10⁶ cubic microns, about 1.5x10⁶ cubic microns, or less). By reducing the size of the sequestration pens, the multiplex assay can be performed more rapidly, thereby avoiding prolonged screening periods during which the plasma cells can undergo cell death.
5. Rather than exporting plasma cells that express Ag+ antibodies, the plasma cells can be lysed within the sequestration pen in the presence of a barcoded bead designed to capture mRNA released by lysed cells. The captured mRNA can then be reverse transcribed into a cDNA library which is attached to the barcoded bead, and the barcoded bead can be exported for subsequent sequencing of the cDNA library off chip. Methods of on chip cell lysis, mRNA capture, and cDNA library generation have been described, for example, in PCT International Application No. PCT/US17/54628, filed September 29, 2017, the entire contents of which is incorporated herein by reference.

### Example 4: Single cell export of antibody expressing B lymphocytes and production of a sequencing library directed against B cell receptor regions

**Table 2. Primers used in this experiment. All primers provided at 10 micromolar concentrations.**

| SEQ ID NO. | Sequence | Identifier |
|---|---|---|
| 2 | biot- AAGCAGTGGTATCAACGCAGAGTACTTTTTTTTTTTT TTTTTTTTTTTTTTTTTTVI | Biotin dTVI |
| 3 | biot- GTGGTATCAACGCAGAGTACACGACGCTCTTCCGATC TNNNNrGrGrG | biot_barcoded TSO |
| 4 | biot-AAGCAGTGGTATCAACGCAGAGT | biot_P1 |
| 5 | GACCACCGAGATCTACACNNNNNNACACTCTTTCCCT ACACGACGCTCTTCCGATCT | Forward Primer 1 (FP1) |
| 6 | ACTGGAGTTCAGACGTGTGCTCTTCCGATCTCTGGAC AGGGATCCAGAGTTCCA | Reverse Primer 1 (RP1) for murine Hc |
| 7 | ACTGGAGTTCAGACGTGTGCTCTTCCGATCTTCGTTCA CTGCCATCAATCTTCCA | Reverse Primer 1 (RP1) for murine Kc |
| 8 | ACTGGAGTTCAGACGTGTGCTCTTCCGATCTAGGKGT ACCATYTRCCTTCCA | Reverse Primer 1 (RP1) for murine λc |
| 9 | AATGATACGGCGACCACCGAGATCTACAC | Forward Primer 2 (FP2) |
| 10 | CAAGCAGAAGACGGCATACGAGATNNNNNNGTGACT GGAGTTCAGACGTGT | Reverse Primer 2 (RP2) |

**Cells:** OKT3 cells, a murine myeloma hybridoma cell line, were obtained from the ATCC (ATCC^{®} Cat. # CRL-8001^{™}). The cells were provided as a suspension cell line.

Cultures were maintained by seeding about 1x10⁵ to about 2x10⁵ viable cells/mL and incubating at 37°C, using 5% carbon dioxide in air as the gaseous environment. Cells were split every 2-3 days. OKT3 cell number and viability were counted and cell density is adjusted to 1x10⁶/ml for loading to the microfluidic device.

**Export plate:** A 96-well full skirted plate (VWR Cat. # 95041-436) was used for the cell export. Each plate was prepared by dispensing 10 microliters mineral oil (Sigma Cat # M5904) followed by 5 microliters of 2X TCL buffer (Qiagen Cat. #1070498). (Other lysis buffers may be suitably used as well, such as Single Cell Lysis Kit, Ambion Catalog No. 4458235 or Clontech lysis buffer, Cat #635013.) The export plate was centrifuged at 200g for 1 min at room temperature and stored at room temperature until use.

**Export Buffer:** Dulbecco's Phosphate Buffered Saline (DPBS) + calcium + magnesium (1000 mL, Lonza Cat. # 17-513F); Bovine Serum Albumin (BSA) (powder, 5g, Fisher Scientific Cat. #BP9706-100); Pluronic F-127 (10ml, Life Technologies Cat. # 50-310-494; and recombinant ribonuclease inhibitor (RNaseOUT) (Life Technologies Cat. #107777019) at 1 microliter/ml final concentration. The export buffer was filtered before use with a 0.22 micron filter unit (VWR Cat. #73520-985).

**Cell Export and Lysis:** OKT3 cells (any kind of primary B cell may be used) were flowed into the microfluidic device and introduced into the NanoPen chambers using the OptoElectroPositioning (OEP) capability of the system, to provide a final distribution of one cell per NanoPen chamber. An IgG assay as described in Example 1 was performed (antigen specific assays may also be used). Cells identified to have IgG expression (and, optionally antigen-specific antibody expression) of interest were individually exported into the 96-well export plate at one cell per well in a 5 microliter export volume. Export was performed using a mixture of OEP forces to export a selected cell out of the NanoPen chamber it had resided within, and then flow of media in the flow region/microfluidic channel exported each selected cell individually in the 5 microliter volume. The export well plate was spun down immediately after the export at 200g for 5 min. at 4°C. The plate was frozen at -80°C until RNA isolation and cDNA synthesis was performed. Well plates were maintained suitably for up to at least one month under these conditions. In some situations, overnight storage or storage for up to one week may be performed.

**RNA Isolation.** The single cell export plate was thawed on ice for 15min and subsequently brought to room temperature. RNAClean XP SPRI beads (Beckman Coulter #A63987) were brought to room temperature and 10 microliters of the bead mixture (IX volume) was added to each well. (1x volume SPRI beads showed higher RNA recovery compared to standard 1.8x to 2.2x.)

Lysate and bead mixture were incubated at room temperature for 15 min. This extended period of incubation provided improved binding of released RNA. The plate was subsequently transferred on to a 96-well plate magnet (MagWell^{™} Magnetic Separator 96, Cat. #57624) and incubated for 5 min. Supernatant was carefully removed and ethanol wash was performed by adding 100 microliters of 80% ethanol (Sigma Cat. # E7023, prepared fresh). After approximately 30 sec., ethanol was aspirated and the ethanol wash was repeated. After the final aspiration the plate was removed from the 96-well plate magnet and the beads were dried for 5 min.

**cDNA synthesis.** The plate was transferred to 4C and the beads were resuspended in 4ul of "RT mix 1": containing 0.8 microliters RNase free water (Ambion Cat no AM9937); 1 microliter of 1:5M ERCC control RNA (ThermoFisher Scientific Cat. # 4456740); 1 microliter of dNTPs (10 mM each, NEB, #N0447L); 1 microliter of biotin-dTVI RNA capture/priming oligonucleotide (SEQ ID NO. 2); and 0.2 microliters of RNaseOUT (4U/microliter, Life Technologies Cat. #107777-019). The 3' inosine of the capture sequence of the biotin-dTVI RNA capture/priming oligonucleotide provided increased binding to released RNA as inosine may bind to any natural nucleotide. The capture sequence having a 3' inosine can provide better capture of released RNA than a capture sequence including a final "N" nucleotide, which may bind to the mRNA only 25% of the time. A schematic of the capture by the capture/priming sequence is shown in Figure 8A as described above. The ERCC RNA controls provided an internal RT control and also provided carrier RNA improving reverse transcription efficiency. The plate was incubated at 72°C for 5 min and immediately transferred to 4°C. 4 microliters of "RT mix 2" containing 1 microliter of betaine (5M, Sigma Cat. #B030075VL); 1.5 microliter of 5X RT mix (Thermo,#EP0753), 0.5microliters of biotinylated barcoded-Template Switching Oligonucleotide (biot_barcoded TSO; SEQ ID NO. 3), 0.5 microliters of 120mM MgCl2 (125mM, Life Technologies Cat. #AM9530G), 0.4 microliters of RNase OUT and 0.1 microliter of Maxima RNaseH minus reverse transcriptase (200U/microliter, Thermo Fisher Cat. #EP0753) was added to each well. Following the additional of the "RT mix 2", reverse transcription was carried out at 42°C for 90min followed by 10 cycles of: 50°C for 2min/42C for 2min. The last thermal cycle was followed by heat inactivation at 75°C for 15 min. The four nucleotide barcode "NNNN" of the biot_barcoded TSO provided internal barcoding for potential multiplex sequencing experiment of multiple export plates, and was not a required feature of this method. A schematic of the initial strand extension is shown in Figure 8B, and the TSO association is shown in Figure 8c, and the complete transcript product of the reverse transcription is shown in Figure 8D.

**Whole mRNA Amplification.** Following cDNA synthesis, the export plate was centrifuged 200g for 5min and 17microliters of PCR mix containing 12.5 microliters 2X Kapa Hi Fi HotStart ReadyMix (Roche Cat. # KK2602), 1 microliter of PI primer (biot_P1, SEQ ID NO. 4) and 3.5microliters of nucleotide-free water (Ambion Cat. #AM9937) was added and PCR was carried out at 98°C for 3min followed by 20 cycles of: 98°C for 15s, 65°C for 30s, 72°C for 5 min, and a final extension of 5min at 72°C was performed. The final extension period was long enough for the polymerase to amplify long cDNA molecules (greater than 2kb). A schematic of this amplification is shown in Figure 8E.

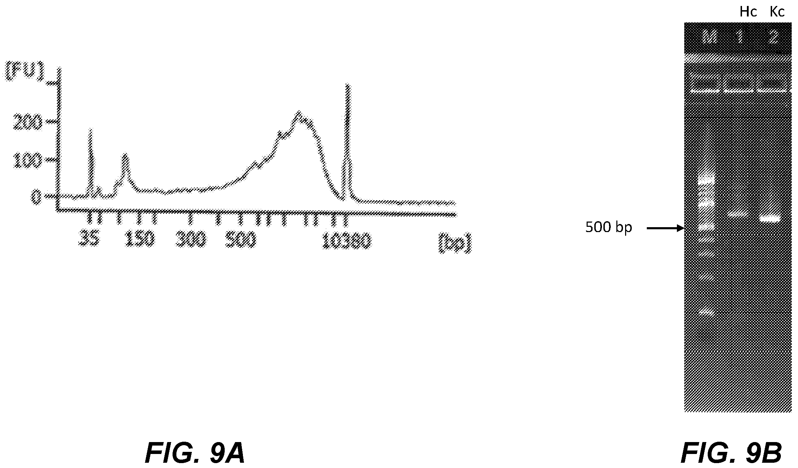

**PCR clean-up**. 25 microliters (1x volume) DNAClean SPRI beads (Beckman Coulter, Cat. # A62881) were added to each well and mixed well, removing primer-dimer and short degraded RNA products which could contaminate the downstream amplification. The mixture was incubated for 10 min at room temperature. Following incubation, the plate was placed on the well plate magnet for 5 min. Supernatant was carefully removed and ethanol wash was performed by adding 100 microliters of 80% ethanol (prepared fresh). After approximately 30sec, ethanol was aspirated. The ethanol wash procedure was repeated once. After the final aspiration the export well plate was removed from the well plate magnet and the beads were dried for 5 min. DNA was eluted from the dried beads with 15 microliters of nuclease-free H2O. Figure 9A showed the BioAnalyzer electropherogram trace for the product, which showed the expected distribution around about 1800bp for an expected average length of the full length cDNA. The typical amount of cDNA was estimated to be approximately 2 ng to about 20 ng after the amplification and cleanup has been performed.

**BCR Amplification and Barcoding.** B-Cell Receptor (BCR) amplification and barcoding of single cells was performed in a 2 step Polymerase Chain Reaction (PCR).

**PCR 1.** In the first PCR, forward primer (FP1, SEQ ID NO. 5) was designed to hybridize to the 3' end of the PI sequence incorporated from the bio_barcoded_TSO, thereby eliminating the PI sequence incorporated in the whole RNA amplification step above. The reverse primer(s) (RP1, SEQ ID NOs. 6, 7, 8) binds to the constant region of the B cell receptor gene segment close to the joint (J) gene segment of heavy and light chains. A schematic of PCR 1 is shown in Figure 8F. The forward primer contained 12 different 6 nucleotide barcodes (NNNNNN) going across the 12 columns of the plate. PCR 1 thus performed a selection for BCR containing RNA sequences out of the whole RNA amplification product, and further selected for a region of the BCR focusing on the variable, joining and diversity segments of the BCR. The amplified product (shown as amplified product 860 of Figure 8F) of this selective amplification further did not contain the polyA/polyT capture sequence nor most of the constant region of the BCR, neither of which provide data of interest.

PCR 1 was performed in a 10 microliter reaction containing 1 microliter of amplified transcriptome, 5 microliter of 2X Kapa Hi Fi HotStart ReadyMix, 0.1 microliter of forward barcoded primer (FP1), 0.2 microliter of reverse heavy constant primer (RP1 for murine Hc, SEQ ID NO. 6), 0.1 microliters of light constant primer, which was a 1:1 mixture of RP1 for murine Kc and RP1 for murine λc (SEQ. ID NOs. 6 and 7) and 3.6 microliter of nuclease free water. Cycling conditions: 98°C for 3min; followed by 5 cycles of: 98°C for 20sec, 70°C for 45sec, and 72°C for 45sec; followed by 10 cycles of: 98°C for 20sec, 68°C for 45sec, and 72°C for 45sec; followed by 10 cycles of: 98°C for 20sec, 65°C for 45sec, and 72° for C 45sec; and finally, an extension of 5min at 72°C.

PCR 2. In the second PCR, a single forward primer (FP2, SEQ ID NO. 9) binds to the barcoded FP1 and 8 barcoded (NNNNNN) reverse primers (RP2, SEQ ID NO. 10) were used across 8 rows of the plate. This strategy permitted the use of only 20 barcodes to uniquely barcode each well in the entire 96 well plate. Multiple plates can be combined with the internal plate barcode incorporated from the biot_barcoded_TSO used in the cDNA synthesis. A schematic of PCR 2 is shown in Figure 8G. A schematic representation of the final product, amplicon 880 is shown in Figure 8H.

PCR 2 is performed using PCR 1 product as the template (e.g., product 860 of Figure 8G). A 10 microliter reaction was carried out containing 1 microliter of PCR 1 product, 5 microliters of 2X Kapa Hi Fi HotStart ReadyMix, 0.1 microliter of forward primer FP2, 0.1 microliter of reverse barcoded primer RP2, and 3.8 microliters of nuclease free water. Figure 9B showed the results of gel electrophoresis for a single cell amplicon provided by this method. The arrow points to the band on the reference sizing ladder (lane M) which represents 500bp. In lane 1, a single cell amplified for heavy chain (Hc), and in lane 2 a single cell amplified for light chain Kc each showed bands of appropriate length for each amplification product. Cycling conditions: PCR was carried out at 98C for 3min; followed by 5 cycles of 98°C for 20sec, 68° for C 45sec, and 72°C for 45sec; followed by 15 cycles of 98°C for 20sec, 65°C for 45sec, and 72°C for 45sec; and finally, an extension of 5min at 72°C.

**Amplicon pooling, cleanup and sequencing.** Amplicons were pooled. 1x volume DNAClean SPRI beads (Beckman Coulter, Cat. # A62881) were added to each well and mixed well. The mixture was incubated for 10 min at room temperature. Following incubation, the plate was placed on the well plate magnet for 5 min. Supernatant was carefully removed and ethanol wash was performed by adding 100 microliters of 80% ethanol (prepared fresh). After approximately 30sec, ethanol was aspirated and the ethanol wash was repeated once more. After the final aspiration the plate was removed from the magnet and the beads were dried for 5 min. DNA was eluted from the dried beads with 15ul of nuclease-free water.

While this experiment was adapted for Illumina TruSeq, other adaptors may be used instead and may provide libraries suitable for any other Next Generation Sequencing (NGS) instrumentation or may alternatively amplified to be suitable for Sanger sequencing.

Alternatively, when amplicons from individual wells of the wellplate were not combined, quantification on Qubit^{™} after the two rounds of PCR and cleanup showed that approximately 25 ng to about 90 ng of amplification product was produced per well. This can be sufficient for carrying through sequencing as described above.

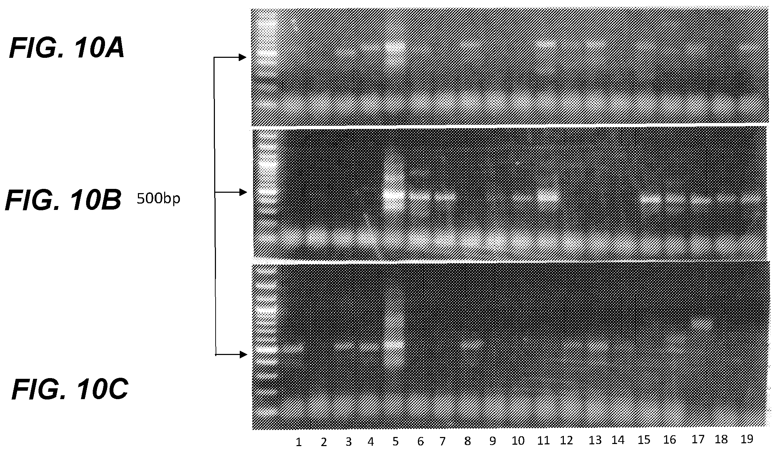

In Figures 10 A-C, the results of gel electrophoresis corroborating the ability to detect specific amplification products were shown. In this experiment, 19 single OKT3 cells were exported and the whole RNA amplification product produced as above was split into three portions. Each of the three portions was amplified individually for heavy chain Hc, light chain kappa Kc, and light chain lambda λc, using the primers and process described above. In Figure 10A, heavy chain was shown to be amplified from all 19 single cells (faint bands were confirmed by analysis with Qubit). In Figures 10B and 10C, light chain kappa (Figure 7B) or light chain lambda (Figure 7C) showed that each cell had either kappa or lambda, and not both. For example, Lanes 8, 12, 13, 14 have lambda light chain and not kappa, while lanes 9, 10, 11 had kappa light chain and not lambda. The arrows at the left edge of gels point to bands in the sizing ladder of 500 bp, which confirms the size of the expected products.

### LISTING OF SELECT EMBODIMENTS

1. A method of detecting a B cell lymphocyte expressing an antibody that specifically binds to an antigen of interest, the method comprising: introducing a sample comprising B cell lymphocytes into a microfluidic device, the microfluidic device comprising: an enclosure having a flow region and a sequestration pen, where the sequestration pen comprises an isolation region having a single opening and a connection region, the connection region providing a fluidic connection between the isolation region and the flow region, and where the isolation region of the holding pen is an unswept region of the micro-fluidic device; loading a B cell lymphocyte from the sample into the isolation region of the sequestration pen; introducing the antigen of interest into the flow region of the enclosure such that the antigen of interest is proximal to the B cell lymphocyte; and, monitoring binding of the antigen of interest to the antibody expressed by the B cell lymphocyte.
2. The method of embodiment 1, where the isolation region of the sequestration pen comprises at least one conditioned surface. In some embodiments, the at least one condition surface may include a plurality of conditioned surfaces.
3. The method of embodiment 2, where the conditioned surface is substantially non-reactive with B cell lymphocytes.
4. The method of embodiment 2 or 3, where the at least one conditioned surface (or each conditioned surface of the plurality) comprises a layer of covalently linked hydrophilic molecules.
5. The method of embodiment 4, where the hydrophilic molecules comprise polyethylene glycol (PEG)-containing polymers.
6. The method of any one of embodiments 1 to 5, where the enclosure of the microfluidic device further comprises a dielectrophoresis (DEP) configuration.
7. The method of any one of embodiments 1 to 6, where the enclosure of the microfluidic device further comprises a base, a microfluidic circuit structure, and a cover which together define a microfluidic circuit, and where the microfluidic circuit comprises the flow region and the sequestration pen.
8. The method of embodiment 7, where: the base comprises a first electrode; the cover comprises a second electrode; and the base or the cover comprises an electrode activation substrate, where the electrode activation substrate has a surface comprising a plurality of DEP electrode regions, and where the surface of the electrode activation substrate provides an inner surface of the flow region.
9. The method of any one of embodiments 1 to 8, where the connection region has a width W_{con} of about 20 microns to about 60 microns.
10. The method of any one of embodiments 1 to 9, where the connection region has a length L_{con}, and where a ratio of the length L_{con} of the connection region to the width W_{con} of the connection region has a value of at least 1.5.
11. The method of embodiment 9 or 10, where the isolation region has a width Wiso that is greater than the width W_{con} of the connection region.
12. The method of any one of embodiments 9 to 11, where the isolation region has a width Wiso that is about 50 microns to about 250 microns.
13. The method of any one of embodiments 1 to 12, where the sequestration pen comprises a volume of about 0.5 nL to about 2.5 nL.
14. The method of any one of embodiments 1 to 13, where the isolation region of the sequestration pen comprises at least one surface (e.g., a plurality of surfaces) coated with a coating material.
15. The method of embodiment 14, where the coating material comprises hydrophilic molecules that are substantially non-reactive with B cell lymphocytes.
16. The method of embodiment 14 or 15, where the coating material comprises polyethylene glycol (PEG)-containing polymers (e.g., in some embodiments, the PEG containing polymers comprise PEG-PPG block co-polymers).
17. The method of any one of embodiments 1 to 16, where the sample comprising B cell lymphocytes is a sample of peripheral blood, a spleen biopsy, a bone marrow biopsy, a lymph node biopsy, or a tumor biopsy.
18. The method of any one of embodiments 1 to 16, where the sample comprising B cell lymphocytes is a sample of peripheral blood.
19. The method of any one of embodiments 1 to 16, where the sample comprising B cell lymphocytes is a bone marrow biopsy.
20. The method of embodiment 17 or 18, where the B cell lymphocyte is a memory B cell.
21. The method of embodiment 17 or 19, where the B cell lymphocyte is a plasma B cell.
22. The method of any one of embodiments 1 to 21, where the sample comprising B cell lymphocytes is obtained from a mammal or avian animal.
23. The method of embodiment 22, where the sample comprising B cell lymphocytes is obtained from a human, mouse, rat, guinea pig, gerbil, hamster, rabbit, goat, sheep, llama, chicken, ferret, pig, horse, cow or turkey.
24. The method of embodiment 22 or 23, where the mammal has been immunized against the antigen of interest.
25. The method of embodiment 22 or 23, where the mammal has been exposed to or immunized against a pathogen associated with the antigen of interest.
26. The method of embodiment 22 or 23, where the mammal has cancer and the cancer is associate with the antigen of interest.
27. The method of embodiment 22 or 23, where the mammal has an auto-immune disease and the auto-immune disease is associated with the antigen of interest.
28. The method of any one of embodiments 1 to 27, where the sample comprising B cell lymphocytes has been depleted of cell types other than B cell lymphocytes.
29. The method of any one of embodiments 1 to 28, where the sample comprising B cell lymphocytes has been depleted of B cell lymphocytes expressing IgM antibodies, IgA antibodies, IgD antibodies, or any combination thereof.
30. The method of any one of embodiments 1 to 19 and 21 to 29, where the sample comprising B cell lymphocytes has been enriched for B cell lymphocytes expressing CD27.
31. The method of any one of embodiments 1 to 20 and 22 to 29, where the sample comprising B cell lymphocytes has been enriched for B cell lymphocytes expressing CD138.
32. The method of any one of embodiments 1 to 31, where the sample comprising B cell lymphocytes has been contacted with DNase prior to being introduced into the microfluidic device.
33. The method of any one of embodiments 1 to 32, where a single B cell lymphocyte is loaded into the isolation region.
34. The method of any one of embodiments 1 to 32, where a plurality of B cell lymphocytes is loaded into the isolation region.
35. The method of any one of embodiments 1 to 34, further comprising: contacting the B cell lymphocyte with a stimulating agent that stimulates B cell activation.
36. The method of embodiment 35, where the stimulating agent comprises a CD40 agonist.
37. The method of embodiment 36, where the CD40 agonist comprises CD40L, a derivative thereof, or an anti-CD40 antibody and, optionally, where the CD40 agonist is linked to a micro-object (e.g., a bead).
38. The method of embodiment 35, where the stimulating agent comprises one or more CD40L⁺ feeder cells (e.g., irradiated T cells) or a derivative thereof.
39. The method of any one of embodiments 35 to 38, where the stimulating agent further comprises a B cell receptor (BCR)-ligating molecule.
40. The method of embodiment 39, where the BCR-ligating molecule comprises Protein A or Protein G.
41. The method of embodiment 39 or 40, where the BCR-ligating molecule is linked to a micro-object (e.g., a bead).
42. The method of embodiment 35, where contacting the B cell lymphocytes with a stimulating agent comprises contacting the B cell lymphocytes with a mixture of CD40L⁺ feeder cells and Protein A conjugated to beads (e.g., at a ratio of about 1:1 to about 1:10).
43. The method of embodiment 42, where contacting the B cell lymphocytes with a stimulating agent comprises loading the mixture into the isolation region of the sequestration pen (e.g., using gravity or DEP force).
44. The method of any one of embodiments 35 to 43, where the stimulating agent further comprises a toll-like receptor (TLR) agonist.
45. The method of embodiment 44, where the TLR agonist is a CpG oligonucleotide.
46. The method of any one of embodiments 35 to 45, where the B cell lymphocyte is contacted with the stimulating agent for a period of one to ten days (in some embodiments, the period of contact may be 3 to 5 days).
47. The method of embodiment 46, where the B cell lymphocyte is contacted with the stimulating agent substantially continuously for the period of one to ten days (in some embodiments, the period of contact may be 3 to 5 days).
48. The method of any one of embodiments 35 to 47, further comprising: providing culture medium to the B cell lymphocyte, where the culture medium comprises one or more agents that promote B cell expansion and/or activation.
49. The method of embodiment 48, where the culture medium at least one agent selected from the group consisting of IL-2, IL-4, IL-6, IL-10, IL-21, BAFF and April.
50. The method of embodiment 48 or 49, where the culture medium comprises a TLR agonist.
51. The method of any one of embodiments 48 to 50, where the B cell lymphocyte is provided culture medium for a period of one to ten days (in some embodiments, the period of contact may be 3 to 5 days).
52. The method of any one of embodiments 48 to 50, where the contacting with a stimulating agent and the providing of culture medium are preformed over a substantially coextensive period of time.
53. The method of any one of embodiments 35 to 52, where contacting the B cell lymphocyte with the stimulating agent is performed prior to introducing the B cell lymphocyte into the microfluidic device.
54. The method of any one of embodiments 3f to 53, where contacting the B cell lymphocyte with the stimulating agent is performed after introducing the B cell lymphocyte into the microfluidic device (e.g., after loading the B cell lymphocyte into the isolation region of the sequestration pen).
55. The method of any one of embodiments 35 to 54, where contacting the B cell lymphocyte with the stimulating agent is performed during the monitoring step.
56. The method of embodiment 6, where loading the B cell lymphocyte into the isolation region of the sequestration pen comprises using DEP force to move the B cell lymphocyte into the isolation region.
57. The method of embodiment 56, where the B cell lymphocyte is moved from the flow region to the isolation region.
58. The method of any one of embodiments 1 to 57, where providing the antigen of interest comprises flowing a solution comprising soluble antigen of interest into or through the flow region.
59. The method of embodiment 58, where the antigen of interest is covalently bound to a first detectable label (e.g., a fluorescent label).
60. The method of embodiment 58 or 59 further comprising providing a micro-object comprising a first antibody-binding agent, where the first antibody-binding agent binds to the antibody expressed by the B cell lymphocyte without inhibiting the binding of antigen of interest to the antibody expressed by the B cell lymphocyte, and where monitoring of binding of the antigen of interest to the antibody expressed by the B cell lymphocyte comprises detecting indirect binding of the antigen of interest to the micro-object.
61. The method of embodiment 60, where the first antibody-binding agent binds to an Fc domain of the antibody expressed by the B cell lymphocyte.
62. The method of embodiment 60 or 61, where the micro-object is a bead.
63. The method of any one of embodiments 60 to 62, where providing the micro-object comprises flowing a solution comprising the micro-object into the flow region and stopping the flow when the micro-object is located proximal to the sequestration pen.
64. The method of embodiment 63, where providing the micro-object further comprises loading the micro-object into the sequestration pen.
65. The method of embodiment 63 or 64, where the solution comprising the micro-object and the solution comprising the soluble antigen of interest are the same solution.
66. The method of embodiment 63, where the solution comprising the micro-object and the solution comprising the soluble antigen or interest are different solutions, and where providing the micro-object occurs before providing the antigen of interest.
67. The method of any one of embodiments 60 to 65 further comprising: providing a second antibody-binding agent, where the second antibody-binding agent comprises a second detectable label (e.g., a fluorescent label); and monitoring indirect binding of the second antibody-binding agent to the micro-object.
68. The method of embodiment 67, where the second antibody-binding agent binds (which may optionally specifically bind) to IgG antibodies (e.g., an anti-IgG secondary antibody).
69. The method of embodiment 67 or 68, where the first detectable label is different from the second detectable label (and can be differentially detected).
70. The method of any one of embodiments 67 to 69, where providing the second antibody-binding agent comprises flowing a solution comprising soluble second antibody-binding agent into or through the flow region.
71. The method of embodiment 70, where the solution comprising the soluble second antibody-binding agent and the solution comprising the soluble antigen of interest are the same solution.
72. The method of embodiment 70, where the solution comprising the soluble second antibody-binding agent and the solution comprising the soluble antigen or interest are different solutions (e.g., which are provided sequentially).
73. The method of any one of embodiments 1 to 57, where providing the antigen of interest comprises providing a micro-object that comprises the antigen of interest, where the micro-object is a cell, a liposome, a lipid nanoraft, or a bead.
74. The method of embodiment 73 further comprising: providing a labeled antibody-binding agent prior to or concurrently with the antigen of interest, where the monitoring of binding of the antigen of interest to the antibody expressed by the B cell lymphocyte comprises detecting indirect binding of the labeled antibody-binding agent to the antigen of interest.
75. The method of embodiment 74, where the labeled antibody-binding agent binds (which may optionally specifically bind) to anti-IgG antibodies (e.g., is an anti-IgG secondary antibody).
76. The method of embodiment 74 or 75, where the labeled antibody-binding agent is covalently bound to a fluorescent label.
77. The method of any one of embodiments 74 to 76, where the labeled antibody-binding agent is provided in a mixture with the antigen of interest.
78. The method of any one of embodiments 74 to 76, where the labeled antibody-binding agent is provided after providing the antigen of interest.
79. The method of any one of embodiments 1 to 78, where monitoring binding of the antigen of interest to the antibody expressed by the B cell lymphocyte comprises imaging all or part of the sequestration pen of the microfluidic device.
80. The method of embodiment 79, where the imaging comprises fluorescence imaging.
81. The method of embodiment 79 or 80, where the imaging comprises taking a plurality of images.
82. The method of embodiment 81, where the plurality of images are taken at fixed time intervals.
83. The method of any of embodiments 1 to 82, where the microfluidic device comprises a plurality of the sequestration pens, each having an isolation region and a connection region, each the connection region providing a fluidic connection between the isolation region and the flow region, the method further comprising: loading one or more of the plurality of B cell lymphocytes into the isolation region of each of two or more sequestration pens of the plurality; introducing the antigen of interest into the microfluidic device such that the antigen of interest is proximal to each of the two or more sequestration pens loaded with one or more B cell lymphocytes; and monitoring of binding of the antigen of interest to the antibody expressed by each of the loaded B cell lymphocytes.
84. The method of embodiment 83, where a single B cell lymphocyte is loaded into the isolation region of each of the two or more sequestration pens of the plurality.
85. The method of any one of embodiments 1 to 84, further comprising: detecting binding of the antigen of interest to the antibody expressed by the loaded B cell lymphocyte, or ones of the loaded B cell lymphocytes; identifying the loaded B cell lymphocyte, or the ones of the loaded B cell lymphocytes, as expressing an antibody that specifically binds to the antigen of interest.
86. A method of characterizing an antibody that specifically binds to an antigen of interest, the method comprising: identifying a B cell lymphocyte, or a clonal population thereof, that expresses an antibody that specifically binds to the antigen of interest, where the identifying is performed according to the method of embodiment 85; isolating from the B cell lymphocyte, or the clonal population thereof, a nucleic acid encoding an immunoglobulin heavy chain variable region (V_{H}) and/or an immunoglobulin light chain variable region (V_{L}); and sequencing at least a portion of the nucleic acid encoding the immunoglobulin heavy chain variable region (V_{H}) and/or at least a portion of the nucleic acid encoding the immunoglobulin light chain variable region (V_{L}).
87. The method of embodiment 86, where sequencing the immunoglobulin heavy chain variable region (V_{H}) comprises: lysing the identified B cell lymphocyte, or B cell lymphocyte(s) of the clonal population thereof; reverse transcribing mRNA isolated from the B cell lymphocyte, or the B cell lymphocyte(s) of the clonal population thereof, where the mRNA encodes the immunoglobulin heavy chain variable region (V_{H}), thereby forming V_{H} cDNA; and sequencing at least a portion of the V_{H} cDNA.
88. The method of embodiment 86 or 87, where sequencing the immunoglobulin light chain variable region (V_{L}) comprises: lysing the identified B cell lymphocyte, or B cell lymphocyte(s) of the clonal population thereof; reverse transcribing mRNA isolated from the B cell lymphocyte, or the clonal population thereof, where the mRNA encodes the immunoglobulin light chain variable region (V_{L}), thereby forming V_{L} cDNA; and sequencing at least a portion of the V_{L} cDNA.
89. The method of embodiment 87 or 88, where reverse transcribing the mRNA comprises contacting the mRNA with a capture/priming oligonucleotide.
90. The method of embodiment 89, where the reverse transcribing is performed in the presence of a transcript switching oligonucleotide.
91. The method of any one of embodiments 87 to 90, where the identified B cell lymphocyte, or the B cell lymphocyte(s) of the clonal population thereof, is(are) exported from the microfluidic device prior to being lysed.
92. The method of embodiment 91, where exporting the identified B cell lymphocyte, or the clonal population thereof, comprises: moving the identified B cell lymphocyte, or the B cell lymphocyte(s) of the clonal population thereof, from the isolation region of the sequestration pen into the flow region of the microfluidic device; and flowing the identified B cell lymphocyte, or the B cell lymphocyte(s) of the clonal population thereof, through the flow region and out of the microfluidic device.
93. The method of embodiment 92, where moving the identified B cell lymphocyte, or the B cell lymphocyte(s) of the clonal population thereof, from the isolation region of the sequestration pen comprises capturing and moving the identified B cell lymphocyte, or the clonal population thereof, using DEP force.
94. The method of any one of embodiments 91 to 93, where the identified B cell lymphocyte is exported as a single cell.
95. The method of any one of embodiments 91 to 93, where the identified B cell lymphocyte(s) of the clonal population thereof are exported as a group.
96. The method of embodiment 87 or 88, where the identified B cell lymphocyte, or the B cell lymphocyte(s) of the clonal population thereof, is(are) lysed within the microfluidic device.
97. The method of embodiment 96, further comprising: providing one or more capture beads in close proximity to the identified B cell lymphocyte, or the B cell lymphocyte(s) of the clonal population thereof, where the one or more capture beads each comprises oligonucleotides capable of binding the V_{H} mRNA and/or the V_{L} mRNA; lysing the identified B cell lymphocyte, or the clonal population thereof; and allowing the V_{H} mRNA and/or the V_{L} mRNA from the lysed B cell lymphocyte, or from the lysed B cell lymphocyte(s) of the clonal population thereof, to be bound by the one or more capture beads.
98. The method of embodiment 94, where each capture bead of the one or more capture beads comprises a plurality of capture/priming oligonucleotides.
99. The method of embodiment 97 or 98, where the one or more capture beads is provided prior to lysing the identified B cell lymphocyte, or the B cell lymphocyte(s) of the clonal population thereof.
100. The method of any one of embodiments 97 to 99, where each of the one or more capture beads is loaded into the sequestration pen containing the identified B cell lymphocyte or the B cell lymphocyte(s) of the clonal population thereof.
101. The method of any one of embodiments 97 to 100, further comprising: moving the one or more capture beads to a substantially RNA-free region of the microfluidic device.
102. The method of embodiment 101, where, prior to moving the one or more capture beads to the substantially RNA-free region of the microfluidic device, the substantially RNA-free region did not include any B cell lymphocytes.
103. The method of embodiment 101 or 102, where the substantially RNA-free region is within a sequestration pen different from the sequestration pen in which the identified B cell lymphocyte was loaded.
104. The method of any one of embodiments 97 to 103, where the bound V_{H} mRNA and/or the bound V_{L} mRNA is reverse transcribed into V_{H} cDNA and/or V_{L} cDNA while bound to the one or more capture beads.
105. The method of embodiment 104, where the bound V_{H} mRNA and/or the bound V_{L} mRNA is reverse transcribed into V_{H} cDNA and/or V_{L} cDNA while the one or more capture beads is contained within the microfluidic device (e.g., within the sequestration pen).
106. The method of embodiment 104 or 105, where the bound V_{H} and/or the bound V_{L} mRNA is reverse transcribed into V_{H} cDNA and/or V_{L} cDNA by flowing reverse transcriptase, nucleotides, and an appropriate buffer into or through the flow region of the microfluidic device.
107. The method of any one of embodiments 104 to 106, where the V_{H} cDNA and/or V_{L} cDNA is exported from the microfluidic device while bound to the one or more capture beads.
108. The method of any one of embodiments 97 to 104, further comprising: exporting the one or more capture beads from the microfluidic device prior to reverse transcribing the V_{H} mRNA and/or the V_{L} mRNA into V_{H} cDNA and/or V_{L} cDNA.
109. The method of embodiment 87 or 88, further comprising amplifying the V_{H} cDNA and/or the V_{L} cDNA prior to the sequencing.
110. The method of embodiment 109, where the amplifying comprises PCR amplification.
111. The method of embodiment 109 or 110, where the amplifying comprises increasing the representation of V_{H} cDNA and/or V_{L} cDNA, or fragments thereof, in the reverse transcribed mRNA isolated from the B cell lymphocyte.
112. The method of embodiment 111, where the amplifying comprises: a first round of amplification which increases the representation of V_{H} cDNA and/or V_{L} cDNA, or fragments thereof, in the reverse transcribed mRNA isolated from the B cell lymphocyte; and a second round of amplification which introduces barcode sequences into the V_{H} cDNA and/or V_{L} cDNA, or fragments thereof, amplified in the first round.
113. The method of embodiment 87 or 88, where the V_{H} cDNA and/or the V_{L} cDNA is sequenced without prior PCR amplification.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation, and use may be made without departing from the principles and concepts set forth herein. Also, as used herein, the examples and embodiments, in all respects, are meant to be illustrative only and should not be construed to be limiting in any manner. It should also be noted, that while the term step is used herein, that term may be used to simply draw attention to different portions of the described methods and is not meant to delineate a starting point or a stopping point for any portion of the methods, or to be limiting in any other way.

**Informal SEQ ID NO. Listing**

| SEQ ID NO. | Sequence | |
|---|---|---|
| 1 | AAGCAGTGGTATCAACGCAGAGT | Artificial Sequence |
| 2 | biot- AAGCAGTGGTATCAACGCAGAGTACTTTTTTTTTTTTTTTTTTTTTTTTT TTTTTVI | Artificial Sequence |
| 3 | biot-GTGGTATCAACGCAGAGTACACGACGCTCTTCCGATCTNNNNrGrGrG | Artificial Sequence |
| 4 | biot-AAGCAGTGGTATCAACGCAGAGT | Artificial Sequence |
| 5 | GACCACCGAGATCTACACNNNNNNACACTCTTTCCCTACACGACGCTCT TCCGATCT | Artificial Sequence |
| 6 | ACTGGAGTTCAGACGTGTGCTCTTCCGATCTCTGGACAGGGATCCAGAG TTCCA | Artificial Sequence |
| 7 | ACTGGAGTTCAGACGTGTGCTCTTCCGATCTTCGTTCACTGCCATCAATCT TCCA | Artificial Sequence |
| 8 | ACTGGAGTTCAGACGTGTGCTCTTCCGATCTAGGKGTACCATYTRCCTTC CA | Artificial Sequence |
| 9 | AATGATACGGCGACCACCGAGATCTACAC | Artificial Sequence |
| 10 | CAAGCAGAAGACGGCATACGAGATNNNNNNGTGACTGGAGTTCAGAC GTGT | Artificial Sequence |

### Clauses:

1. A method of detecting a B cell lymphocyte expressing an antibody that specifically binds to an antigen of interest, the method comprising:
   introducing a sample comprising B cell lymphocytes into a microfluidic device, the microfluidic device comprising:
      an enclosure having a flow region and a sequestration pen,
      wherein said sequestration pen comprises an isolation region having a single opening and a connection region, said connection region providing a fluidic connection between said isolation region and said flow region, and wherein said isolation region of said holding pen is an unswept region of said micro-fluidic device;
   loading a B cell lymphocyte from said sample into said isolation region of said sequestration pen;
   introducing said antigen of interest into said flow region of said enclosure such that said antigen of interest is proximal to said B cell lymphocyte; and
   monitoring binding of said antigen of interest to said antibody expressed by said B cell lymphocyte,
   wherein said isolation region of said sequestration pen comprises at least one conditioned surface.
2. The method of clause 1, wherein said at least one conditioned surface comprises a layer of covalently linked hydrophilic molecules.
3. The method of clause 2, wherein said hydrophilic molecules comprise polyethylene glycol (PEG)-containing polymers.
4. The method of any one of clauses 1 to 3, wherein said enclosure of said microfluidic device further comprises a dielectrophoresis (DEP) configuration.
5. The method of clause 4, wherein said enclosure of said microfluidic device further comprises a base, a microfluidic circuit structure, and a cover which together define a microfluidic circuit, and wherein said microfluidic circuit comprises said flow region and said sequestration pen.
6. The method of clause 5, wherein:
   said base comprises a first electrode;
   said cover comprises a second electrode; and
   said base or said cover comprises an electrode activation substrate,
   wherein said electrode activation substrate has a surface comprising a plurality of DEP electrode regions, and wherein said surface of said electrode activation substrate provides an inner surface of said flow region.
7. The method of clause 1, wherein said connection region has a width W_{con} of about 20 microns to about 60 microns.
8. The method of clause 1, wherein the connection region has a length L_{con}, and wherein a ratio of said length L_{con} of said connection region to said width W_{con} of said connection region has a value of at least 1.5.
9. The method of clause 7 or 8, wherein said isolation region has a width Wiso that is greater than the width W_{con} of said connection region.
10. The method of clause 9, wherein said isolation region has a width Wiso that is about 50 microns to about 250 microns.
11. The method of clause 1, wherein said sequestration pen comprises a volume of about 0.5 nL to about 2.5 nL.
12. The method of clause 1, wherein said isolation region of said sequestration pen comprises at least one surface coated with a coating material.
13. The method of clause 12, wherein said coating material comprises polyethylene glycol (PEG)-containing polymers.
14. The method of clause 1, wherein said sample comprising B cell lymphocytes is a sample of peripheral blood, a spleen biopsy, a bone marrow biopsy, a lymph node biopsy, or a tumor biopsy.
15. The method of clause 14, wherein said sample comprising B cell lymphocytes is a sample of peripheral blood.
16. The method of clause 14, wherein said sample comprising B cell lymphocytes is a bone marrow biopsy.
17. The method of clause 14 or 15, wherein said B cell lymphocyte is a memory B cell.
18. The method of clause 14 or 16, wherein said B cell lymphocyte is a plasma B cell.
19. The method of clause 1, wherein said sample comprising B cell lymphocytes is obtained from a mammal or avian animal.
20. The method of clause 19, wherein said sample comprising B cell lymphocytes is obtained from a human, mouse, rat, guinea pig, gerbil, hamster, rabbit, goat, sheep, llama, or chicken.
21. The method of clause 19 or 20, wherein said mammal has been immunized against said antigen of interest, wherein said mammal has been exposed to or immunized against a pathogen associated with said antigen of interest, wherein said mammal has cancer and said cancer is associate with said antigen of interest, or wherein said mammal has an auto-immune disease and said auto-immune disease is associated with said antigen of interest.
22. The method of clause 1, wherein said sample comprising B cell lymphocytes has been depleted of cell types other than B cell lymphocytes.
23. The method of clause 1 or 22, wherein said sample comprising B cell lymphocytes has been enriched for B cell lymphocytes expressing CD27.
24. The method of clause 1 or 22, wherein said sample comprising B cell lymphocytes has been enriched for B cell lymphocytes expressing CD138.
25. The method of clause 1, wherein said sample comprising B cell lymphocytes has been contacted with DNase prior to being introduced into said microfluidic device.
26. The method of clause 1, wherein a single B cell lymphocyte is loaded into said isolation region.
27. The method of clause 1, wherein a plurality of B cell lymphocytes are loaded into said isolation region.
28. The method of clause 1, further comprising:
   contacting said B cell lymphocyte with a stimulating agent that stimulates B cell activation.
29. The method of clause 28, wherein said stimulating agent comprises a CD40 agonist.
30. The method of clause 29, wherein said stimulating agent comprises one or more CD40L⁺ feeder cells.
31. The method of clause 29 or 30, wherein said stimulating agent further comprises a B cell receptor (BCR)-ligating molecule, wherein said BCR-ligating molecule comprises Protein A or Protein G.
32. The method of clause 31, wherein said BCR-ligating molecule is linked to a micro-object.
33. The method of clause 28, wherein contacting said B cell lymphocytes with a stimulating agent comprises contacting said B cell lymphocytes with a mixture of CD40L⁺ feeder cells and Protein A conjugated to beads, wherein contacting said B cell lymphocytes with a stimulating agent comprises loading said mixture into said isolation region of said sequestration pen.
34. The method of clause 29, wherein said B cell lymphocyte is contacted substantially continuously with said stimulating agent for a period of 3 to 5 days.
35. The method of clause 29, further comprising:
   providing culture medium to said B cell lymphocyte, wherein said culture medium comprises one or more agents that promote B cell expansion and/or activation.
36. The method of clause 35, wherein said culture medium comprises IL-2, IL-4, IL-6, IL-21, and BAFF or April.
37. The method of clause 35, wherein said culture medium comprises a TLR agonist.
38. The method of clause 37, wherein said TLR agonist is CpG oligonucleotides.
39. The method of clause 35, wherein said B cell lymphocyte is provided culture medium for a period of one to 3 to 5 days.
40. The method of clause 35, wherein said contacting with a stimulating agent and said providing of culture medium are preformed over a substantially coextensive period of time.
41. The method of clause 4, wherein loading said B cell lymphocyte into said isolation region of said sequestration pen comprises using DEP force to move said B cell lymphocyte into said isolation region.
42. The method of clause 41, wherein said B cell lymphocyte is moved from said flow region to said isolation region.
43. The method of clause 1, wherein providing said antigen of interest comprises flowing a solution comprising soluble antigen of interest into or through said flow region.
44. The method of clause 43, wherein said antigen of interest is covalently bound to a first detectable label.
45. The method of clause 43 or 44 further comprising providing a micro-object comprising a first antibody-binding agent, wherein said first antibody-binding agent binds to said antibody expressed by said B cell lymphocyte without inhibiting the binding of antigen of interest to said antibody expressed by said B cell lymphocyte, and wherein monitoring of binding of said antigen of interest to said antibody expressed by said B cell lymphocyte comprises detecting indirect binding of said antigen of interest to said micro-object.
46. The method of clause 45, wherein said first antibody-binding agent binds to an Fc domain of said antibody expressed by said B cell lymphocyte.
47. The method of clause 45, wherein providing said micro-object comprises flowing a solution comprising said micro-object into said flow region and stopping said flow when said micro-object is located proximal to said sequestration pen.
48. The method of clause 45, wherein said solution comprising said micro-object and said solution comprising said soluble antigen of interest are the same solution.
49. The method of clause 45 further comprising:
   providing a second antibody-binding agent, wherein said second antibody-binding agent comprises a second detectable label; and
   monitoring indirect binding of said second antibody-binding agent to said micro-object,
   wherein said first detectable label is different from said second detectable label.
50. The method of clause 49, wherein said second antibody-binding agent binds to IgG antibodies.
51. The method of clause 1, wherein providing said antigen of interest comprises providing a micro-object that comprises said antigen of interest, wherein said micro-object is a cell, a liposome, a lipid nanoraft, or a bead.
52. The method of clause 51 further comprising:
   providing a labeled antibody-binding agent prior to or concurrently with said antigen of interest,
   wherein said monitoring of binding of said antigen of interest to said antibody expressed by said B cell lymphocyte comprises detecting indirect binding of said labeled antibody-binding agent to said antigen of interest.
53. The method of clause 52, wherein said labeled antibody-binding agent binds to anti-IgG antibodies.
54. The method of clause 1, wherein monitoring binding of said antigen of interest to said antibody expressed by said B cell lymphocyte comprises imaging all or part of said sequestration pen of said microfluidic device.
55. The method of clause 54, wherein said imaging comprises fluorescence imaging.
56. The method of clause 54, wherein said imaging comprises taking a plurality of images.
57. The method of clause 1, wherein said microfluidic device comprises a plurality of said sequestration pens, each having an isolation region and a connection region, each said connection region providing a fluidic connection between said isolation region and said flow region, said method further comprising:
   loading one or more of said plurality of B cell lymphocytes into said isolation region of each of two or more sequestration pens of said plurality;
   introducing said antigen of interest into said microfluidic device such that said antigen of interest is proximal to each of said two or more sequestration pens loaded with one or more B cell lymphocytes; and
   monitoring of binding of said antigen of interest to said antibody expressed by each of said loaded B cell lymphocytes.
58. The method of clause 57, wherein a single B cell lymphocyte is loaded into said isolation region of each of said two or more sequestration pens of said plurality.
59. The method of clause 57, further comprising:
   detecting binding of said antigen of interest to said antibody expressed by said loaded B cell lymphocyte, or ones of said loaded B cell lymphocytes;
   identifying said loaded B cell lymphocyte, or said ones of said loaded B cell lymphocytes, as expressing an antibody that specifically binds to said antigen of interest.
60. A method of characterizing an antibody that specifically binds to an antigen of interest, the method comprising:
   identifying a B cell lymphocyte, or a clonal population thereof, that expresses an antibody that specifically binds to said antigen of interest, wherein said identifying is performed according to the method of claim 59;
   isolating from said B cell lymphocyte, or said clonal population thereof, a nucleic acid encoding an immunoglobulin heavy chain variable region (V_{H}) and/or an immunoglobulin light chain variable region (V_{L}); and
   sequencing at least a portion of said nucleic acid encoding said immunoglobulin heavy chain variable region (V_{H}) and/or at least a portion of said nucleic acid encoding said immunoglobulin light chain variable region (V_{L}).
61. The method of clause 60, wherein sequencing said immunoglobulin heavy chain variable region (V_{H}) comprises:
   lysing said identified B cell lymphocyte, or B cell lymphocyte(s) of said clonal population thereof;
   reverse transcribing mRNA isolated from said B cell lymphocyte, or said B cell lymphocyte(s) of said clonal population thereof, wherein said mRNA encodes said immunoglobulin heavy chain variable region (V_{H}), thereby forming V_{H} cDNA; and
   sequencing at least a portion of said V_{H} cDNA.
62. The method of clause 60, wherein sequencing said immunoglobulin light chain variable region (V_{L}) comprises:
   lysing said identified B cell lymphocyte, or B cell lymphocyte(s) of said clonal population thereof;
   reverse transcribing mRNA isolated from said B cell lymphocyte, or said clonal population thereof, wherein said mRNA encodes said immunoglobulin light chain variable region (V_{L}), thereby forming V_{L} cDNA; and
   sequencing at least a portion of said V_{L} cDNA.
63. The method of clause 61 or 62, wherein reverse transcribing said mRNA comprises contacting said mRNA with a capture/priming oligonucleotide.
64. The method of clause 63, wherein said reverse transcribing is performed in the presence of a transcript switching oligonucleotide.
65. The method of clause 61 or 62, wherein said identified B cell lymphocyte, or said B cell lymphocyte(s) of said clonal population thereof, is(are) exported from said microfluidic device prior to being lysed.
66. The method of clause 65, wherein exporting said identified B cell lymphocyte, or said clonal population thereof, comprises:
   moving said identified B cell lymphocyte, or said B cell lymphocyte(s) of said clonal population thereof, from said isolation region of said sequestration pen into said flow region of said microfluidic device; and
   flowing said identified B cell lymphocyte, or said B cell lymphocyte(s) of said clonal population thereof, through said flow region and out of said microfluidic device.
67. The method of clause 66, wherein moving said identified B cell lymphocyte, or said B cell lymphocyte(s) of said clonal population thereof, from said isolation region of said sequestration pen comprises capturing and moving said identified B cell lymphocyte, or said clonal population thereof, using DEP force.
68. The method of clause 61 or 62, wherein said identified B cell lymphocyte, or said B cell lymphocyte(s) of said clonal population thereof, is(are) lysed within said microfluidic device.
69. The method of clause 68, further comprising:
   providing one or more capture beads in close proximity to said identified B cell lymphocyte, or said B cell lymphocyte(s) of said clonal population thereof, where said one or more capture beads each comprises oliogonucleotides capable of binding said V_{H} mRNA and/or said V_{L} mRNA;
   lysing said identified B cell lymphocyte, or said clonal population thereof; and
   allowing said V_{H} mRNA and/or said V_{L} mRNA from said lysed B cell lymphocyte, or from said lysed B cell lymphocyte(s) of said clonal population thereof, to be bound by said one or more capture beads.
70. The method of clause 69, wherein each capture bead of said one or more capture beads comprises a plurality of capture/priming oligonucleotides.
71. The method of clause 69, wherein said bound V_{H} mRNA and/or said bound V_{L} mRNA is reverse transcribed into V_{H} cDNA and/or V_{L} cDNA while bound to said one or more capture beads.
72. The method of clause 71, wherein said V_{H} cDNA and/or V_{L} cDNA is exported from said microfluidic device while bound to said one or more capture beads.
73. The method of clause 61 or 62, further comprising amplifying said V_{H} cDNA and/or said V_{L} cDNA prior to said sequencing.
74. The method of clause 73, wherein said amplifying comprises increasing the representation of V_{H} cDNA and/or V_{L} cDNA, or fragments thereof, in the reverse transcribed mRNA isolated from said B cell lymphocyte.
75. The method of clause 74, wherein said amplifying comprises:
   a first round of amplification which increases the representation of V_{H} cDNA and/or V_{L} cDNA, or fragments thereof, in the reverse transcribed mRNA isolated from said B cell lymphocyte; and
   a second round of amplification which introduces barcode sequences into the V_{H} cDNA and/or V_{L} cDNA, or fragments thereof, amplified in the first round.

## Claims

1. A method of characterizing an antibody capable of binding specifically to an antigen of interest for preparation of a cDNA library, the method comprising:
identifying a B cell lymphocyte expressing an antibody that specifically binds to the antigen of interest, wherein identifying is performed by:
introducing a sample comprising B cell lymphocytes into a microfluidic device, the microfluidic device comprising: an enclosure having a flow region and a sequestration pen, wherein the sequestration pen comprises an isolation region having a single opening and a connection region, the connection region providing a fluidic connection between the isolation region and the flow region, and wherein the isolation region of the holding pen is an unswept region of the micro-fluidic device;
loading a B cell lymphocyte from the sample into the isolation region of the sequestration pen;
introducing the antigen of interest into the flow region of the enclosure to a location proximal to the B cell lymphocyte;
monitoring binding of the antigen of interest to the antibody expressed by the B cell lymphocyte; wherein monitoring binding of the antigen of interest to the antibody comprises detecting binding of the antigen of interest to the antibody expressed by the loaded B cell lymphocyte; and
identifying the loaded B cell lymphocyte as expressing the antibody capable of binding specifically to the antigen of interest;
isolating from the B cell lymphocyte a nucleic acid encoding an immunoglobulin heavy chain variable region (V_{H}) and/or an immunoglobulin light chain variable region (V_{L}); and
sequencing at least a portion of the nucleic acid encoding the immunoglobulin heavy chain variable region (V_{H}) and/or at least a portion of the nucleic acid encoding the immunoglobulin light chain variable region (V_{L}).

2. The method of claim 1, wherein the isolation region of the sequestration pen comprises at least one conditioned surface.

3. The method of claim 2, wherein the at least one conditioned surface comprises a layer of covalently linked hydrophilic molecules; and optionally wherein the hydrophilic molecules comprise polyethylene glycol (PEG)-containing polymers.

4. The method of any one of claims 1 to 3, wherein the sample comprising B cell lymphocytes is a sample of peripheral blood, a spleen biopsy, a bone marrow biopsy, a lymph node biopsy, or a tumor biopsy; and optionally wherein the B cell lymphocyte is a plasma B cell.

5. The method of any one of claims 1 to 4, wherein identifying a B cell lymphocyte that expresses an antibody that specifically binds to the antigen of interest further comprises:
contacting the B cell lymphocyte with a stimulating agent; and optionally wherein the stimulating agent comprises a CD40 agonist.

6. The method of claim 5, wherein identifying a B cell lymphocyte that expresses an antibody that specifically binds to the antigen of interest further comprises: providing culture medium to the B cell lymphocyte, wherein the culture medium comprises one or more growth-inducing agents; and optionally wherein the one or more growth-inducing agents comprises at least one agent selected from the group consisting of CpG oligonucleotide, IL-2, IL-4, IL-6, IL-10, IL-21, BAFF and April.

7. The method of any one of claims 1 to 6; wherein providing the antigen of interest comprises flowing a solution comprising soluble antigen of interest into or through the flow region; and optionally wherein the antigen of interest is covalently bound to a first detectable label.

8. The method of claim 7, wherein identifying a B cell lymphocyte that expresses the antibody capable of binding specifically to the antigen of interest further comprises: providing a micro-object comprising a first antibody-binding agent, wherein the first antibody-biding agent binds to the expressed antibody without inhibiting binding of the antigen of interest to the expressed antibody, and further wherein monitoring binding of the antigen of interest to the expressed antibody comprises detecting indirect binding of the antibody of interest to the micro-obj ect.

9. The method of claim 8, wherein identifying a B cell lymphocyte that expresses the antibody that specifically binds to the antigen of interest further comprises:
providing a second antibody-binding agent, wherein the second antibody-binding agent comprises a second detectable label; and
monitoring indirect binding of the second antibody-binding agent to the micro-object.

10. The method of any one of embodiments 1 to 7, wherein providing the antigen of interest comprises providing a micro-object comprising the antigen of interest, wherein the micro-object is a cell, a liposome, a lipid nanoraft, or a bead; and optionally wherein identifying the B cell lymphocyte expressing the antibody that specifically binds to the antigen of interest further comprises: providing a labeled antibody-binding agent prior to or concurrently with the antigen of interest, wherein monitoring binding of the antigen of interest to the antibody expressed by the B cell lymphocyte comprises detecting indirect binding of the labeled antibody-binding agent to the antigen of interest.

11. The method of any one of claims 1 to 10, wherein the microfluidic device comprises a plurality of sequestration pens, each having an isolation region and a connection region, each connection region providing a fluidic connection between the isolation region and the flow region, and further wherein identifying the B cell lymphocyte that expresses the antibody that specifically binds to the antigen of interest further comprises:
loading one or more of the plurality of B cell lymphocytes into the isolation region of each of two or more sequestration pens of the plurality;
introducing the antigen of interest into the microfluidic device to a location proximal to each of the two or more sequestration pens loaded with one or more B cell lymphocytes;
monitoring of binding of the antigen of interest to the antibody expressed by each of the loaded B cell lymphocytes; and
detecting binding of the antigen of interest to the antibody expressed by the loaded B cell lymphocyte, or ones of the loaded B cell lymphocytes, thereby identifying the loaded B cell lymphocyte or ones of the loaded B cell lymphocytes as expressing the antibody specifically binding to the antigen of interest.

12. The method of any one of claims 1 to 11, wherein sequencing the immunoglobulin heavy chain variable region (V_{H}) comprises:
lysing the identified B cell lymphocyte;
reverse transcribing mRNA isolated from the B cell lymphocyte, wherein the mRNA encodes the immunoglobulin heavy chain variable region (V_{H}), thereby forming V_{H} cDNA; and
sequencing at least a portion of the V_{H} cDNA.

13. The method of any one of claims 1 to 12, wherein sequencing the immunoglobulin light chain variable region (V_{L}) comprises:
lysing the identified B cell lymphocyte;
reverse transcribing mRNA isolated from the B cell lymphocyte, wherein the mRNA encodes the immunoglobulin light chain variable region (V_{L}), thereby forming V_{L} cDNA; and
sequencing at least a portion of the V_{L} cDNA.

14. The method of claim 12 or 13, wherein reverse transcribing the mRNA comprises contacting the mRNA with a capture/priming oligonucleotide; and optionally the reverse transcribing is performed in the presence of a transcript switching oligonucleotide.

15. The method of any one of claims 12 to 14, wherein the identified B cell lymphocyte is exported from the microfluidic device prior to being lysed.

16. The method of claim 15, wherein exporting the identified B cell lymphocyte comprises:
moving the identified B cell lymphocyte from the isolation region of the sequestration pen into the flow region of the microfluidic device; and
flowing the identified B cell lymphocyte through the flow region and out of the microfluidic device; and
optionally further wherein moving the identified B cell lymphocyte from the isolation region of the sequestration pen comprises capturing and moving the identified B cell lymphocyte using DEP force.

17. The method of claim 12 or 13, wherein the identified B cell lymphocyte is lysed within the microfluidic device.

18. The method of claim 17, further comprising:
providing one or more capture beads in close proximity to the identified B cell lymphocyte, where the one or more capture beads each comprises oliogonucleotides capable of binding the V_{H} mRNA and/or the V_{L} mRNA;
lysing the identified B cell lymphocyte; and
allowing the V_{H} mRNA and/or the V_{L} mRNA from the lysed B cell lymphocyte to be bound by the one or more capture beads; and
optionally further wherein each of the one or more capture beads comprises a plurality of capture/priming oligonucleotides.

19. The method of claim 18, wherein the bound V_{H} mRNA and/or the bound V_{L} mRNA is reverse transcribed into V_{H} cDNA and/or V_{L} cDNA while bound to the one or more capture beads.

20. The method of claim 19, wherein the V_{H} cDNA and/or V_{L} cDNA is exported from the microfluidic device while bound to the one or more capture beads.

21. The method of claim 12 or 13, further comprising amplifying the V_{H} cDNA and/or the V_{L} cDNA prior to the sequencing.

22. The method of claim 21, wherein amplifying comprises increasing the representation of V_{H} cDNA and/or V_{L} cDNA, or fragments thereof, in the reverse transcribed mRNA isolated from the B cell lymphocyte; and optionally further wherein amplifying comprises:
a first round of amplification which increases the representation of V_{H} cDNA and/or V_{L} cDNA, or fragments thereof, in the reverse transcribed mRNA isolated from the B cell lymphocyte; and
a second round of amplification which introduces barcode sequences into the V_{H} cDNA and/or V_{L} cDNA, or fragments thereof, amplified in the first round.
